# EUROPÄISCHE PATENTSCHRIFT

(11) **EP 2 258 695 B1**
(45) Veröffentlichungstag und Bekanntmachung des Hinweises auf die Patenterteilung: **29.05.2013**
(21) Anmeldenummer: 10008870.7
(22) Anmeldetag: 28.09.2005
(51) Int. Cl.: C07D 311/58, C07D 311/60, C09K 19/34

(54) **CHROMAN-DERIVATE SOWIE IHRE VERWENDUNG IN FLÜSSIGKRISTALLINEN MEDIEN**
CHROMAN DERIVATIVES AND USE THEREOF IN LIQUID CRYSTAL MEDIA
DERIVES DE CHROMANE, PROCEDES DE PRODUCTION ET UTILISATION DE CES DERIVES

(30) Priorität: 07.10.2004 DE 102004048853
(43) Veröffentlichungstag der Anmeldung: 08.12.2010
(62) Teilanmeldung aus: 05796927.1
(73) Patentinhaber: Merck Patent GmbH, 64293 Darmstadt (DE)
(72) Erfinder: Klein, Markus, 64291 Darmstadt (DE); Kirsch, Peer, Yokohama Kanagawa 224-0033 (JP); Poetsch, Eike, 64367 Muehltal (DE); Heckmeier, Michael, 69502 Hemsbach (DE); Best, Peter, 64289 Darmstadt (DE); Taugerbeck, Andreas, 64285 Darmstadt (DE); Klasen-Memmer, Melanie, 67259 Heuchelheim (DE)

(56) Entgegenhaltungen:
- EP-A1- 0 025 598
- PATENT ABSTRACTS OF JAPAN Bd. 018, Nr. 656 (C-1286), 13. Dezember 1994 (1994-12-13) -& JP 06, 256337, A, (DAINIPPON INK & CHEM INC; OTHERS: 01), 13. September 1994 (1994-09-13)
- PATENT ABSTRACTS OF JAPAN Bd. 018, Nr. 656 (C-1286), 13. Dezember 1994 (1994-12-13) -& JP 06, 256339, A, (DAINIPPON INK & CHEM INC; OTHERS: 01), 13. September 1994 (1994-09-13)
- PATENT ABSTRACTS OF JAPAN Bd. 017, Nr. 309 (C-1070), 14. Juni 1993 (1993-06-14) -& JP 05, 025158, A, (KANTO CHEM CO INC), 2. Februar 1993 (1993-02-02)

## Beschreibung

Die vorliegende Erfindung betrifft Chroman-Derivate, Verfahren zu ihrer Herstellung sowie ihre Verwendung als Komponente(n) in flüssigkristallinen Medien. Darüber hinaus betrifft die vorliegende Erfindung Flüssigkristall- und elektrooptische Anzeigeelemente, die die erfindungsgemäßen, flüssigkristallinen Medien enthalten.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (Electrically controlled birefringence), dem IPS-Effekt (In Plane Switching) oder dem Effekt der dynamischen Streuung beruhen.

Benzokondensierte Sauerstoffheterocyclen sind geeignete Komponenten für flüssigkristalline Mischungen, die in Flüssigkristall- und elektrooptischen Anzeigeelementen verwendet werden können.

So werden Dihydrobenzofuran- und Chromanderivate der folgenden Formel 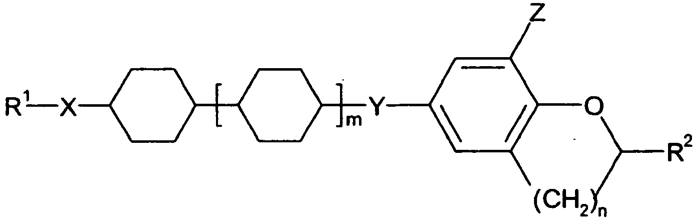 als Komponenten flüssigkristalliner Mischungen in der JP 06/256337 offenbart, wobei R¹, R², X, Y, Z, m und n die in diesem Dokument angegebenen Bedeutungen aufweisen.

Chromanderivate der folgenden Formel 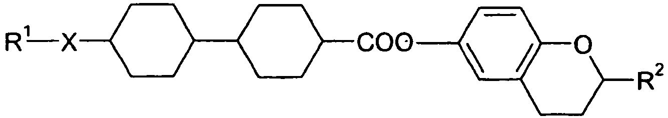 als Komponenten flüssigkristalliner Mischungen werden in der JP 06/256339 offenbart, wobei R¹, R² und X die in diesem Dokument angegebenen Bedeutungen aufweisen.

In den oben genannten Dokumenten werden darüber hinaus auch Verfahren zur Herstellung von Benzofuran- und Chroman-Derivaten offenbart.

Der Erfindung lag die Aufgabe zugrunde, neue stabile, flüssigkristalline oder mesogene Verbindungen aufzufinden, die als Komponente(n) flüssigkristalliner Medien, insbesondere für TN-, STN-, IPS-, TFT- und VA-Displays, geeignet sind.

Darüber hinaus war es Aufgabe der vorliegenden Erfindung, flüssigkristalline Verbindungen bereitzustellen, die eine hohe dielektrische Anisotropie Δε aufweisen, und zwar je nach Substitution entweder positiv oder negativ. Darüber hinaus sollten die erfindungsgemäßen Verbindungen thermisch, chemisch und photochemisch stabil sein. Ferner sollten die erfindungsgemäßen Verbindungen eine möglichst breite nematische Phase aufweisen sowie hervorragend mit nematischen Basismischungen, insbesondere bei tiefen Temperaturen, mischbar sein.

Überraschenderweise wurde gefunden, dass die erfindungsgemäßen Chroman-Derivate vorzüglich als Komponente(n) flüssigkristalliner Medien geeignet sind. Mit ihrer Hilfe lassen sich stabile, flüssigkristalline Medien, insbesondere geeignet für TFT- oder STN-Displays, erhalten. Die erfindungsgemäßen Verbindungen sind sowohl thermisch als auch UV-stabil. Auch zeichnen sie sich durch hohe dielektrische Anisotropien Δε aus, aufgrund derer in der Anwendung niedrigere Schwellenspannungen erforderlich sind. Darüber hinaus weisen die erfindungsgemäßen Verbindungen einen breiten nematischen Phasenbereich sowie ein hohes Voltage Holding Ratio auf. Vorteilhaft ist auch die gute Löslichkeit der erfindungsgemäßen Verbindungen, aufgrund derer sie besonders geeignet sind zur Erhöhung der Tieftemperaturstabilität von polaren Flüssigkristallmischungen.

Durch geeignete Wahl der Ringglieder und/oder der terminalen Substituenten lassen sich die physikalischen Eigenschaften der erfindungsgemäßen Flüssigkristalle in weiten Bereichen variieren.

Da die Chromaneinheit in ihrer Länge zwischen der der üblichen sechsgliedrigen 1- und 2-Kerner liegt, zeichnen sich die erfindungsgemäßen Derivate zudem durch positive elastische Eigenschaften aus.

Flüssigkristalline Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt.

Mit der Bereitstellung der erfindungsgemäßen Chroman-Derivate wird ganz allgemein die Palette der flüssigkristallinen Substanzen, die sich unter verschiedenen, anwendungstechnischen Gesichtspunkten zur Herstellung flüssigkristalliner Gemische eignen, erheblich verbreitert.

Die erfindungsgemäßen Chroman-Derivate besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die erfindungsgemäßen Chroman-Derivate sind in reinem Zustand farblos und bilden flüssigkristalline Mesophasen in einem für die elektrooptische Anwendung günstig gelegenen Temperaturbereich. Chemisch, thermisch und gegen Licht sind sie stabil.

Gegenstand der vorliegenden Erfindung sind somit Chroman-Derivate der allgemeinen Formel (la) 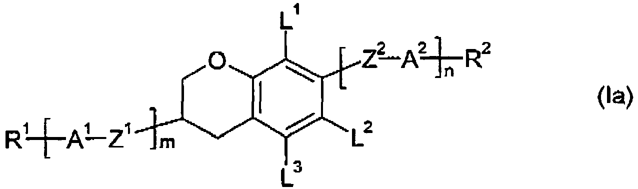 worin
- R¹: einen linearen oder verzweigten, gegebenenfalls chiralen, unsubstituierten, einen einfach durch CN oder CF₃ oder einen mindestens einfach durch Halogen substituierten Alkylrest mit 1 bis 15 C-Atomen oder Alkenylrest mit 2 bis 15 C-Atomen, worin auch eine oder mehrere CH₂-Gruppen jeweils unabhängig voneinander durch -O-, -S-, -CO-, -CO-O-, -O-CO-, -O-CO-O-, -CH=CH-, -CH=CF-, -CF=CH-, -CF=CF-, -C≡C- oder 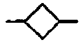 so ersetzt sein können, dass Heteroatome nicht direkt miteinander verknüpft sind,
- R²: H, F, Cl, NCS, CN, SF₅, einen Alkyl- oder Alkoxyrest mit 1 bis 15 C-Atomen, einen Alkenyl- oder Alkenyloxyrest mit 2 bis 15 C-Atomen, einen durch ein oder mehrere Fluoratome substituierten Alkyl- oder Alkoxyrest mit 1 bis 15 C-Atomen oder einen durch ein oder mehrere Fluoratome substituierten Alkenyl- oder Alkenyloxyrest mit 2 bis 15 C-Atomen,
- A¹, A²: jeweils unabhängig voneinander, gleich oder verschieden
a) trans-1,4-Cyclohexylen, worin auch eine oder mehrere nicht benachbarte CH₂-Gruppen durch -O- und/oder -S-ersetzt sein können,
b) 1,4-Phenylen, worin eine oder zwei CH-Gruppen durch N ersetzt sein können und worin auch ein oder mehrere H-Atome durch F ersetzt sein können,
c) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl, oder
d) 1,4-Cyclohexenylen,
- Z¹, Z²: jeweils unabhängig voneinander, gleich oder verschieden -O-, -CH₂O-, -OCH₂-, -CO-O-, -O-CO-, -CF₂O-, -OCF₂-, -CF₂CF₂-, -CH₂CF₂-, -CF₂CH₂-, -CH₂CF₂O-, -OCF₂CH₂-, -CH₂CH₂-, -CH=CH-, -CH=CF-, -CF=CH-, -CF=CF-, -CF=CF-CO-O-, -O-CO-CF=CF-, -C≡C- oder eine Einfachbindung,
- L¹, L², L³: jeweils unabhängig voneinander, gleich oder verschieden H, F, Cl, NCS, CN, SF₅, einen durch ein oder mehrere Fluoratome substituierten Alkyl- oder Alkoxyrest mit 1 bis 15 C-Atomen oder einen durch ein oder mehrere Fluoratome substituierten Alkenyl- oder Alkenyloxyrest mit 2 bis 15 C-Atomen, vorzugsweise H, F, Cl oder CN, und besonders bevorzugt H oder F,
- m: 0, 1, 2 oder 3, und besonders bevorzugt 0, 1 oder 2, bedeutet, und
- n: 1, 2, 3 oder 4, vorzugsweise 1, 2 oder 3 und besonders bevorzugt 1 oder 2, bedeutet,
jedoch mit der Maßgabe, dass die Summe (m + n) = 1, 2, 3 oder 4, vorzugsweise 1, 2 oder 3 und besonders bevorzugt 1 oder 2, ist, und der allgemeinen Formel (II) 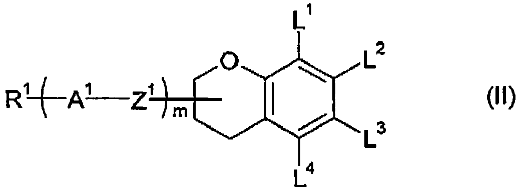 worin R¹, A¹ und Z¹ die in bezug auf Formel (I) angegebenen Bedeutungen aufweisen,
- L¹, L², L³ und L⁴: jeweils unabhängig voneinander, gleich oder verschieden H, F, Cl, NCS, CN, SF₅, einen durch ein oder mehrere Fluoratome substituierten Alkyl- oder Alkoxyrest mit 1 bis 15 C-Atomen oder einen durch ein oder mehrere Fluoratome substituierten Alkenyl- oder Alkenyloxyrest mit 2 bis 15 C-Atomen, vorzugsweise H, F, Cl oder CN, und besonders bevorzugt H oder F, bedeuten, wobei
einer der beiden Reste L² und L³ zusätzlich auch die Bedeutung von R² in bezug auf Formel (Ia) annehmen kann

Ein weiterer Gegenstand der vorliegenden Erfindung ist die Verwendung von Chroman-Derivaten der Formeln (la) und (IIa) als Komponente(n) in flüssigkristallinen Medien.

Ebenfalls Gegenstand der vorliegenden Erfindung sind flüssigkristalline Medien mit mindestens zwei flüssigkristallinen Komponenten, welche mindestens ein Chroman-Derivat der Formeln (Ia) und (IIa) enthalten.

Gegenstand der vorliegenden Erfindung sind auch Flüssigkristall-Anzeigeelemente, insbesondere elektrooptische Anzeigeelemente, welche als Dielektrikum ein erfindungsgemäßes, flüssigkristallines Medium enthalten.

In einer bevorzugten Ausführungsform weisen die erfindungsgemäßen Verbindungen der Formel (la) und (IIa) ein negatives Δε auf. Aufgrund des negativen Δε eignen diese Verbindungen sich insbesondere für eine Verwendung in VA-Displays.

Gegenstand der vorliegenden Erfindung sind somit insbesondere auch VA-TFT-Displays, mit Dielektrika, welche mindestens ein Chroman-Derivat der Formeln (la) und (IIa) mit negativem Δε enthalten.

In einer weiteren bevorzugten Ausführungsform weisen die erfindungsgemäßen Verbindungen der Formel (Ia)/(IIa) ein positives Δε auf. Aufgrund des positiven Δε eignen diese Verbindungen sich insbesondere für eine Verwendung in hochpolaren Mischungen.

Gegenstand der vorliegenden Erfindung sind somit insbesondere auch TFT-Displays mit niedriger Schwellenspannung (sog. "low Vₜₕ-TFT-Displays") sowie IPS-Displays (sog. "In Plane Switching-Displays"), mit Dielektrika, welche mindestens ein Chroman- und/oder Chromen-Derivat der Formeln (Ia)/(IIa) mit positivem Δε enthalten.

Sofern die erfindungsgemäßen Verbindungen der Formel (Ia)/(IIa) neben einem positiven Δε zusätzlich noch eine niedrige Doppelbrechung Δn aufweisen, eignen diese Verbindungen sich insbesondere für eine Verwendung in reflektiven und transflektiven Flüssigkristall-Anzeigeelementen sowie anderen Flüssigkristall-Anzeigen mit niedriger Doppelbrechung Δn, sogenannte "low Δn mode displays", wie z.B. reflektive und transflektive TN-Anzeigen.

Gegenstand der vorliegenden Erfindung sind somit insbesondere auch reflektive und transflektive TN-Displays, mit Dielektrika, welche mindestens ein Chroman- und/oder Chromen-Derivat der Formeln (Ia)/(IIa) mit positivem Δε enthalten.

Darüber hinaus finden die erfindungsgemäßen Chroman- und Chromen-Derivate der Formeln (Ia)/(IIa) mit positivem Δε Verwendung als polare Hochklärer in Displays, die bei einer Temperatur betrieben werden, bei der die Steuermedien in der isotropen Phase oder in einer optisch isotropen Phase vorliegen. Solche Displays werden z.B. in der DE-A-102 17 273, der DE-A-102 53 325, der DE-A-102 53 606 sowie der DE-A-103 13 979 beschrieben.

Die Bedeutung der Formeln (Ia)/(IIa) schließt alle Isotope der in den Verbindungen der Formeln (Ia)/(IIa) gebundenen chemischen Elemente ein. In enantiomerenreiner oder -angereicherter Form eignen sich die Verbindungen der Formeln (Ia)/(IIa) auch als chirale Dotierstoffe und generell zur Erzielung chiraler Mesophasen.

Vor- und nachstehend haben R¹, R², A¹, A², Z¹, Z², L¹, L², L³, L⁴, L⁵, L⁶, m und n die angegebenen Bedeutungen, sofern nicht ausdrücklich etwas anderes vermerkt ist. Kommen die Reste A¹ und Z¹ sowie A² und Z² mehrfach vor, so können sie unabhängig voneinander, gleiche oder verschiedene Bedeutungen annehmen.

Der Einfachheit halber bedeuten im folgenden Cyc einen 1,4-Cyclohexylenrest, Che einen 1,4-Cyclohexenylenrest, Dio einen 1,3-Dioxan-2,5-diylrest, Thp einen Tetrahydropyran-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest, Bco einen Bicyclo-(2,2,2)-octylenrest und Dec einen Decahydronaphthalinrest, wobei Cyc und/oder Phe unsubstituiert bzw. ein- oder mehrfach durch -CH₃, -Cl, -F und/oder -CN substituiert sein können.

Bevorzugt sind Verbindungen der Formeln (Ia)/(IIa), worin R¹ H, einen linearen Alkyl- bzw. Alkoxyrest mit 1 bis 10 C-Atomen oder einen linearen Alkenyl- bzw. Alkenyloxyrest mit 2 bis 10 C-Atomen bedeutet.

Ist R¹ Halogen, so bedeutet es vorzugsweise F oder Cl, besonders bevorzugt F.

Bevorzugt sind Verbindungen der Formeln (Ia)/(IIa), worin R² F, Cl, CN, SF₅, CF₃, OCF₃ oder OCHF₂, besonders bevorzugt F, CN, CF₃ oder OCF₃ und insbesondere F, bedeutet.

A¹ und A² bedeuten vorzugsweise Phe, Cyc, Che, Pyd, Pyr oder Dio, und besonders bevorzugt Phe oder Cyc. Desweiteren bevorzugt sind Verbindungen der Formeln (I) bis (VI), die nicht mehr als einen der Reste Dio, Dit, Pyd, Pyr oder Bco enthalten.

Phe ist vorzugsweise

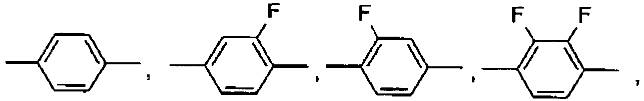

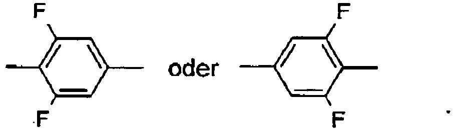

Phe ist besonders bevorzugt

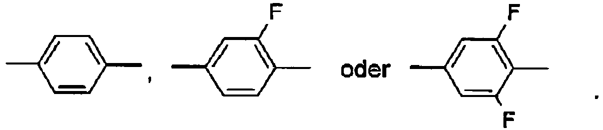

Die Begriffe 1,3-Dioxan-2,5-diyl und Dio umfassen jeweils die beiden Stellungsisomeren

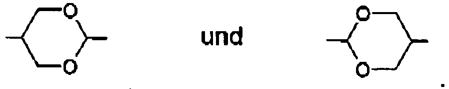

Die Cyclohexen-1,4-diyl-Gruppe hat vorzugsweise folgende Strukturen:

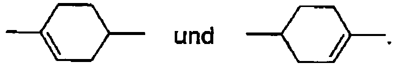

Z¹ und Z² bedeuten vorzugsweise -CH₂CH₂-, -CH=CH-, -C≡C-, -CF₂CF₂-, -CF=CF-, -COO-, -OCO-, -CF₂O-, -OCF₂- oder eine Einfachbindung, besonders bevorzugt -CF₂O-, -COO- oder eine Einfachbindung.

L¹, L², L³, L⁴, L⁵ und L⁶ bedeuten vorzugsweise H oder F.

Bevorzugte Chroman-Derivate der allgemeinen Formel (Ia) stellen die folgenden Formeln (Ia1) bis (Ia6) dar: 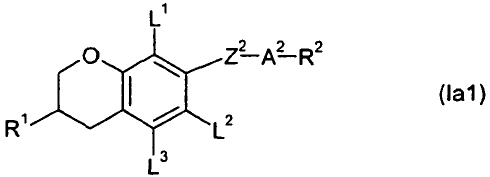 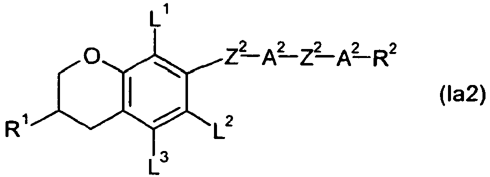 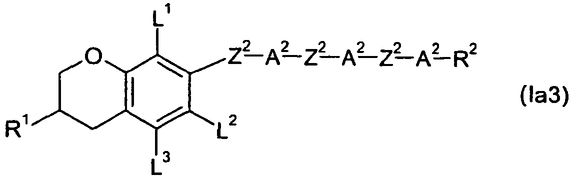 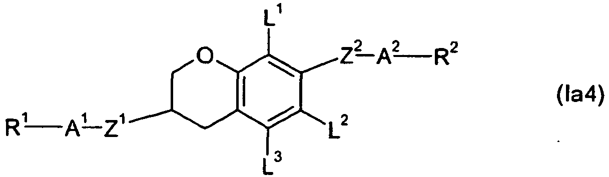 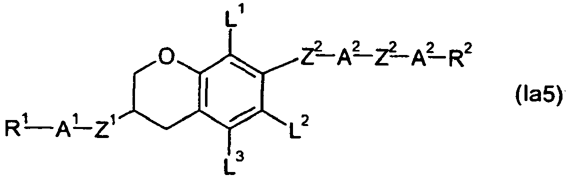 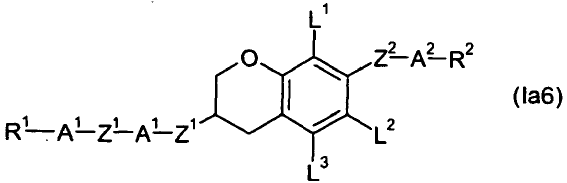 worin R¹, R², A¹, A², Z¹, Z², L¹, L² und L³ die in bezug auf Formel (I) angegebenen Bedeutungen aufweisen.

Besonders bevorzugt sind dabei die Chroman-Derivate der allgemeinen Formeln (Ia1) bis (Ia5), das heißt Chroman-Derivate der allgemeinen Formel (la), bei denen m = 0 oder 1 ist.

Desweiteren bevorzugt sind Chroman-Derivate der allgemeinen Formeln (Ia1) bis (Ia6), bei denen L³ = H und L¹ und L² unabhängig voneinander, gleich oder verschieden H oder F bedeuten, wobei es besonders bevorzugt ist, wenn L¹ = L² = F, L¹ = H und L² = F oder L¹ = L² = H.

Eine besonders bevorzugte Verbindung der Teilformel (Ia1) stellt diejenige der Teilformel (Ia1a) dar: 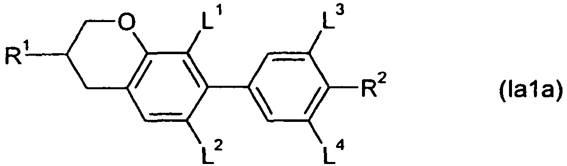 worin R¹ und R² die in bezug auf Formel (I) angegebenen Bedeutungen aufweisen und L¹, L², L³ und L⁴ unabhängig voneinander, gleich oder verschieden H oder F bedeuten.

Besonders bevorzugte Verbindungen der Teilformel (Ia2) stellen diejenigen der Teilformel (Ia2a) bis (Ia2c) dar: 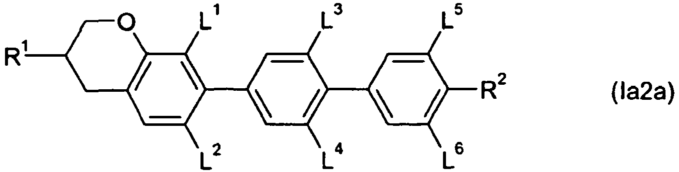 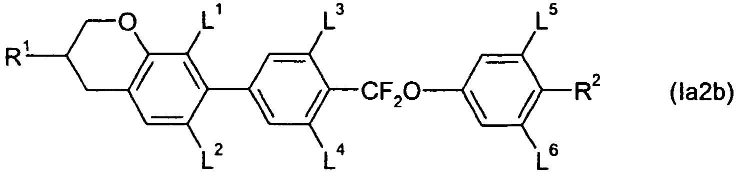 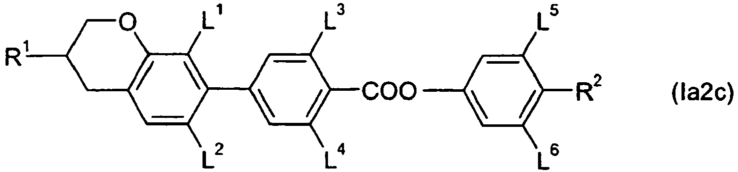 worin R¹ und R² die in bezug auf Formel (I) angegebenen Bedeutungen aufweisen und L¹, L², L³, L⁴, L⁵ und L⁶ unabhängig voneinander, gleich oder verschieden H oder F bedeuten.

Eine besonders bevorzugte Verbindung der Teilformel (Ia3) stellt diejenige der Teilformel (Ia3a) dar: 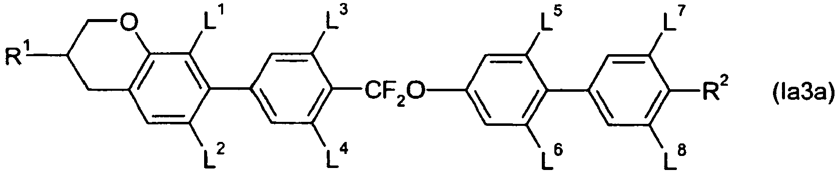 worin R¹ und R² die in bezug auf Formel (I) angegebenen Bedeutungen aufweisen und L¹, L², L³, L⁴, L⁵, L⁶, L⁷ und L⁸ unabhängig voneinander, gleich oder verschieden H oder F bedeuten.

Besonders bevorzugte Verbindungen der Teilformel (Ia4) stellen diejenigen der Teilformeln (Ia4a) bis (Ia4c) dar: 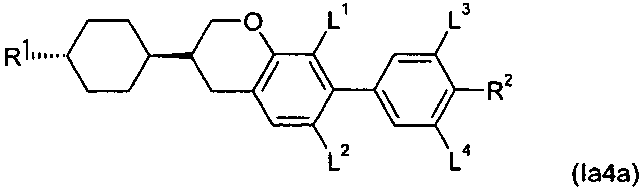 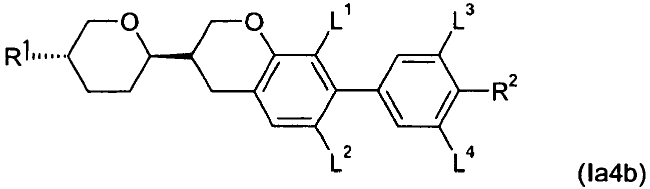 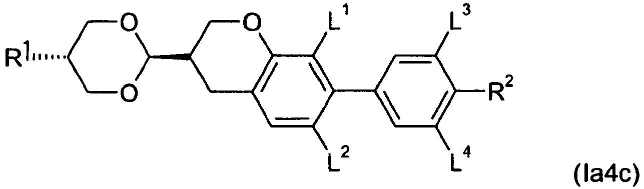 worin R¹ und R² die in bezug auf Formel (I) angegebenen Bedeutungen aufweisen und L¹, L², L³ und L⁴ unabhängig voneinander, gleich oder verschieden H oder F bedeuten.

Besonders bevorzugte Verbindungen der Teilformel (Ia5) stellen diejenigen der Teilformeln (Ia5a) bis (Ia5i), insbesondere diejenigen der Teilformeln (Ia5a) bis (Ia5c), dar: 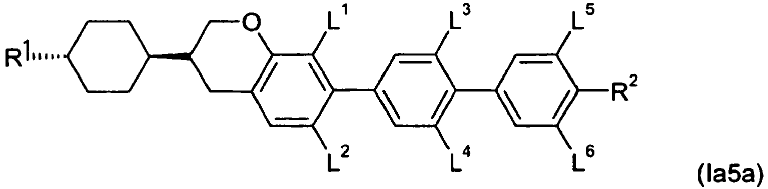 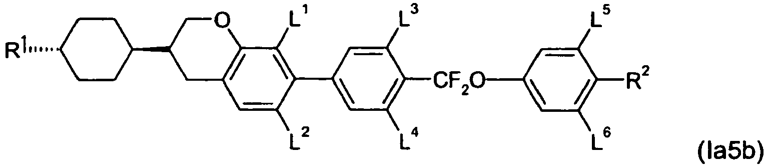 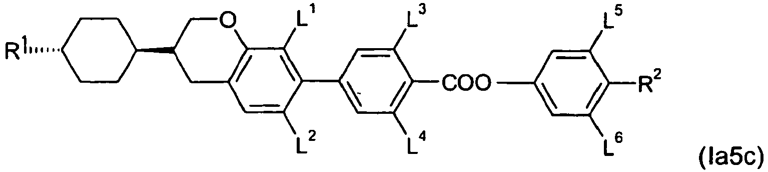 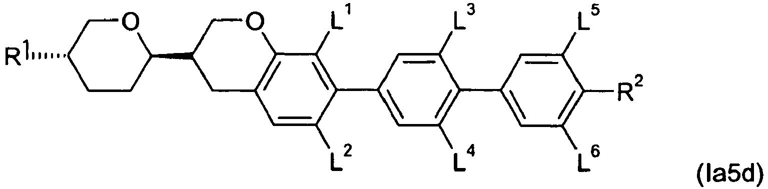 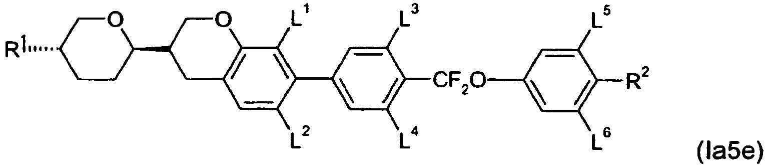 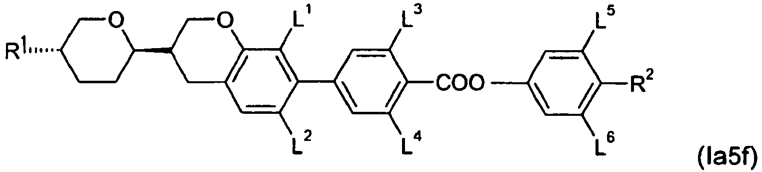 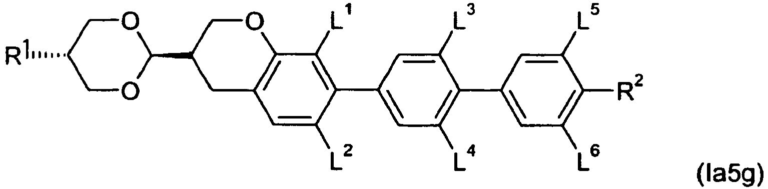 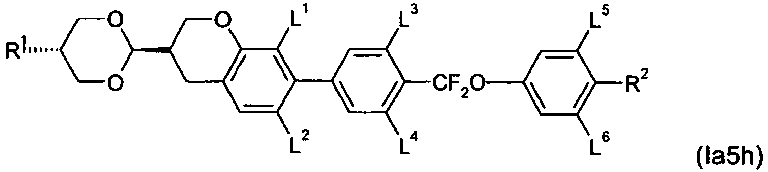 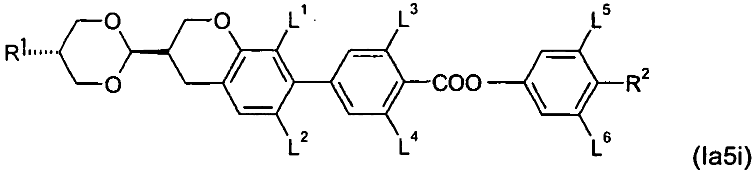 worin R¹ und R² die in bezug auf Formel (I) angegebenen Bedeutungen aufweisen und L¹, L², L³, L⁴, L⁵ und L⁶ unabhängig voneinander, gleich oder verschieden H oder F bedeuten.

Bevorzugte Chroman-Derivate der allgemeinen Formel (II) stellen die folgenden Formeln (IIa) bis (IId) dar: 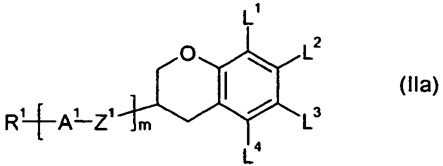 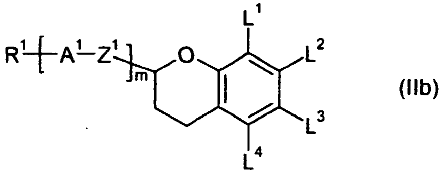 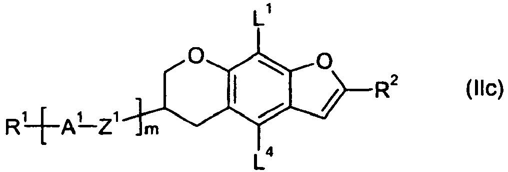 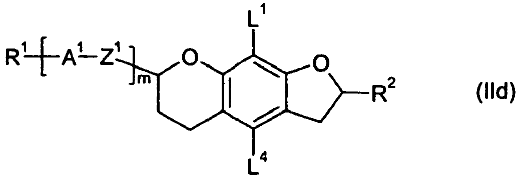 worin R¹, A¹, Z¹, L¹, L², L³, L⁴ und m die in bezug auf Formel (II) und R² die in bezug auf Formel (I) angegebenen Bedeutungen aufweisen.

Besonders bevorzugt sind dabei die Chroman-Derivate der allgemeinen Formeln (IIa) und (IIb).

Bevorzugte Chroman-Derivate der allgemeinen Formel (IIa) stellen die folgenden Formeln (IIa1) bis (IIa3) dar: 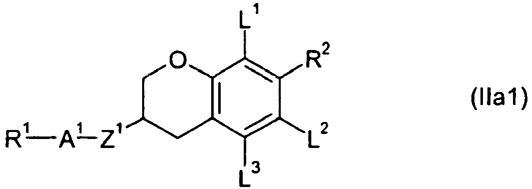 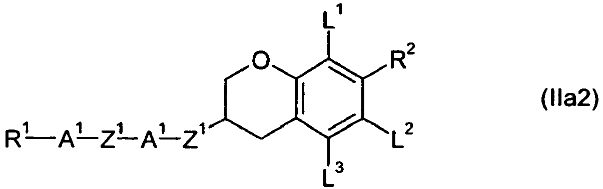 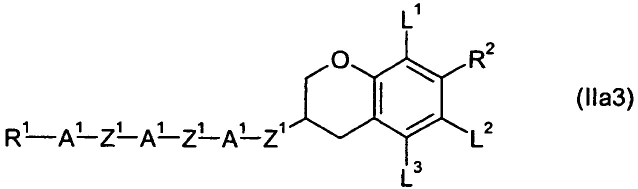 worin R¹, A¹, Z¹, L¹, L² und L³ die in bezug auf Formel (II) und R² die in bezug auf Formel (I) angegebenen Bedeutungen aufweisen.

Besonders bevorzugte Verbindungen der Teilformel (IIa1) stellen diejenigen der Teilformel (IIa1a) und (IIa1b) dar: 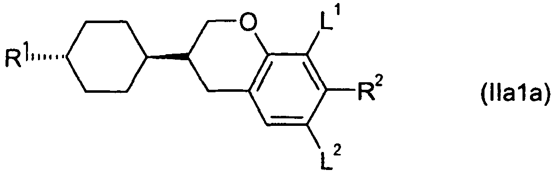 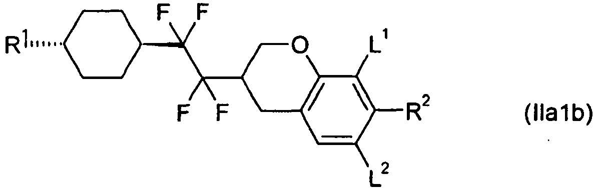 worin R¹ und R² die in bezug auf Formel (I) angegebenen Bedeutungen aufweisen und L¹ und L² unabhängig voneinander, gleich oder verschieden H oder F bedeuten.

Die Chroman-Derivate der allgemeinen Formeln (IIa1) bis (IIa3) weisen dabei vorzugsweise die folgenden Strukturen auf: 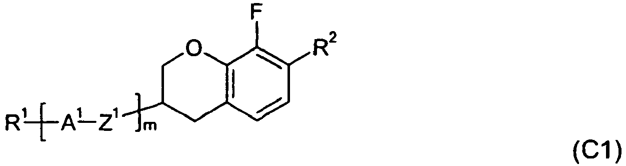 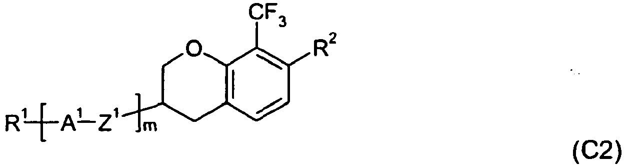 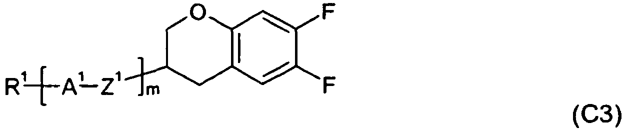 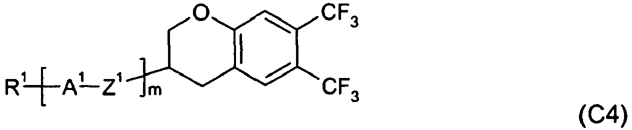 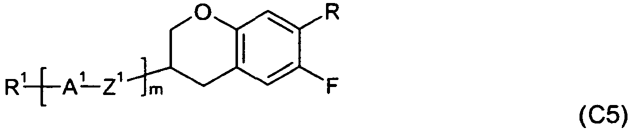 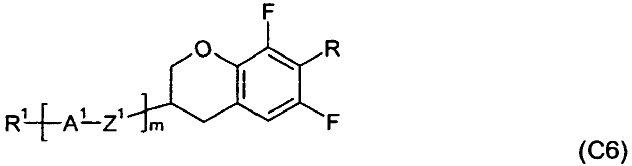 worin R¹, A¹ und Z¹ die in bezug auf Formel (II) angegebenen Bedeutungen annehmen, R² die in bezug auf Formel (I) angegebenen Bedeutungen annimmt und m = 1, 2 oder 3 bedeutet.

Die Verbindungen der Formel (II), (IIa) bis (IId) sowie deren Unterformeln umfassen Verbindungen mit einem Ring in der mesogenen Gruppe R¹(-A¹-Z¹)ₘ- der Teilformeln a und b:

R¹-A¹- a

R¹-A¹-Z¹- b

Verbindungen mit zwei Ringen in der mesogenen Gruppe R¹(-A¹-Z¹)ₘ- der Teilformeln c bis f:

R¹-A¹-A¹- c

R¹-A¹-A¹-Z¹- d

R¹-A¹-Z¹-A¹- e

R¹-A¹-Z¹-A¹-Z¹- f

sowie Verbindungen mit drei Ringen in der mesogenen Gruppe R¹(-A¹-Z¹)ₘ- der Teilformeln g bis o:

R¹-A¹-A¹-A¹- g

R¹-A¹-Z¹-A¹-A¹- h

R¹-A¹-A¹-Z¹-A¹- i

R¹-A¹-A¹-A¹-Z¹- j

R¹-A¹-Z¹-A¹-Z¹-A¹- k

R¹-A¹-Z¹-A¹-A¹-Z¹- m

R¹-A¹-A¹-Z¹-A¹-Z¹- n

R¹-A¹-Z¹-A¹-Z¹-A¹-Z¹- o

Darunter sind diejenigen der Teilformeln a, b, c, d, e, g, h und i besonders bevorzugt.

Die bevorzugten Verbindungen der Teilformel a umfassen diejenigen der Teilformeln aa bis ad:

R¹-Phe- aa

R¹-Cyc- ab

R¹-Thp- ac

R¹-Dio- ad

Darunter sind diejenigen der folgenden Teilformeln besonders bevorzugt:

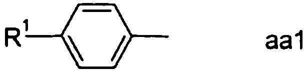

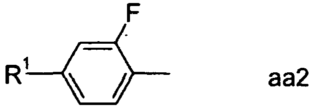

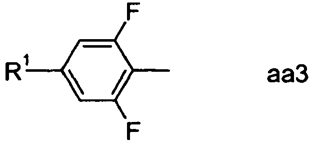

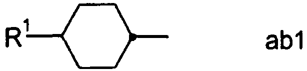

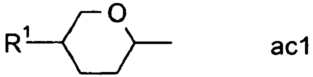

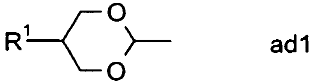

Die bevorzugten Verbindungen der Teilformel b umfassen diejenigen der Teilformeln ba und bb:

R¹-Phe-Z¹- ba

R¹-Cyc-Z¹- bb

Die bevorzugten Verbindungen der Teilformel Ic umfassen diejenigen der Teilformeln ca bis cm:

R¹-Cyc-Cyc- ca

R¹-Cyc-Thp- cb

R¹-Cyc-Dio- cc

R¹-Cyc-Phe- cd

R¹-Thp-Cyc- ce

R¹-Dio-Cyc- cf

R¹-Phe-Cyc- cg

R¹-Thp-Phe- ch

R¹-Dio-Phe- ci

R¹-Phe-Phe- cj

R¹-Pyr-Phe- ck

R¹-Pyd-Phe- cm

Darunter sind diejenigen der folgenden Teilformeln besonders bevorzugt:

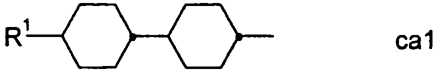

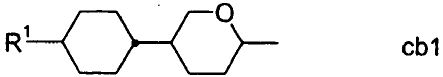

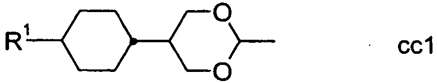

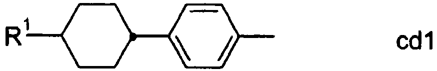

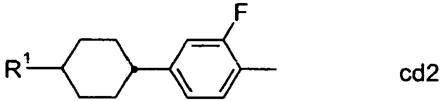

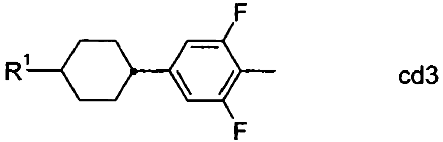

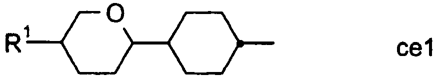

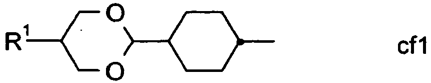

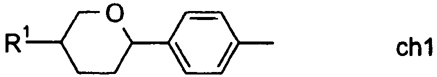

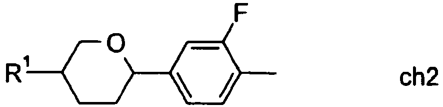

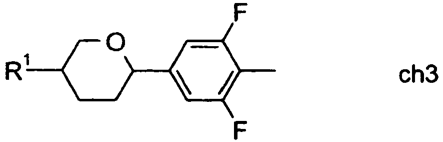

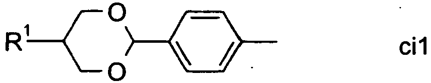

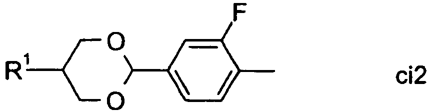

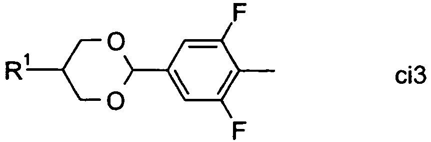

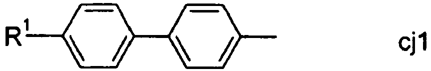

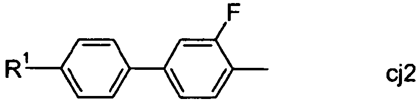

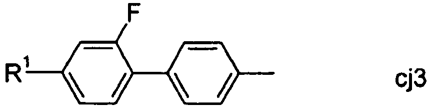

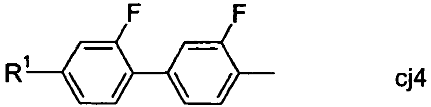

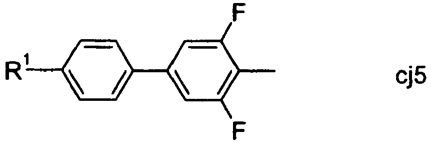

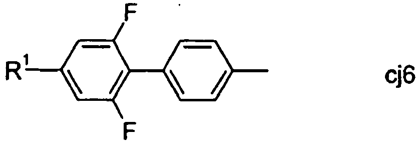

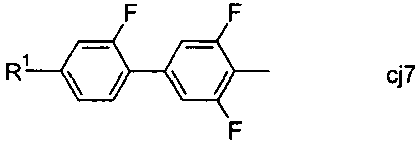

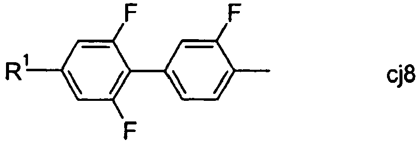

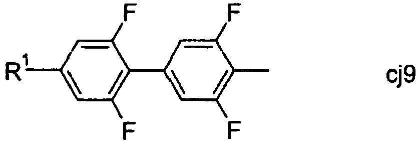

Die bevorzugten Verbindungen der Teilformel d umfassen diejenigen der Teilformeln da bis dn:

R¹-Cyc-Cyc-Z¹- da

R¹-Cyc-Thp-Z¹- db

R¹-Cyc-Dio-Z¹- dc

R¹-Cyc-Phe-Z¹- dd

R¹-Thp-Cyc-Z¹- de

R¹-Dio-Cyc-Z¹- df

R¹-Thp-Phe-Z¹- dg

R¹-Dio-Phe-Z¹- dh

R¹-Phe-Phe-Z¹- di

R¹-Pyr-Phe-Z¹- dj

R¹-Pyd-Phe-Z¹- dk

R¹-Cyc-Phe-CH₂CH₂- dm

R¹-A¹-Phe-CH₂CH₂- dn

Darunter sind diejenigen der folgenden Teilformeln besonders bevorzugt:

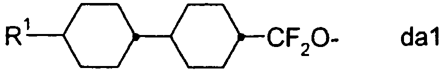

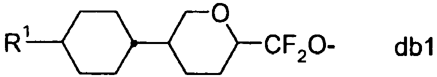

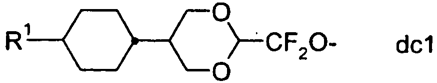

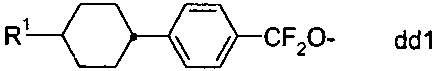

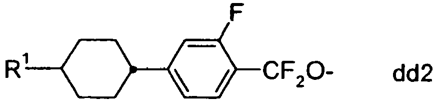

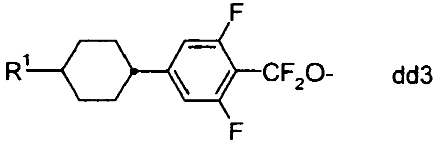

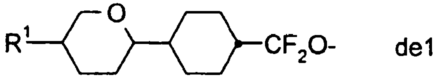

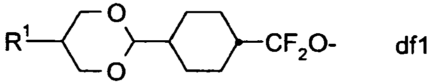

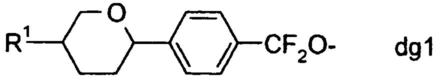

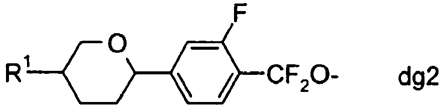

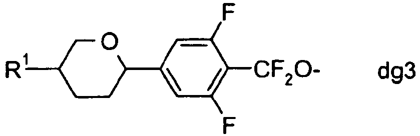

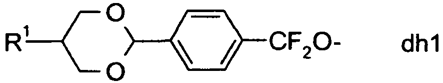

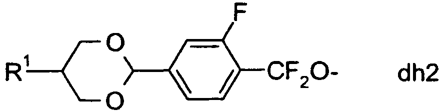

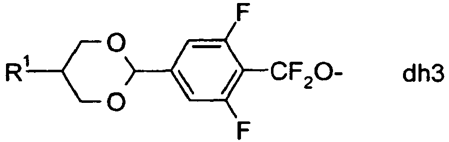

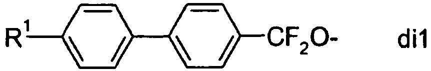

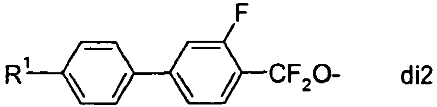

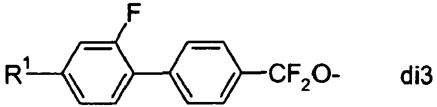

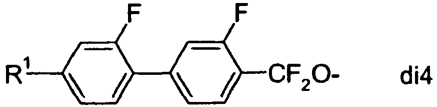

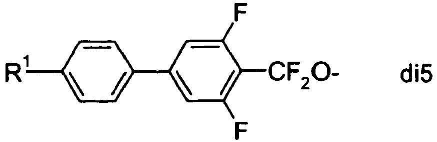

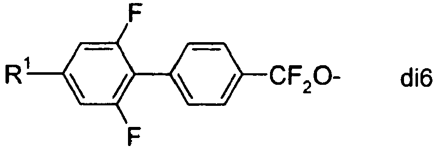

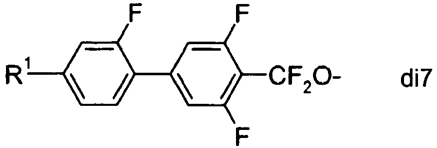

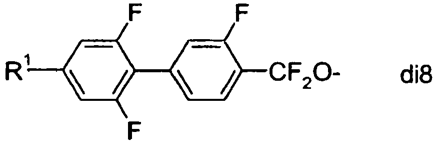

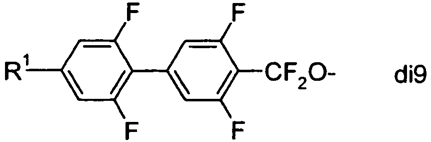

Die bevorzugten Verbindungen der Teilformel e umfassen diejenigen der Teilformeln ea bis ej:

R¹-Cyc-Z¹-Cyc- ea

R¹-Thp-Z¹-Cyc- eb

R¹-A¹- CH₂CH₂-A¹- ec

R¹-Cyc-Z¹-Phe- ed

R¹-Thp-Z¹-Phe- ee

R¹-A¹-OCO-Phe- ef

R¹-Phe-Z¹-Phe- eg

R¹-Pyr-Z¹-A¹- eh

R¹-Pyd-Z¹-A¹- ei

R¹-Dio-Z¹-A¹- ej

Darunter sind diejenigen der folgenden Teilformeln besonders bevorzugt:

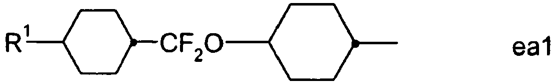

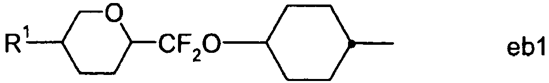

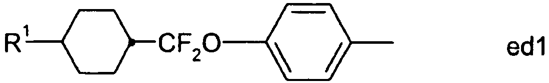

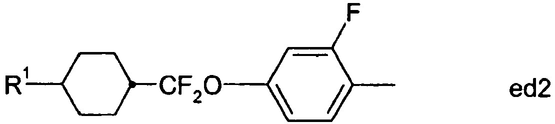

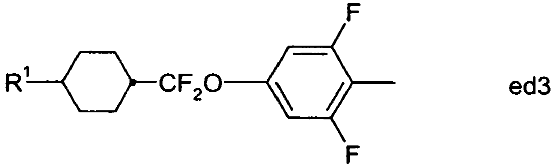

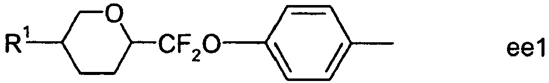

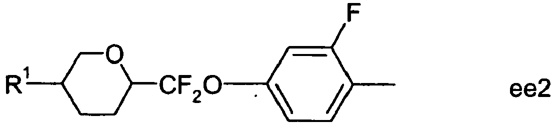

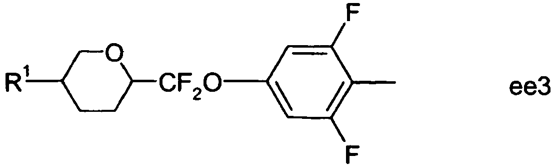

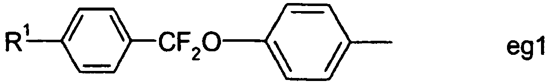

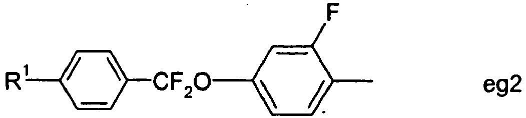

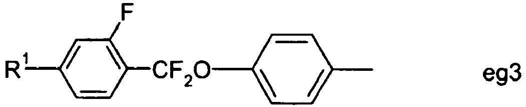

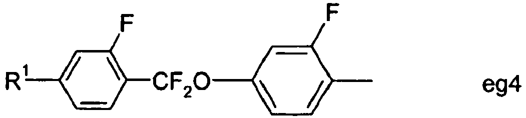

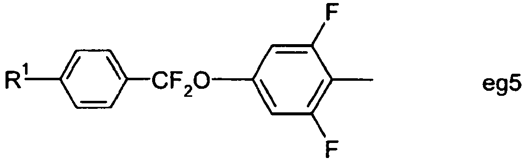

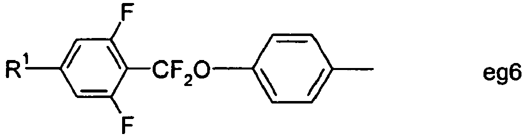

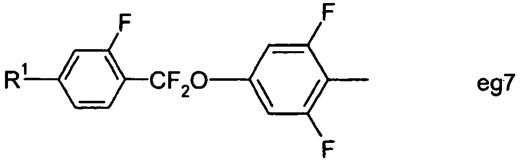

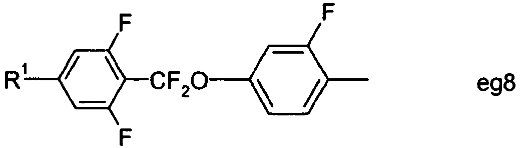

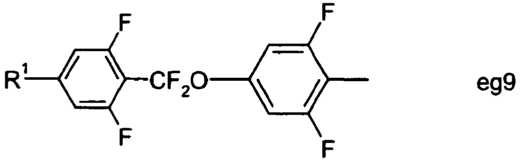

Die bevorzugten Verbindungen der Teilformel f umfassen diejenigen der Teilformeln fa bis fe:

R¹-Phe-CH₂CH₂-A¹-Z¹- fa

R¹-A¹-COO-Phe-Z¹- fb

R¹-Cyc-Z¹-Cyc-Z¹- fc

R¹-Phe-Z¹-Phe-Z¹- fd

R¹-Cyc-CH₂CH₂-Phe-Z¹- fe

Die bevorzugten Verbindungen der Teilformeln g bis n umfassen diejenigen der folgenden Teilformeln ga bis ma:

R¹-A¹-Cyc-Cyc- ga

R¹-A¹-Cyc-Phe- gb

R¹-Phe-Phe-Phe- gc

R¹-A¹-CH₂CH₂-A¹-Phe- ha

R¹-Phe-Z¹-A¹-Phe- hb

R¹-A¹-Phe-Z¹-Phe- ia

R¹-Cyc-Z¹-A¹-Z¹-Phe- ka

R¹-A¹-Z¹-Cyc-Phe-Z¹- ma

In den vorstehenden, bevorzugten Formeln besitzen R¹, A¹ und Z¹ die oben angegebenen Bedeutungen. Treten A¹ und/oder Z¹ in einer der Teilformeln mehrmals auf, so können sie unabhängig voneinander, gleich oder verschieden sein.

In den vorstehenden, bevorzugten Formeln bedeutet R¹ vorzugsweise einen linearen Alkyl- bzw. Alkoxyrest mit 1 bis 7 C-Atomen oder einen linearen Alkenyl- bzw. Alkenyloxyrest mit 2 bis 7 C-Atomen und besonders bevorzugt einen linearen Alkylrest mit 1 bis 7 C-Atomen oder einen linearen Alkenylrest mit 2 bis 7 C-Atomen.

In den vorstehenden, bevorzugten Formeln bedeutet Z¹ vorzugsweise -CH₂CH₂-, -C≡C-, -CF₂CF₂-, -COO-, -OCO-, -CF₂O- oder -OCF₂-.

Falls R¹ oder R² in den vor- und nachstehenden Formeln einen Alkylrest bedeutet, so kann dieser geradkettig oder verzweigt sein. Besonders bevorzugt ist er geradkettig, hat 1, 2, 3, 4, 5, 6 oder 7 C-Atome und bedeutet demnach Methyl, Ethyl, Propyl, Butyl, Pentyl, Hexyl oder Heptyl, ferner Octyl, Nonyl, Decyl, Undecyl, Dodecyl, Tridecyl, Tetradecyl oder Pentadecyl.

Falls R¹ oder R² einen Alkylrest bedeutet, in dem eine CH₂-Gruppe durch -O- ersetzt ist, so kann dieser geradkettig oder verzweigt sein. Vorzugsweise ist er geradkettig und hat 1 bis 10 C-Atome. Besonders bevorzugt ist die erste CH₂-Gruppe dieses Alkylrestes durch -O- ersetzt, so dass der Rest R¹ die Bedeutung Alkoxy erhält und Methoxy, Ethoxy, Propoxy, Butoxy, Pentyloxy, Hexyloxy, Heptyloxy, Octyloxy oder Nonyloxy bedeutet.

Weiterhin kann auch eine CH₂-Gruppe an anderer Stelle durch -O- ersetzt sein, so dass der Rest R¹ vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

Falls R¹ oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Bevorzugte Alkenylgruppen sind C₂-C₇-1 E-Alkenyl, C₄-C₇-3E-Alkenyl, C₅-C₇-4-Alkenyl, C₆-C₇-5-Alkenyl, und C₇-6-Alkenyl, besonders bevorzugt C₂-C₇-1E-Alkenyl, C₄-C₇-3E-Alkenyl und C₅-C₇-4-Alkenyl.

Beispiele besonders bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Gruppen mit bis zu 5 Kohlenstoffatomen sind insbesondere bevorzugt.

Falls R¹ einen Alkylrest bedeutet, in dem eine CH₂-Gruppe durch -O- und eine durch -CO- ersetzt ist, so sind diese vorzugsweise benachbart. Somit beinhalten diese eine Acyloxygruppe -CO-O- oder eine Oxycarbonylgruppe -O-CO-. Besonders bevorzugt sind diese geradkettig und haben 2 bis 6 C-Atome.

Sie bedeuten demnach insbesondere Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl und 4-(Methoxycarbonyl)butyl.

Falls R¹ einen Alkylrest bedeutet, in dem eine CH₂-Gruppe durch unsubstituiertes oder substituiertes -CH=CH- und eine benachbarte CH₂-Gruppe durch -CO-, -CO-O- oder -O-CO- ersetzt ist, so kann dieser geradkettig oder verzweigt sein. Vorzugsweise ist er geradkettig und hat 4 bis 13 C-Atome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl und 9-Methacryloyloxynonyl.

Falls R¹ einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R¹ oder R² einen mindestens einfach durch Halogen substituierten Alkyl- oder Alkenylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formeln (I) bis (VI) mit verzweigter Flügelgruppe R¹ oder R² können gelegentlich wegen einer besseren Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponente(n) für ferroelektrische Materialien.

Verzweigte Gruppen dieser Art enthalten vorzugsweise nicht mehr als eine Kettenverzweigung. Bevorzugte verzweigte Reste R¹ oder R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy und 1-Methylheptyloxy.

Die Formeln (I) bis (VI) umfassen sowohl die Racemate dieser Verbindungen als auch die optischen Antipoden sowie deren Gemische.

Unter den Verbindungen der Formeln (I) bis (VI) sowie den Unterformeln sind diejenigen bevorzugt, in denen mindestens einer der darin enthaltenen Reste eine der angegebenen bevorzugten Bedeutungen hat.

In den Verbindungen der Formeln (I) bis (VI) sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die Verbindungen der allgemeinen Formeln (I) bis (VI) können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsmaterialien für die obigen Verfahren sind entweder bekannt oder können in Analogie zu bekannten Verbindungen hergestellt werden. Sie sind somit nach allgemein zugänglichen Literaturvorschriften oder käuflich zu erhalten.

Die Ausgangsstoffe können gegebenenfalls auch in situ gebildet werden, derart, dass man sie aus dem Reaktionsgemisch nicht isoliert, sondern sofort weiter zu den Verbindungen der allgemeinen Formeln (I) bis (VI) umsetzt.

Eine bevorzugte Synthese der Verbindungen der allgemeinen Formeln (Ib) sowie (III) kann nach den in der Literatur, z.B. in Houben Weyl, Methoden der organischen Chemie, Georg Thieme Verlag, Stuttgart, New York, 4.Aufl. 1993, beschriebenen Verfahren erfolgen.

Ein bevorzugtes Verfahren ist die Herstellung von Verbindungen der allgemeinen Formel (Ib) durch Ringschlußmetathese der entsprechend substituierten Diene **3**, die gemäß S. Chang, R.H. Grubbs, J. Org. Chem. 1998, 63, 864-866, zugänglich sind. Die so erhaltenen Chromene der allgemeinen Formel (III) können durch katalytische Hydrierung zu den Chromanen der allgemeinen Formel (Ib) umgesetzt werden, wie in Schema 1 dargestellt.

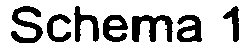

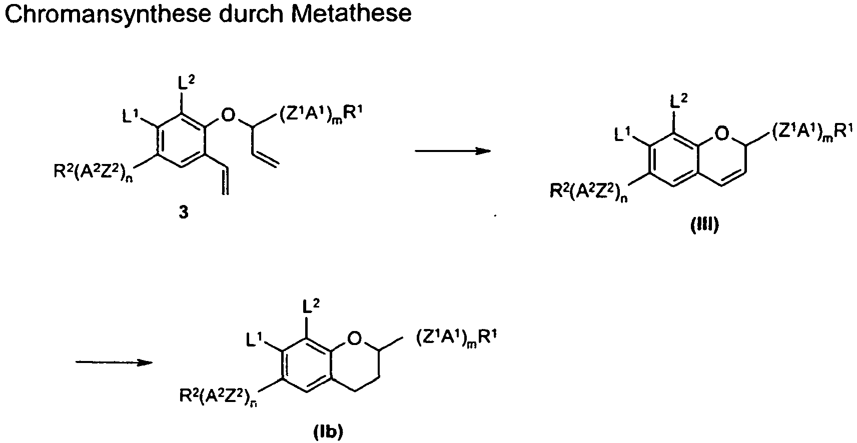

Alternativ dazu können die erfindungsgemäßen Verbindungen der allgemeinen Formel (Ib) auch durch intramolekulare Cyclisierung von Diolen erhalten werden, wie z.B. von S. Kelly, B.C. Vanderplas, in J. Org. Chem. 1991, 56, 1325-1327 beschrieben und in Schema 2 dargestellt.

Dabei erhält man durch Aldolkondensation der Salicylaldehydderivate **4** mit Methylketonen und anschließende Hydrierung und Abspaltung der Schutzgruppe die Ketone **5,** die nach Reduktion zum Alkohol **6** z.B. mit Natriumborhydrid und durch anschließendes Behandeln mit Schwefelsäure in Eisessig zu den Verbindungen der Formel (Ib) cyclisieren.

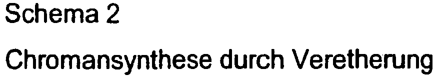

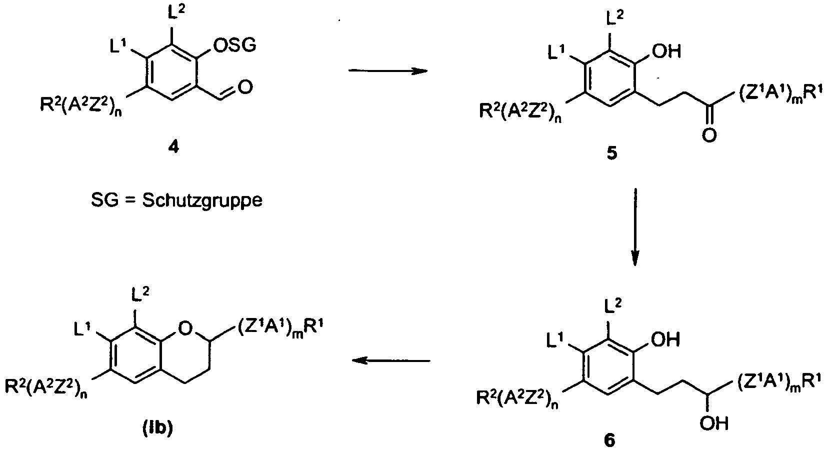

Als Ausgangsmaterial für die Verbindungen **3** und **4** können Salicylaldehyde verwendet werden. Ein mögliches Verfahren zur Herstellung dieser Salicylaldehyde ist die Umsetzung von kommerziellen Flüssigkristallvorstufen **7** gemäß dem folgenden Schema 3.

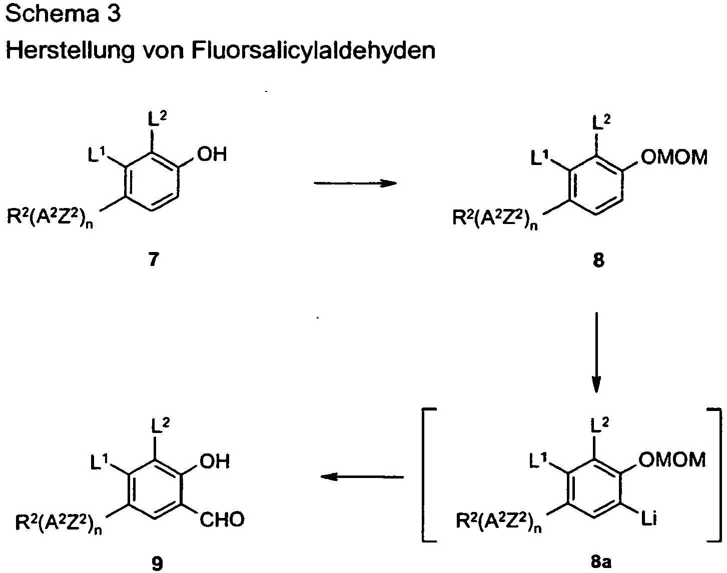

Nach Überführen der Phenole **7** in ein geeignetes Derivat, z.B. MOM-Ether **8,** können durch ortho-Metallierung, Abfangen mit einem Formamidderivat, wie z.B. DMF, und nachfolgendes Entschützen direkt die Salicylaldehyde **9** erhalten werden, wie z.B. von I.R. Hardcastle, P. Quayle, E.L.M. Ward in Tetrahedron Lett. 1994, 35, 1747-1748 beschrieben.

Alternativ können die Phenole **7** auch zunächst halogeniert und anschließend nach Schutz der Hydroxylgruppe durch Halogen-Lithium-Austausch metalliert und analog Schema 4 zu Salicylaldehyden umgesetzt werden, wie z.B. von G.C. Finger, M.J. Gortakowski, R.H. Shiley, R.H. White in J. Amer. Chem. Soc. 1959, 81, 94-101 beschrieben und in Schema 4 dargestellt.

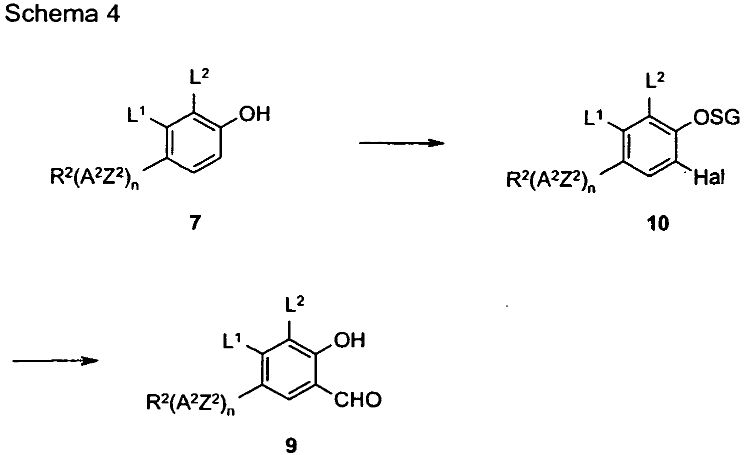

Die Herstellung der erfindungsgemäßen Chroman-Derivate der allgemeinen Formel (II), erfolgt vorzugsweise dadurch, dass man
a) ein Oxetan der allgemeinen Formel (Vlla) oder (Vllb) 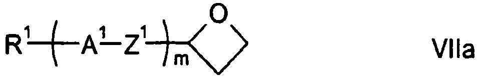 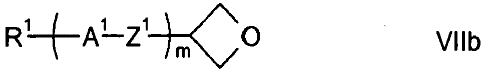 worin R¹, A¹, Z¹ und m die in Bezug auf Formel (II) angegebenen Bedeutungen haben, mit einem in ortho-Position metallierten Fluoraromaten in einem organischen Lösungsmittel und bei tiefen Temperaturen zu dem entsprechenden Propanolderivat der allgemeinen Formel (VIIIa) oder (VIIIb) umsetzt, 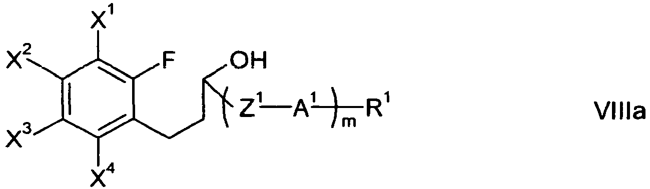 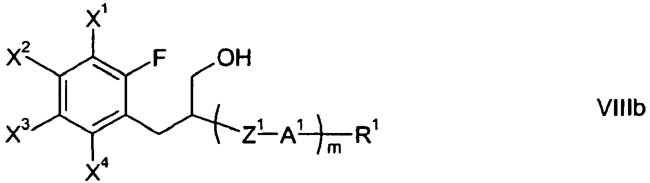 worin R¹, A¹, X¹, X², X³, X⁴, Z¹ und m die in Bezug auf Formel (II) angegebenen Bedeutungen haben, und
b) das gebildete Propanolderivat der allgemeinen Formel (VIIIa) oder (Vlllb) durch Einwirkung einer starken, nicht nucleophilen Base zum entsprechenden Chromanderivat der allgemeinen Formel II cyclisiert.

Das auf diese Weise erhaltene Chroman-Derivat lässt sich gegebenenfalls durch Dehydrierung zum entsprechenden Chromen-Derivat umsetzen.

Die Umsetzung in Schritt a) erfolgt dabei vorzugsweise in Gegenwart einer Lewissäure. Als Lewissäuren können dabei prinzipiell alle dem Fachmann bekannten Verbindungen eingesetzt werden, sofern sie keine aciden Protonen aufweisen. Besonders bevorzugt sind starke Lewissäuren, insbesondere BF₃-Etherat. Im Fall besonders reaktiver Verbindungen kann die Umsetzung auch ohne den Zusatz einer Lewissäure erfolgen.

Als organische Lösungsmittel können in Schritt a) alle dem Fachmann für diesen Zweck geeigneten Lösungsmittel eingesetzt werden. Bevorzugt als Lösungsmittel sind jedoch Diethylether, Tetrahydrofuran (THF) und Dimethoxyethan (DME) sowie Gemische derselben.

Unter tiefer Temperatur wird in der vorliegenden Anmeldung eine Temperatur im Bereich von -40°C bis -100°C, vorzugsweise von -65°C bis -85°C verstanden.

Die Herstellung der Oxetane kann dabei nach allen dem Fachmann bekannten Verfahren erfolgen. Vorzugsweise geht man aber von Diolen der folgenden Formeln aus, die entweder kommerziell erhältlich sind oder leicht herstellbar sind. Ein Verfahren zu deren Herstellung wird z.B. in der EP 0 967 261 B1 beschrieben. Diese Diole können dann beispielsweise nach dem von Picard et al., in: Synthesis, 1981, 550-552, beschriebenen Verfahren in Oxetane überführt werden, wie im folgenden Schema 5 dargestellt ist.

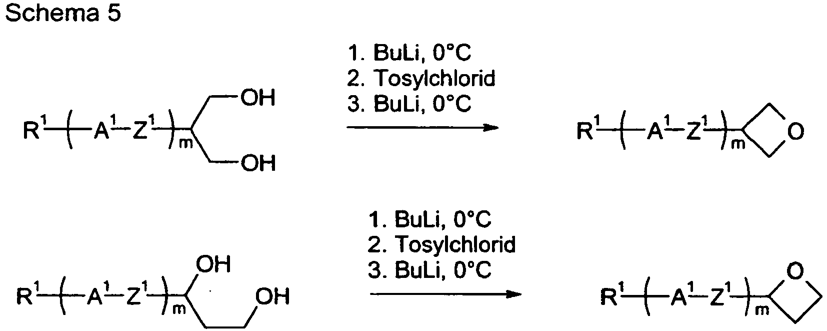

Auch die Herstellung der ortho-metallierten Fluoraromaten kann nach allen dem Fachmann bekannten Verfahren erfolgen. Bevorzugte Verfahren sind jedoch die ortho-Metallierung von Fluoraromaten mit Butyllithium (BuLi), gegebenenfalls unter Zusatz von TMEDA oder ähnlichen Verbindungen zur Erhöhung der Reaktivität des aggregierten Butyllithiums, Schlosser-Lochmann-Base oder Lithiumdiisopropylamid (LDA) jeweils bei tiefen Temperaturen oder der Halogen-Metallaustausch von Jod-Fluoraromaten oder Brom-Fluoraromaten mit BuLi bei tiefen Temperaturen (z.B. nach Org. React. 6, 1951, 339-366) oder mit iso-Propylmagnesiumchlorid bei Temperaturen im Bereich von -50°C bis -10°C (Knochel et al., Angewandte Chemie, Int. Ed. 42, 2003, 4302-4320).

Gegebenenfalls kann sich an diesen Schritt auch eine Ummetallierung anschließen. So lassen sich Lithiumaromaten durch Reaktion mit einer ZnCl₂-Lösung leicht in die entsprechenden Zinkaromaten überführen.

Der ortho-metallierte Fluoraromat wird dann in einem organischen Lösungsmittel bei tiefer Temperatur mit dem Oxetan umgesetzt, vorzugsweise in Gegenwart einer Lewissäure, wie in den beiden Schemata 6a und 6b dargestellt.

Je nach verwendetem Oxetan können auf diese Weise auch die strukturisomeren Alkohole erhalten werden.

Die Öffnung des Oxetans erfolgt dabei mit hoher Regioselektivität an der weniger stark substituierten Seite.

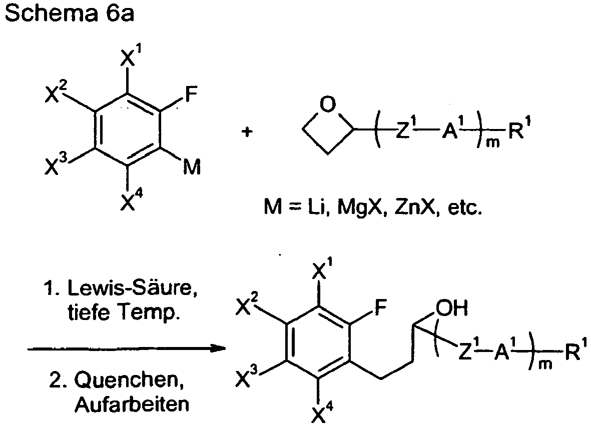

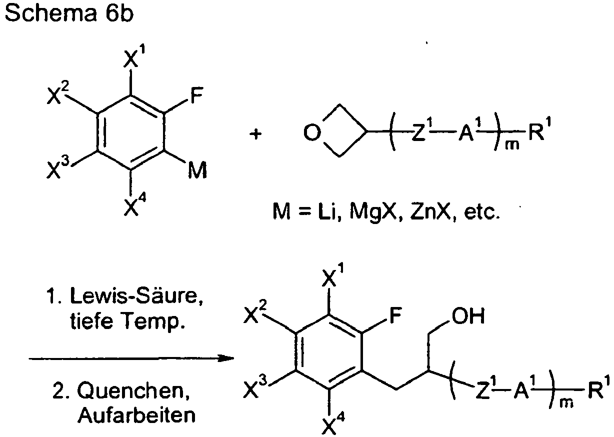

Das aus dem ortho-metallierten Fluoraromaten und dem Oxetan gebildete Propanolderivat wird anschließend in Gegenwart etwa 1 Äquivalents einer starken, nicht nucleophilen Base, z.B. Alkalihydrid, ausgewählt aus NaH, KH, RbH oder CsH, und Kaliumhexamethyldisilazan (KHMDS), vorzugsweise Alkalihydrid, besonders bevorzugt KH, in einem organischen Lösungsmittel intramolekular cyclisiert. Die Reaktion ist im folgenden Schema 7 dargestellt. Vorzugsweise erfolgt diese Cyclisierung in einem Temperaturbereich zwischen 0°C und 78°C. Besonders bevorzugt ist die Verwendung von 1 bis 1,5 Äquivalenten Kaliumhydrid (KH) in Tetrahydrofuran (THF). 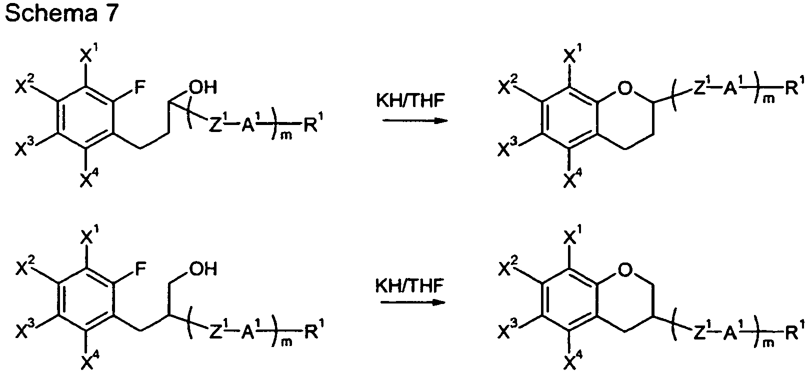 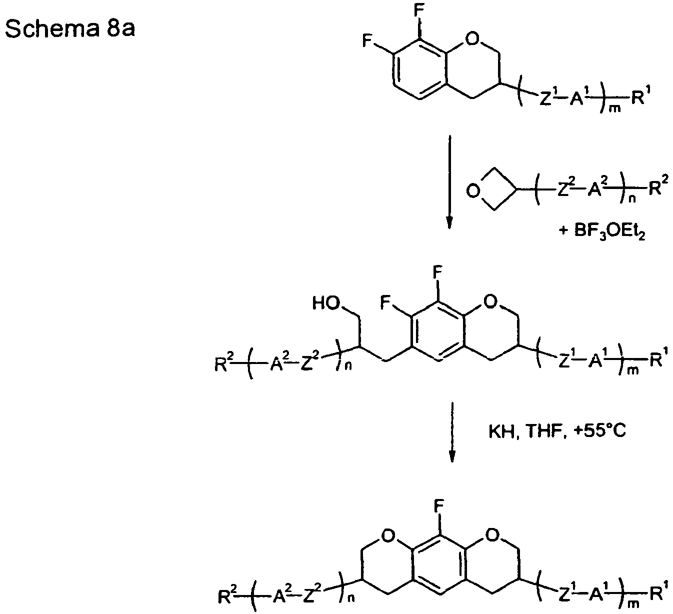 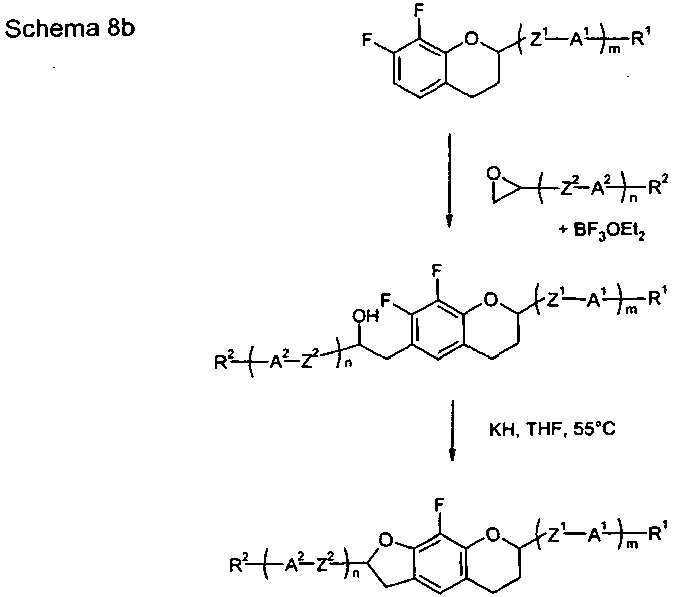 Die auf diese Weise erhaltenen Produkte können gegebenenfalls erneut als Edukte eingesetzt werden. Auf diese Weise lassen sich bei geeigneter Fluorsubstitution auch erfindungsgemäße Verbindungen mit zwei Heterocyclen aufbauen, wie in den beiden obigen Reaktionsschemata 8a und 8b dargestellt.

An die Cyclisierungsreaktionen können sich weitere Reaktionen anschließen, z.B. die Funktionalisierung des aromatischen Restes durch Einführung von weiteren Halogensubstituenten, wie zum Beispiel Chlor, Brom oder Iod, oder durch Einführung von Boronsäuregruppen nach literaturbekannten Verfahren.

Entsprechende Reaktionsbeispiele zeigt das folgende Schema 9:

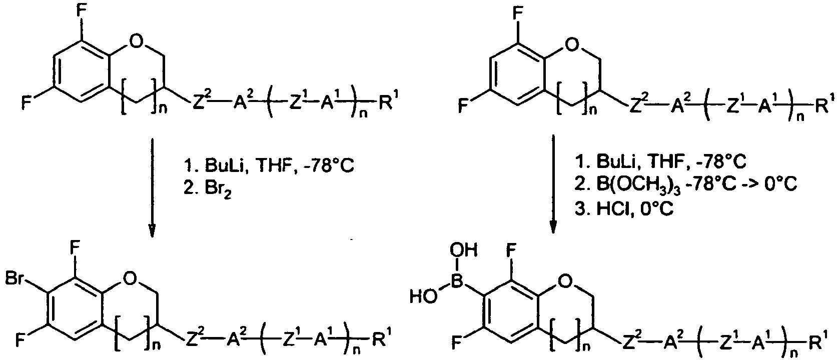

Eine bevorzugte Synthese zum Aufbau von arylsubstituierten Fluor-Benzochroman-Derivaten der allgemeinen Formel (la) erfolgt durch Suzuki-Kupplung von entsprechenden Boronsäuren oder Boronsäureestern mit 7-Brom-8-fluor-chromanen oder 7-Brom-6,8-difluor-chromanen nach dem folgenden Schema 10. Die benötigten Boronsäurederivate werden aus bromsubstituierten Vorstufen nach bekannten Methoden hergestellt, wie zum Beispiel im J. Org. Chem. 1995, 60, 7508-7510 offenbart. Die Synthese kann durch die Wahl geeigneter Ausgangsprodukte an die jeweils gewünschten Verbindungen der allgemeinen Formel (la) angepasst werden. Auf diese Weise lassen sich unter anderem die besonders bevorzugten Verbindungen der Teilformeln (Ia1a) und (la2b) darstellen.

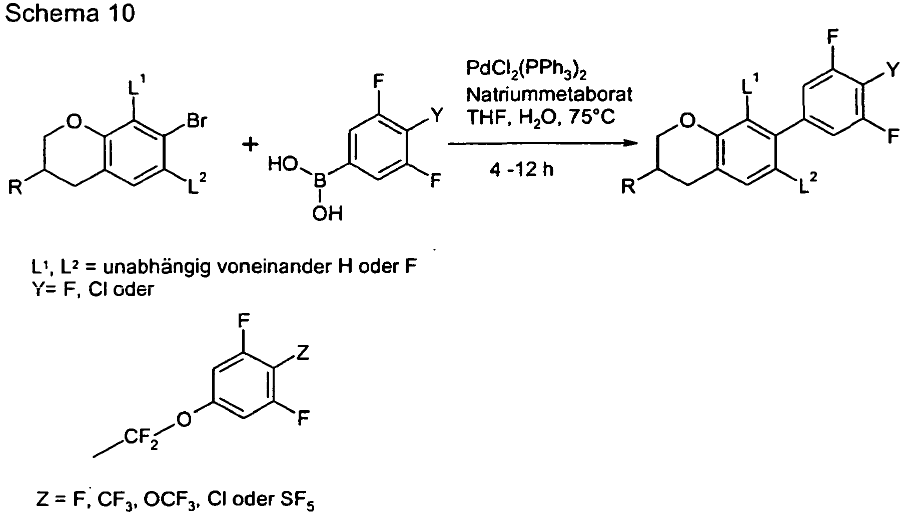

Die dargestellten Reaktionen sind nur als beispielhaft aufzufassen. Der Fachmann kann entsprechende Varianten der vorgestellten Synthesen vornehmen sowie auch andere geeignete Synthesewege beschreiten, um die erfindungsgemäßen Verbindungen der Formeln (I) bis (VI) zu erhalten.

Die Synthesen verschiedener erfindungsgemäßer Chroman-Derivate werden außerdem exemplarisch in den Beispielen beschrieben.

Gegenstand der vorliegenden Erfindung sind auch flüssigkristalline Medien enthaltend neben einer oder mehreren erfindungsgemäßen Verbindungen der Formeln (I) bis (VI) als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch ein- oder mehrfach fluoriert sein.

Die wichtigsten als weitere Bestandteile der erfindungsgemäßen Medien in Frage kommenden Verbindungen lassen sich durch die Formeln 1, 2, 3, 4, 5 und 6 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

R'-L-CF₂O-E-R" 6

In den Formeln 1, 2, 3, 4, 5 und 6 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4, 5 und 6, worin L und E ausgewählt sind aus der Gruppe Cyc und Phe und gleichzeitig eine oder mehrere Komponenten ausgewählt aus den Verbindungen der Formeln 1, 2, 3, 4, 5 und 6, worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4, 5 und 6, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS, -(O)ᵢCH₃₋₍ₖ₊ₗ₎FₖCl_{l,} wobei i 0 oder 1, k und l unabhängig voneinander, gleich oder verschieden, 0, 1, 2 oder 3 sind, jedoch mit der Maßgabe, dass die Summe (k + l) 1, 2 oder 3 ist.

R' und R" bedeuten in einer kleineren Untergruppe der Verbindungen der Formeln 1, 2, 3, 4, 5 und 6 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a, 5a und 6a bezeichnet. Bei den meisten dieser Verbindungen sind R' und R" voneinander verschieden, wobei einer dieser Reste meist Alkyl, Alkenyl, Alkoxy oder Alkoxyalkyl ist.

In der als Gruppe A bezeichneten kleineren Untergruppe der Verbindungen der Formeln 1, 2, 3, 4, 5 und 6 bedeutet E in einer bevorzugten Ausführungsform

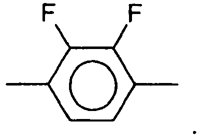

In einer anderen als Gruppe B bezeichneten kleineren Untergruppe der Verbindungen der Formeln 1, 2, 3, 4, 5 und 6 bedeutet R" -F, -Cl, -NCS oder -(O)ᵢ CH₃-(ₖ₊ₗ) FₖClₗ, wobei i 0 oder 1, k und l unabhängig voneinander 0, 1, 2 oder 3 sind, jedoch mit der Maßgabe, dass die Summe (k + l) 1, 2 oder 3 ist. Die Verbindungen, in denen R" diese Bedeutung hat, werden mit den Teilformeln 1 b, 2b, 3b, 4b, 5b und 6b bezeichnet. Besonders bevorzugt sind solche Verbindungen der Teilformeln 1 b, 2b, 3b, 4b, 5b und 6b, in denen R" die Bedeutung -F, -Cl, -NCS, -CF₃, -OCHF₂ oder -OCF₃ hat.

In den Verbindungen der Teilformeln 1b, 2b, 3b, 4b, 5b und 6b hat R' die bei den Verbindungen der Teilformeln 1a bis 6a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4, 5 und 6 bedeutet R" -CN. Diese Untergruppe wird im folgenden als Gruppe C bezeichnet und die Verbindungen dieser Untergruppe werden entsprechend mit Teilformeln 1 c, 2c, 3c, 4c, 5c und 6c beschrieben. In den Verbindungen der Teilformeln 1c, 2c, 3c, 4c, 5c und 6c hat R' die bei den Verbindungen der Teilformeln 1a bis 6a angegebene Bedeutung und ist vorzugsweise Alkyl, Alkenyl, Alkoxy oder Alkoxyalkyl.

Neben den bevorzugten Verbindungen der Gruppen A, B und C sind auch andere Verbindungen der Formeln 1, 2, 3, 4, 5 und 6 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formeln (I), (II), (III), (IV), (V) und/oder (VI) vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:
- Gruppe A:: 0 bis 90 %, vorzugsweise 20 bis 90 %, besonders bevorzugt 30 bis 90 %;
- Gruppe B:: 0 bis 80 %, vorzugsweise 10 bis 80 %, besonders bevorzugt 10 bis 70 %;
- Gruppe C:: 0 bis 80 %, vorzugsweise 5 bis 80 %, besonders bevorzugt 5 bis 50 %;
wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen. Desweiteren bevorzugt sind Medien, enthaltend mehr als 40 %, besonders bevorzugt 45 bis 90 %, an erfindungsgemäßen Verbindungen. Die Medien enthalten vorzugsweise eine, zwei, drei, vier oder fünf erfindungsgemäße Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z.B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe und/oder chirale Dotierstoffe zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. n und m bedeuten ganze Zahlen, vorzugsweise 1, 2, 3, 4, 5, 6, 7, 8, 9 und 10, wobei n = m oder n ≠ m sein kann. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R^{1*}, R^{2*}, L^{1*} und L^{2*}:

| Code für R^{1*}, R^{2*}, L^{1*}, L^{2*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nOF | OCₙH₂ₙ₊₁ | F | H | H |
| nF.F | CₙH₂ₙ₊₁ | F | F | H |
| nmF | CₙH₂ₙ₊₁ | CₘH₂ₘ₊₁ | F | H |
| nOCF₃ | CₙH₂ₙ₊₁ | OCF₃ | H | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A:**

| | |
|---|---|
| 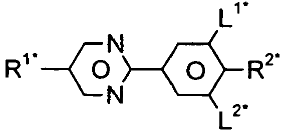 | 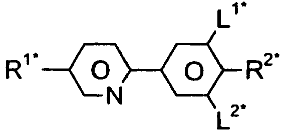 |
| **PYP** | **PYRP** |
| 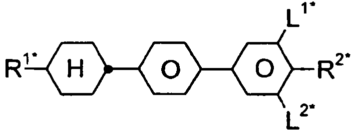 | 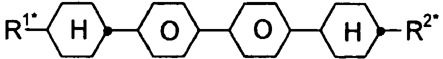 |
| **BCH** | **CBC** |
| 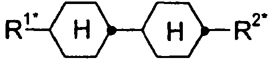 | 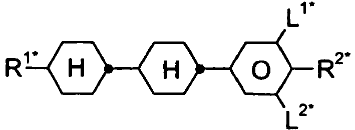 |
| **CCH** | **CCP** |
| 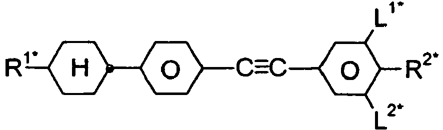 | 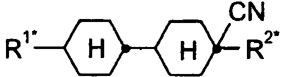 |
| **CPTP** | **CCN** |
| 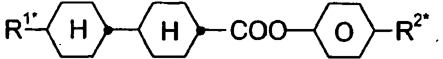 | |
| **CP** | |
| 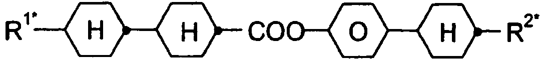 | |
| **CCPC** | |
| 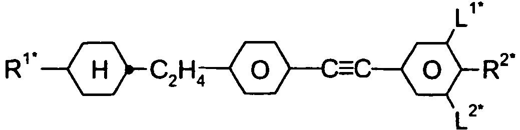 | |
| **CEPTP** | |
| 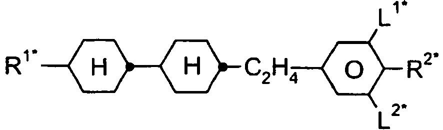 | 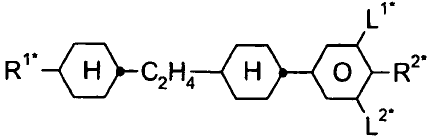 |
| **ECCP** | **CECP** |
| 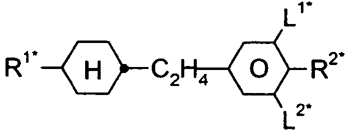 | 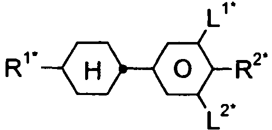 |
| **EPCH** | **PCH** |
| 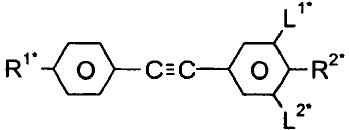 | 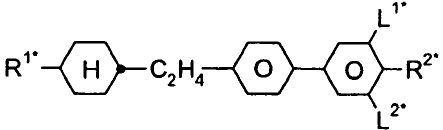 |
| **PTP** | **BECH** |
| 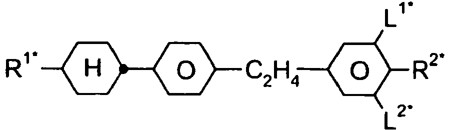 | 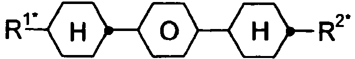 |
| **EBCH** | **CPC** |
| 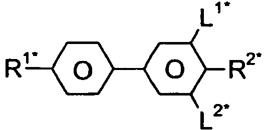 | 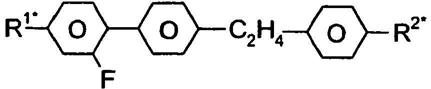 |
| **B** | **FET-nF** |
| 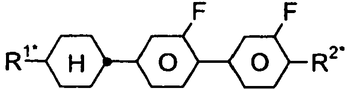 | 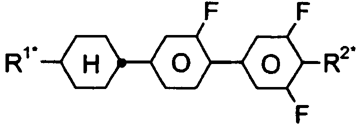 |
| **CGG** | **CGU** |
| 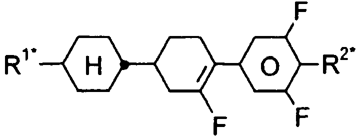 | |
| **CFU** | |

**Tabelle B:**

| | |
|---|---|
| 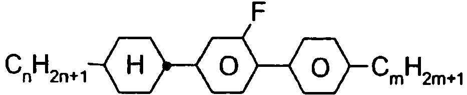 | 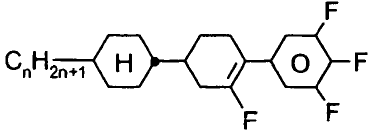 |
| **BCH-n.Fm** | **CFU-n-F** |
| 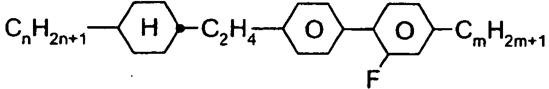 | |
| **I-nm** | |
| 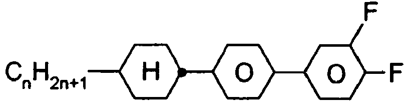 | 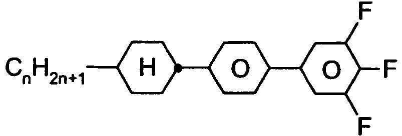 |
| **BCH-nF.F** | **BCH-nF.F.F** |
| 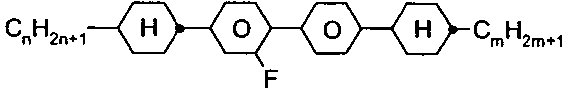 | |
| **CBC-nmF** | |
| 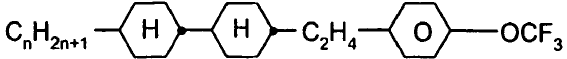 | |
| **ECCP-nOCF₃** | |
| 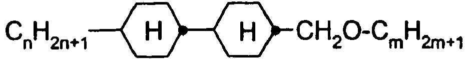 | |
| **CCH-n1Em** | |
| 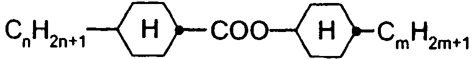 | |
| **OS-nm** | |
| 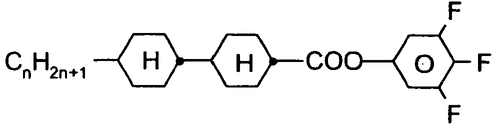 | |
| **CCZU-n-F** | |
| 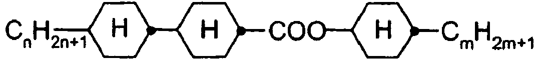 | 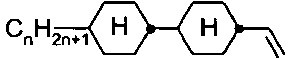 |
| **CH-nm** | **CC-n-V** |
| 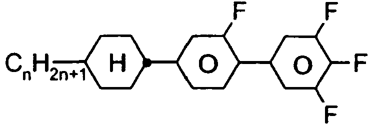 | 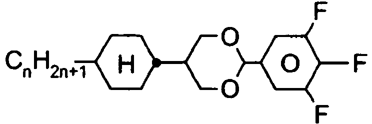 |
| **CGU-n-F** | **CDU-n-F** |
| 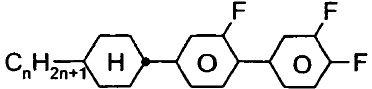 | 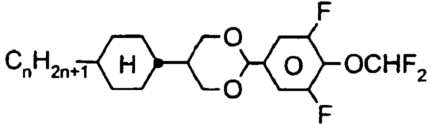 |
| **CGG-n-F** | **CDU-n-OD** |
| 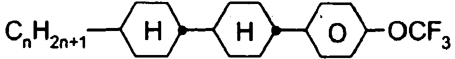 | 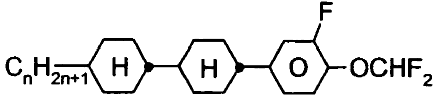 |
| **CCP-nOCF₃** | **CCP-nOCF₂.F** |
| 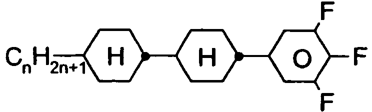 | 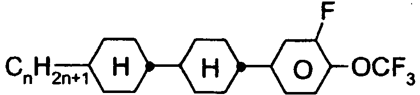 |
| **CCP-nF.F.F** | **CCP-nOCF₃.F** |
| 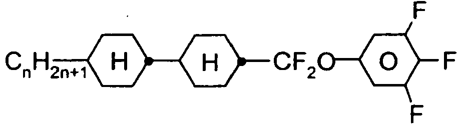 | 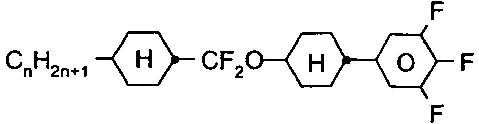 |
| **CCQU-n-F** | **CQCU-n-F** |
| 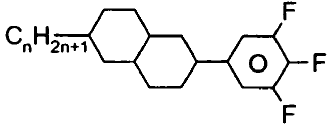 | 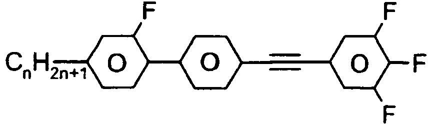 |
| **Dec-U-n-F** | **GPTU-n-F** |
| 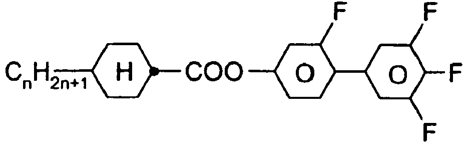 | 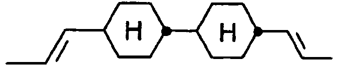 |
| **CZGU-n-F** | **CC-1V-V1** |
| 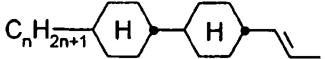 | 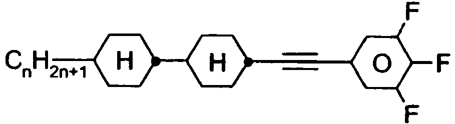 |
| **CC-n-V1** | **CCTU-n-F** |
| 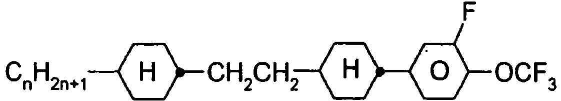 | |
| **CECG-n-OT** | |
| 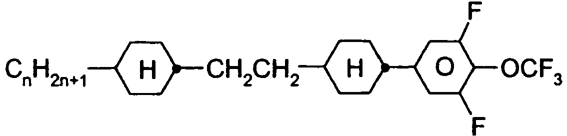 | |
| **CECU-n-OT** | |
| 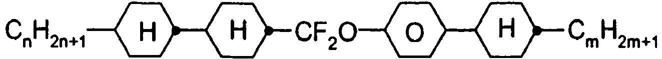 | |
| **CCQPC-n-m** | |

**Tabelle C:**

| In der Tabelle C werden mögliche Dotierstoffe angegeben, die vorzugsweise den erfindungsgemäßen Mischungen zugesetzt werden. | |
|---|---|
| 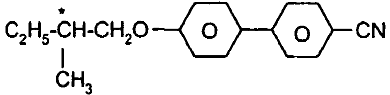 | 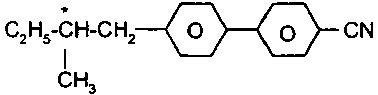 |
| **C 15** | **CB 15** |
| 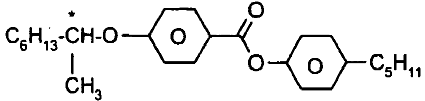 | |
| **CM 21** | |
| 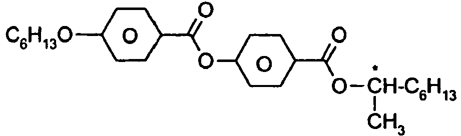 | |
| **CM 33** | |
| 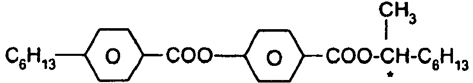 | |
| **R/S 811** | |
| 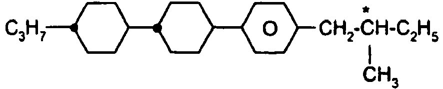 | |
| **CM 44** | |
| 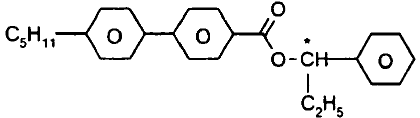 | |
| **CM 45** | |
| 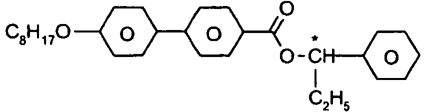 | |
| **CM 47** | |
| 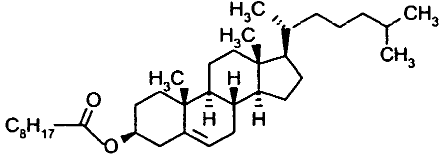 | |
| **CN** | |
| 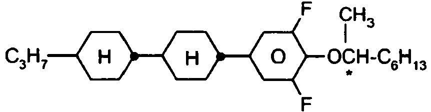 | |
| **R/S 2011** | |

Besonders bevorzugt sind erfindungsgemäße Mischungen, die neben einer oder mehreren Verbindungen der Formeln (I), (II), (III), (IV), (V) und/oder (VI) zwei, drei oder mehr Verbindungen ausgewählt aus den Tabellen A und/oder B enthalten.

Die folgenden Beispiele sollen die Erfindung erläutern, ohne sie zu beschränken. Vor- und nachstehend bedeuten Prozentangaben Gewichtsprozent. Alle Temperaturen sind in Grad Celsius angegeben. Tg ist die Glasübergangstemperatur und Klp. ist der Klärpunkt. Ferner bedeuten K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20°C), Δε die dielektrische Anisotropie (1 kHz, 20°C) und γ₁ die Rotationsviskosität bei 20°C [mPas].

Die Δn- und Δε-Werte der erfindungsgemäßen Verbindungen wurden durch Extrapolation aus flüssigkristallinen Mischungen erhalten, die zu 10 % aus der jeweiligen erfindungsgemäßen Verbindung und zu 90 % entweder aus dem kommerziell erhältlichen Flüssigkristall ZLI 4792 (Δn - und positive Δε-Werte) oder dem ebenfalls kommerziell erhältlichen Flüssigkristall ZLI 2857 (negative Δε-Werte), beide Fa. Merck, Darmstadt, bestanden.

Vor- und nachstehend werden folgende Abkürzungen verwendet:
- AlBN: Azoisobuttersäurenitril
- BuLi: Butyllithium
- DCM: Dichlormethan
- EE: Essigester
- KH: Kaliumhydrid
- KHMDS: Kaliumhexamethyldisilazan
- LDA: Lithiumdiisopropylamid
- MCPBA: 3-Chlorperoxybenzoesäure
- MTBE: tert.-Butylmethylether
- NBS: N-Bromsuccinimid
- RT: Raumtemperatur
- THF: Tetrahydrofuran
- TMEDA: Tetramethylethylendiamin

### Beispiel 1

### trans 3-(4-Ethyl-cyclohexyl)-oxetan

### Die Verbindung der folgenden Formel

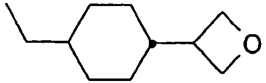

wird wie folgt hergestellt:

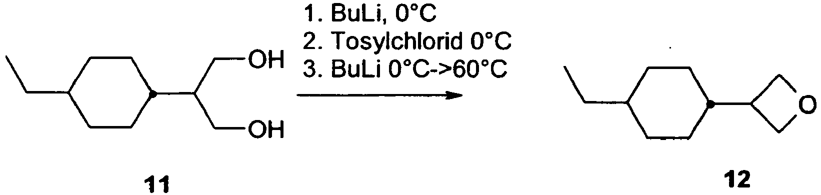

Zu einer Lösung von 20 g Diol (**11**) in 100 ml THF werden bei 0°C 67,4 ml BuLi (15% in Hexan) getropft. Nach 30 Minuten wird eine Lösung von 19 g Tosylchlorid in 100 ml THF zugetropft (exotherm, Erwärmung auf ca 40°C) und die resultierende Mischung 1 Stunde bei Raumtemperatur gerührt, bevor weitere 67,4 ml BuLi unter Eiskühlung zugegeben werden. Die Reaktion wird 4 Stunden bei 60°C erhitzt. Das THF wird am Rotationsverdampfer entfernt, der Rückstand mit Wasser und MTBE behandelt, die organische Phase abgetrennt, getrocknet und einrotiert. Reinigung des Rückstands durch Säulenchromatographie (Heptan/EE 6:1) ergibt 11,1 g eines farblosen Öls (**12**).
Ausbeute: 61 %

Analog Beispiel 6 werden unter Verwendung der entsprechenden Vorstufen die folgenden Verbindungen der Beispiele 7 und 8 erhalten:

### Beispiel 2

### trans 3-(4-n-Propyl-cyclohexyl)-oxetan

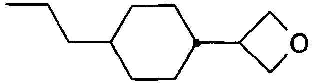

Ausbeute: 68%

### Beispiel 3

### trans 3-(4'-n-Propyl-bicyclohexyl-4-yl)-oxetan

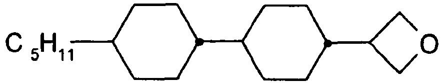

Ausbeute: 57%

### Beispiel 4 (Ortho-Lithiierung)

### trans-2-(4-Ethyl-cyclohexyl)-3-(2,3,4-trifluoro-phenyl)-propan-1-ol

Die Verbindung der folgenden Formel 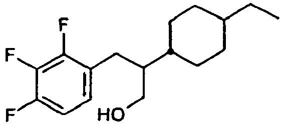 wird wie folgt hergestellt:

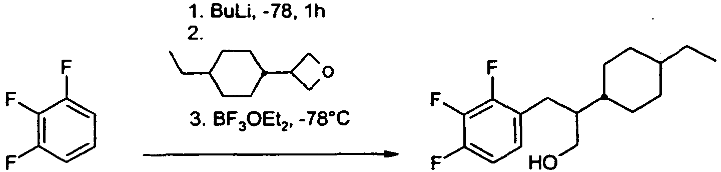

Zu einer Lösung von 8,7 g Trifluorbenzol in 100 ml THF werden unter Stickstoff bei -78°C 40 ml BuLi (15% in Hexan) langsam zugetropft und 1 Stunde bei dieser Temperatur nachgerührt. Der tiefgelben Lösung werden zuerst 7,6 g des angegebenen Oxetans (HPLC-Gehalt 90 % trans, 10 % cis) zugespritzt und nach 15 Minuten tropfenweise 5,1 ml BF₃-Etherat. Bei der Zugabe des BF₃-Etherats muß gut gekühlt werden, um die Reaktionstemperatur unter -70°C zu halten. Nach 60 Minuten Nachrühren bei -80°C (DC-Kontrolle, vollständiger Umsatz) wird mit 50 ml Ammoniumchloridlösung bei -78°C gequencht. Die aufgetaute Reaktionsmischung wird mit MTBE versetzt, mit 2N HCl schwach angesäuert, die wässrige Phase abgetrennt und diese mehrfach mit MTBE nachextrahiert. Die vereinigten organischen Phasen werden mit Wasser und gesättigter Natriumchloridlösung gewaschen, über Natriumsulfat getrocknet und einrotiert. Reinigung des Rückstands durch Chromatographie über 500 ml Kieselgel (Eluent: Toluol) ergibt 10,7 g eines farbloses Öls.
Nach HPLC ist der Gehalt der gewünschten trans-Verbindung 81%.
Ausbeute: 71%.

Analog Beispiel 4 werden unter Verwendung der entsprechenden Oxetan-Vorstufen die folgenden Verbindungen der Beispiele 5 bis 12 erhalten. Wird dabei der lithiierte Aromat durch Halogen-Metall-Austausch hergestellt, so wird als Lösungsmittel Diethylether verwendet.

| Beispiel | Ausgangsverbindung | Lösungsmittel | Verbindung | Ausbeute [%] |
|---|---|---|---|---|
| 5 | 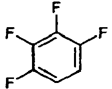 | THF | 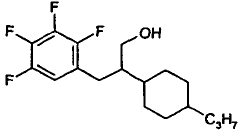 | 85 |
| 6 | 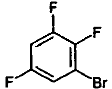 | Diethylether | 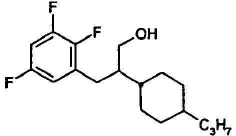 | 95 |
| 7 | 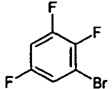 | Diethylether | 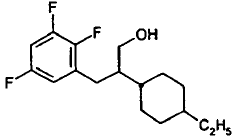 | 99 |
| 8 | 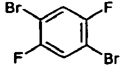 | Diethylether | 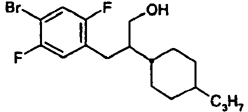 | 83 |
| 9 | 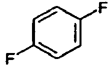 | THF | 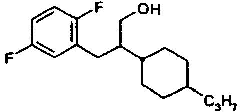 | 94 |
| 10 | 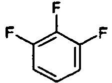 | Diethylether | 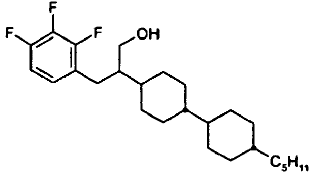 | 79 |
| 11 | 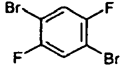 | Diethylether | 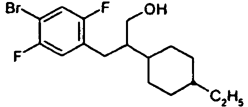 | 91 |
| 12 | 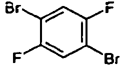 | Diethylether | 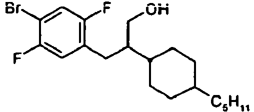 | 62 |

### Beispiel 13 (Cyclisierung)

### trans-7,8-Difluoro-3-(4-(ethyl)-cyclohexyl)-chroman

Die Verbindung der folgenden Formel 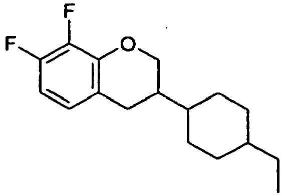 wird wie folgt hergestellt:

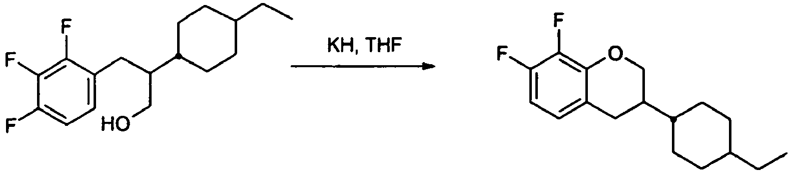

Zu einer Suspension von 4,6 g KH (30% in Paraffinöl) in 500 ml THF wird unter N₂ bei 40°C eine Lösung von 10 g des Alkohols (Gehalt 95%) in 250 ml THF langsam zugetropft. Nach weiteren 2 Stunden bei 55°C ist die Reaktion laut DC-Kontrolle vollständig. Man quencht mit 10 ml gesättigter Ammoniumchloridlösung, entfernt den größten Teil des THFs, versetzt mit Toluol, extrahiert mit Wasser und trennt die organische Phase ab. Die wässrige Phase wird noch dreimal mit Toluol nachextrahiert. Die vereinigten organischen Phasen werden über Natriumsulfat getrocknet, einrotiert und der Rückstand durch Säulenchromatographie (Heptan/Toluol 19:1) gereinigt. Man erhält 6,8 g eines farblosen Feststoffs.
Ausbeute: 76%

Durch Umkristallisation erhält man die reine trans-Verbindung:
K 82 I
Δn: 0,0729
Δε: 11,8
Klp.: -9,1°C

Analog werden unter Verwendung der entsprechenden Vorstufen die folgenden Verbindungen der Beispiele 14 bis 20 erhalten:

| Beispiel | Ausgangsverbindung | Rk.-bed., Lösungsmittel | Verbindung | Ausbeute [%] |
|---|---|---|---|---|
| 14 | 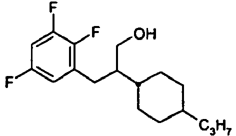 | 4h/40°C KH/THF | 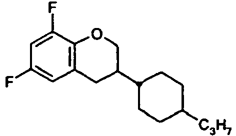 | 23 |
| | | 5h/50°C KH/THF | | 51 |
| 15 | 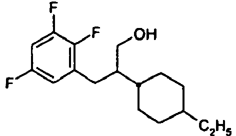 | 5h/60°C KH/THF | 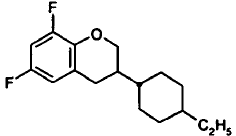 | 49 |
| 16 | 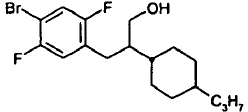 | 6h/50°C KH/THF | 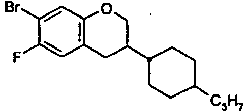 | 87 |
| 17 | 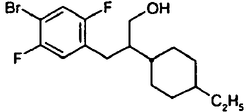 | 3h/50°C KH/THF | 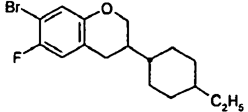 | 80 |
| 18 | 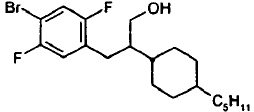 | 16h/55°C NaH/THF | 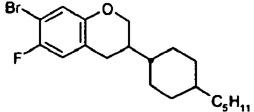 | 30 |
| | | 4h/120°C NaH/Triglyme | | 30 |
| | | 4h/55°C KH/THF | | 81 |
| 19 | 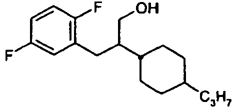 | 4h/50°C | 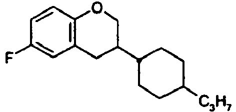 | 90 |
| | | 16h/60°C | | |
| 20 | 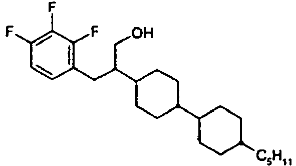 | 1h/40°C | 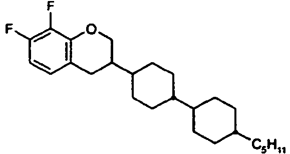 | 55 |
| | | 8h/60°C | | |

### Beispiel 21

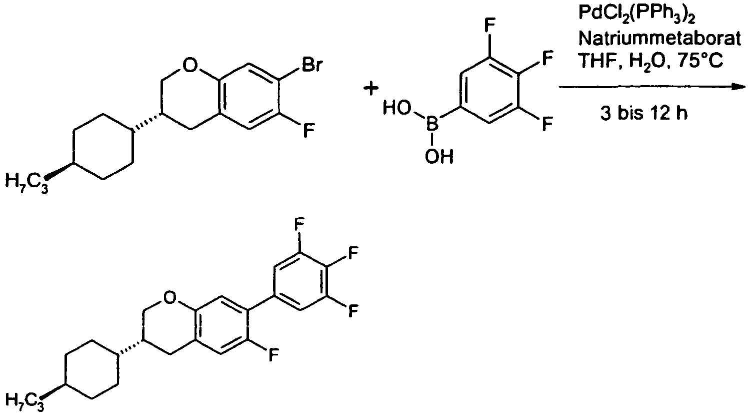

Unter Stickstoff werden 1,01 g 3,4,5-Trifluorphenylboronsäure (1,1 Äquiv.), 2,00 g 6-Fluor-3-(4-propyl-cyclohexyl)-7-Brom-chroman (1,0 Äquiv.), 1,3 g Natriummetaborat-Octahydrat (0,84 Äquiv.) und 141 mg Bis(Triphenylphosphin)palladium(II)chlorid (3,5 mol %) in 20 ml THF und 5 ml Wasser suspendiert. Man erhitzt unter starkem Rühren solange auf 75°C bis das Brom-Chroman vollständig umgesetzt ist (3 bis 12 Stunden). Nach dem Abkühlen wird die wässrige Phase abgetrennt und dreimal mit MTBE nachextrahiert. Die vereinigten organischen Phasen werden mit Natriumchloridlösung gewaschen, getrocknet und einrotiert. Die Reinigung des Rohprodukts erfolgt durch Säulenchromatographie über Kieselgel mit Heptan/Toluol (6:1) als Eluent.

Man erhält 2,05 g Produkt mit einem Gehalt von 91 %, entsprechend einer Ausbeute von 82 %. Durch Umkristallisation aus Heptan/iso-Propanol werden 1,4 g Produkt mit einem Gehalt von >99,5 % erhalten.
K 102 N 115,1 I
Δn: 0,1397
Δε: 24,7
Klp.: 88,5°C
γ₁: 996 mPas

Analog Beispiel 21 werden unter Verwendung der entsprechenden Vorstufen die folgenden Verbindungen der Beispiele 22 bis 26 erhalten:

### Beispiel 22

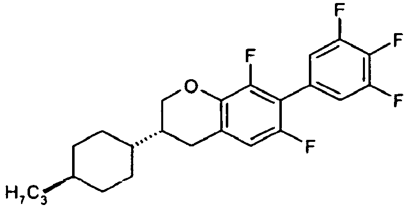

Ausbeute: 68 %
K 101 N (81,9) I
Δn: 0,1305
Δε: 27,4
Klp.: 76,3°C

### Beispiel 23

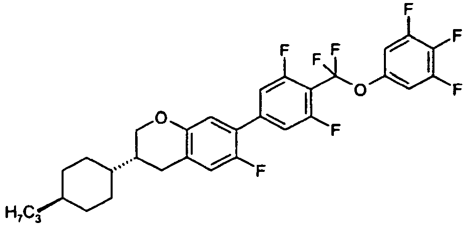

Ausbeute: 86 %
K 100 N 180,1 I
Δn: 0,1442
Δε: 35,1
Klp.: 165,7°C
γ₁: 1261 mPas

### Beispiel 24

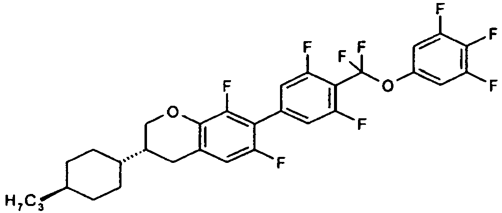

Ausbeute: 72 %
K 106 N 166,6 I
Δn: 0,1320
Δε: 36,2
Klp.: 155,4°C

### Beispiel 25

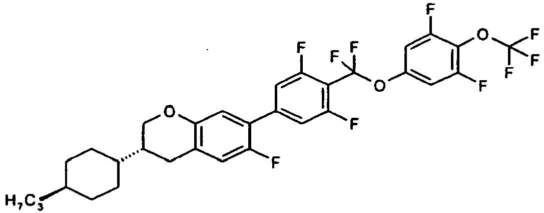

Ausbeute: 88 %
K 95 SmA 141 N 190 I
Δn: 0,1388
Δε: 35,8
Klp.: 177,6°C

### Beispiel 26

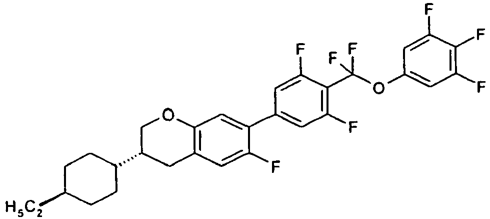

Ausbeute: 71 %
K 109 N 161,5 I
Δn: 0,1404
Δε: 34,8
Klp.: 132,0°C

Analog den Beispielen 1 bis 26 werden unter Verwendung der entsprechenden Vorstufen die folgenden Verbindungen der Beispiele 27 bis 1845 erhalten:

### Beispiele 27 bis 71

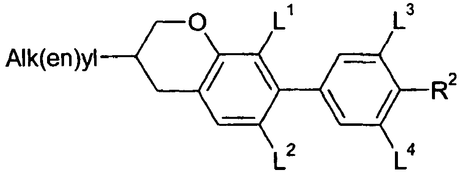

### Beispiele 72 bis 116

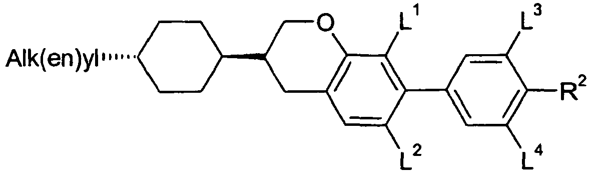

### Beispiele 117 bis 161

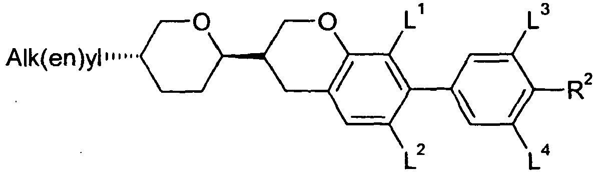

### Beispiele 162 bis 206

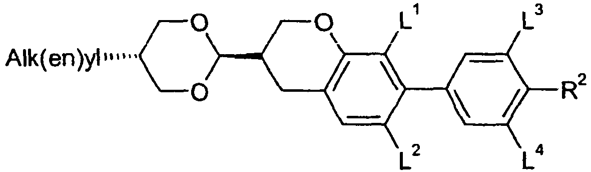

| **Beispiele** | | | | **L¹** | **L²** | **L³** | **L⁴** | **R²** |
|---|---|---|---|---|---|---|---|---|
| 27, | 72, | 117, | 162, | H | H | H | H | F |
| 28, | 73, | 118, | 163, | H | H | H | F | F |
| 29, | 74, | 119, | 164, | H | F | H | H | F |
| 30, | 75, | 120, | 165, | F | F | H | H | F |
| 31, | 76, | 121, | 166, | H | H | F | F | F |
| 32, | 77, | 122, | 167, | H | F | H | F | F |
| 33, | 78, | 123, | 168, | H | F | F | F | F |
| 34, | 79, | 124, | 169, | F | F | H | F | F |
| 35, | 80, | 125, | 170, | F | F | F | F | F |
| 36, | 81, | 126, | 171, | H | H | H | H | CF₃ |
| 37, | 82, | 127, | 172, | H | H | H | F | CF₃ |
| 38, | 83, | 128, | 173, | H | F | H | H | CF₃ |
| 39, | 84, | 129, | 174, | F | F | H | H | CF₃ |
| 40, | 85, | 130, | 175, | H | H | F | F | CF₃ |
| 41, | 86, | 131, | 176, | H | F | H | F | CF₃ |
| 42, | 87, | 132, | 177, | H | F | F | F | CF₃ |
| 43, | 88, | 133, | 178, | F | F | H | F | CF₃ |
| 44, | 89, | 134, | 179, | F | F | F | F | CF₃ |
| 45, | 90, | 135, | 180, | H | H | H | H | OCF₃ |
| 46, | 91, | 136, | 181, | H | H | H | F | OCF₃ |
| 47, | 92, | 137, | 182, | H | F | H | H | OCF₃ |
| 48, | 93, | 138, | 183, | F | F | H | H | OCF₃ |
| 49, | 94, | 139, | 184, | H | H | F | F | OCF₃ |
| 50, | 95, | 140, | 185, | H | F | H | F | OCF₃ |
| 51, | 96, | 141, | 186, | H | F | F | F | OCF₃ |
| 52, | 97, | 142, | 187, | F | F | H | F | OCF₃ |
| 53, | 98, | 143, | 188, | F | F | F | F | OCF₃ |
| 54, | 99, | 144, | 189, | H | H | H | H | Cl |
| 55, | 100, | 145, | 190, | H | H | H | F | Cl |
| 56, | 101, | 146, | 191, | H | F | H | H | Cl |
| 57, | 102, | 147, | 192, | F | F | H | H | Cl |
| 58, | 103, | 148, | 193, | H | H | F | F | Cl |
| 59, | 104, | 149, | 194, | H | F | H | F | Cl |
| 60, | 105, | 150, | 195, | H | F | F | F | Cl |
| 61, | 106, | 151, | 196, | F | F | H | F | Cl |
| 62, | 107, | 152, | 197, | F | F | F | F | Cl |
| 63, | 108, | 153, | 198, | H | H | H | H | CN |
| 64, | 109, | 154, | 199, | H | H | H | F | CN |
| 65, | 110, | 155, | 200, | H | F | H | H | CN |
| 66, | 111, | 156, | 201, | F | F | H | H | CN |
| 67, | 112, | 157, | 202, | H | H | F | F | CN |
| 68, | 113, | 158, | 203, | H | F | H | F | CN |
| 69, | 114, | 159, | 204, | H | F | F | F | CN |
| 70, | 115, | 160, | 205, | F | F | H | F | CN |
| 71, | 116, | 161, | 206, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 207 bis 251

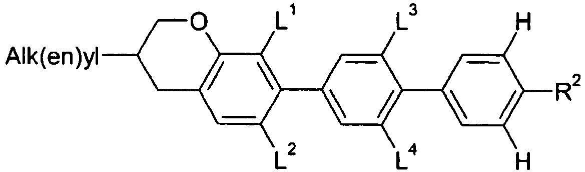

### Beispiele 252 bis 296

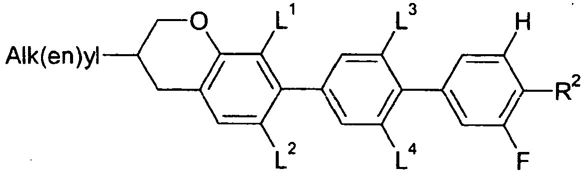

### Beispiele 297 bis 341

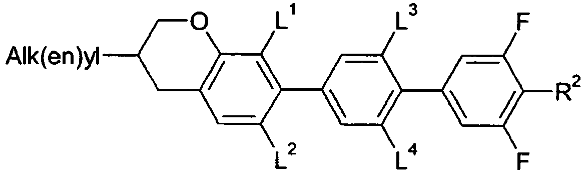

| **Beispiele** | | | **L¹** | **L²** | **L³** | **L⁴** | **R²** |
|---|---|---|---|---|---|---|---|
| 207, | 252, | 297, | H | H | H | H | F |
| 208, | 253, | 298, | H | H | H | F | F |
| 209, | 254, | 299, | H | F | H | H | F |
| 210, | 255, | 300, | F | F | H | H | F |
| 211, | 256, | 301, | H | H | F | F | F |
| 212, | 257, | 302, | H | F | H | F | F |
| 213, | 258, | 303, | H | F | F | F | F |
| 214, | 259, | 304, | F | F | H | F | F |
| 215, | 260, | 305, | F | F | F | F | F |
| 216, | 261, | 306, | H | H | H | H | CF₃ |
| 217, | 262, | 307, | H | H | H | F | CF₃ |
| 218, | 263, | 308, | H | F | H | H | CF₃ |
| 219, | 264, | 309, | F | F | H | H | CF₃ |
| 220, | 265, | 310, | H | H | F | F | CF₃ |
| 221, | 266, | 311, | H | F | H | F | CF₃ |
| 222, | 267, | 312, | H | F | F | F | CF₃ |
| 223, | 268, | 313, | F | F | H | F | CF₃ |
| 224, | 269, | 314, | F | F | F | F | CF₃ |
| 225, | 270, | 315, | H | H | H | H | OCF₃ |
| 226, | 271, | 316, | H | H | H | F | OCF₃ |
| 227, | 272, | 317, | H | F | H | H | OCF₃ |
| 228, | 273, | 318, | F | F | H | H | OCF₃ |
| 229, | 274, | 319, | H | H | F | F | OCF₃ |
| 230, | 275, | 320, | H | F | H | F | OCF₃ |
| 231, | 276, | 321, | H | F | F | F | OCF₃ |
| 232, | 277, | 322, | F | F | H | F | OCF₃ |
| 233, | 278, | 323, | F | F | F | F | OCF₃ |
| 234, | 279, | 324, | H | H | H | H | Cl |
| 235, | 280, | 325, | H | H | H | F | Cl |
| 236, | 281, | 326, | H | F | H | H | Cl |
| 237, | 282, | 327, | F | F | H | H | Cl |
| 238, | 283, | 328, | H | H | F | F | Cl |
| 239, | 284, | 329, | H | F | H | F | Cl |
| 240, | 285, | 330, | H | F | F | F | Cl |
| 241, | 286, | 331, | F | F | H | F | Cl |
| 242, | 287, | 332, | F | F | F | F | Cl |
| 243, | 288, | 333, | H | H | H | H | CN |
| 244, | 289, | 334, | H | H | H | F | CN |
| 245, | 290, | 335, | H | F | H | H | CN |
| 246, | 291, | 336, | F | F | H | H | CN |
| 247, | 292, | 337, | H | H | F | F | CN |
| 248, | 293, | 338, | H | F | H | F | CN |
| 249, | 294, | 339, | H | F | F | F | CN |
| 250, | 295, | 340, | F | F | H | F | CN |
| 251, | 296, | 341, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 342 bis 386

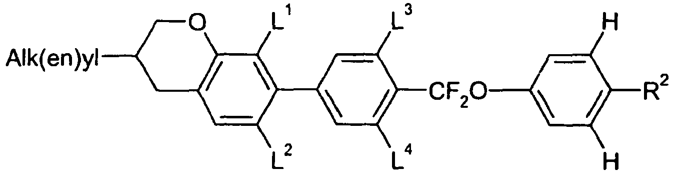

### Beispiele 387 bis 431

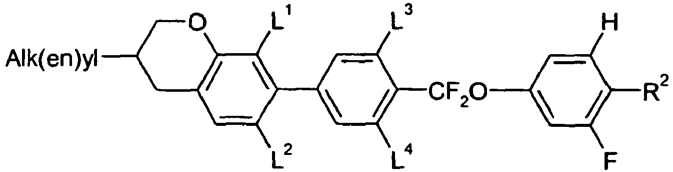

### Beispiele 432 bis 476

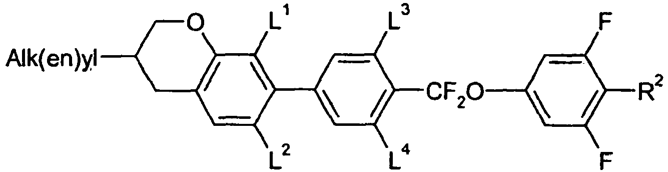

| **Beispiele** | | | **L¹** | **L²** | **L³** | **L⁴** | **R²** |
|---|---|---|---|---|---|---|---|
| 342, | 387, | 432, | H | H | H | H | F |
| 343, | 388, | 433, | H | H | H | F | F |
| 344, | 389, | 434, | H | F | H | H | F |
| 345, | 390, | 435, | F | F | H | H | F |
| 346, | 391, | 436, | H | H | F | F | F |
| 347, | 392, | 437, | H | F | H | F | F |
| 348, | 393, | 438, | H | F | F | F | F |
| 349, | 394, | 439, | F | F | H | F | F |
| 350, | 395, | 440, | F | F | F | F | F |
| 351, | 396, | 441, | H | H | H | H | CF₃ |
| 352, | 397, | 442, | H | H | H | F | CF₃ |
| 353, | 398, | 443, | H | F | H | H | CF₃ |
| 354, | 399, | 444, | F | F | H | H | CF₃ |
| 355, | 400, | 445, | H | H | F | F | CF₃ |
| 356, | 401, | 446, | H | F | H | F | CF₃ |
| 357, | 402, | 447, | H | F | F | F | CF₃ |
| 358, | 403, | 448, | F | F | H | F | CF₃ |
| 359, | 404, | 449, | F | F | F | F | CF₃ |
| 360, | 405, | 450, | H | H | H | H | OCF₃ |
| 361, | 406, | 451, | H | H | H | F | OCF₃ |
| 362, | 407, | 452, | H | F | H | H | OCF₃ |
| 363, | 408, | 453, | F | F | H | H | OCF₃ |
| 364, | 409, | 454, | H | H | F | F | OCF₃ |
| 365, | 410, | 455, | H | F | H | F | OCF₃ |
| 366, | 411, | 456, | H | F | F | F | OCF₃ |
| 367, | 412, | 457, | F | F | H | F | OCF₃ |
| 368, | 413, | 458, | F | F | F | F | OCF₃ |
| 369, | 414, | 459, | H | H | H | H | Cl |
| 370, | 415, | 460, | H | H | H | F | Cl |
| 371, | 416, | 461, | H | F | H | H | Cl |
| 372, | 417, | 462, | F | F | H | H | Cl |
| 373, | 418, | 463, | H | H | F | F | Cl |
| 374, | 419, | 464, | H | F | H | F | Cl |
| 375, | 420, | 465, | H | F | F | F | Cl |
| 376, | 421, | 466, | F | F | H | F | Cl |
| 377, | 422, | 467, | F | F | F | F | Cl |
| 378, | 423, | 468, | H | H | H | H | CN |
| 379, | 424, | 469, | H | H | H | F | CN |
| 380, | 425, | 470, | H | F | H | H | CN |
| 381, | 426, | 471, | F | F | H | H | CN |
| 382, | 427, | 472, | H | H | F | F | CN |
| 383, | 428, | 473, | H | F | H | F | CN |
| 384, | 429, | 474, | H | F | F | F | CN |
| 385, | 430, | 475, | F | F | H | F | CN |
| 386, | 431, | 476, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 477 bis 521

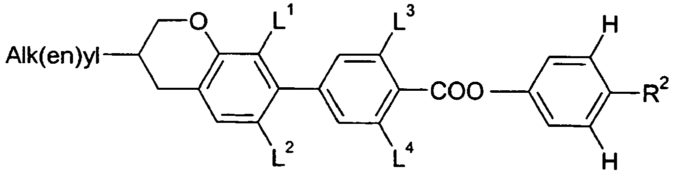

### Beispiele 522 bis 566

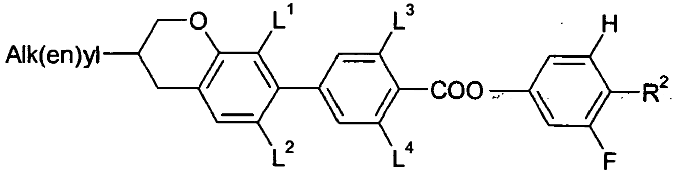

### Beispiele 567 bis 611

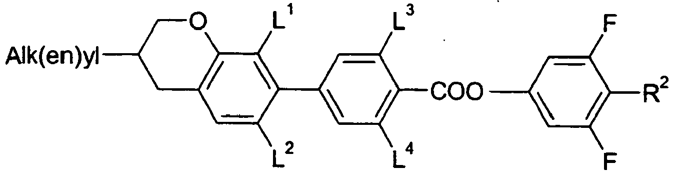

| **Beispiele** | | | **L¹** | **L²** | **L³** | **L⁴** | **R²** |
|---|---|---|---|---|---|---|---|
| 477, | 522, | 567, | H | H | H | H | F |
| 478, | 523, | 568, | H | H | H | F | F |
| 479, | 524, | 569, | H | F | H | H | F |
| 480, | 525, | 570, | F | F | H | H | F |
| 481, | 526, | 571, | H | H | F | F | F |
| 482, | 527, | 572, | H | F | H | F | F |
| 483, | 528, | 573, | H | F | F | F | F |
| 484, | 529, | 574, | F | F | H | F | F |
| 485, | 530, | 575, | F | F | F | F | F |
| 486, | 531, | 576, | H | H | H | H | CF₃ |
| 487, | 532, | 577, | H | H | H | F | CF₃ |
| 488, | 533, | 578, | H | F | H | H | CF₃ |
| 489, | 534, | 579, | F | F | H | H | CF₃ |
| 490, | 535, | 580, | H | H | F | F | CF₃ |
| 491, | 536, | 581, | H | F | H | F | CF₃ |
| 492, | 537, | 582, | H | F | F | F | CF₃ |
| 493, | 538, | 583, | F | F | H | F | CF₃ |
| 494, | 539, | 584, | F | F | F | F | CF₃ |
| 495, | 540, | 585, | H | H | H | H | OCF₃ |
| 496, | 541, | 586, | H | H | H | F | OCF₃ |
| 497, | 542, | 587, | H | F | H | H | OCF₃ |
| 498, | 543, | 588, | F | F | H | H | OCF₃ |
| 499, | 544, | 589, | H | H | F | F | OCF₃ |
| 500, | 545, | 590, | H | F | H | F | OCF₃ |
| 501, | 546, | 591, | H | F | F | F | OCF₃ |
| 502, | 547, | 592, | F | F | H | F | OCF₃ |
| 503, | 548, | 593, | F | F | F | F | OCF₃ |
| 504, | 549, | 594, | H | H | H | H | Cl |
| 505, | 550, | 595, | H | H | H | F | Cl |
| 506, | 551, | 596, | H | F | H | H | Cl |
| 507, | 552, | 597, | F | F | H | H | Cl |
| 508, | 553, | 598, | H | H | F | F | Cl |
| 509, | 554, | 599, | H | F | H | F | Cl |
| 510, | 555, | 600, | H | F | F | F | Cl |
| 511, | 556, | 601, | F | F | H | F | Cl |
| 512, | 557, | 602, | F | F | F | F | Cl |
| 513, | 558, | 603, | H | H | H | H | CN |
| 514, | 559, | 604, | H | H | H | F | CN |
| 515, | 560, | 605, | H | F | H | H | CN |
| 516, | 561, | 606, | F | F | H | H | CN |
| 517, | 562, | 607, | H | H | F | F | CN |
| 518, | 563, | 608, | H | F | H | F | CN |
| 519, | 564, | 609, | H | F | F | F | CN |
| 520, | 565, | 610, | F | F | H | F | CN |
| 521, | 566, | 611, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 612 bis 656

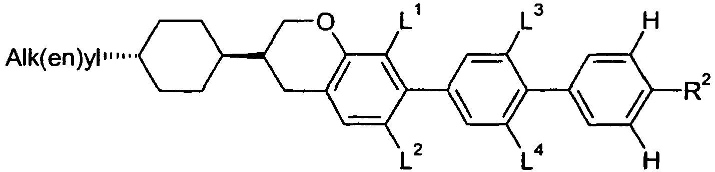

### Beispiele 657 bis 701

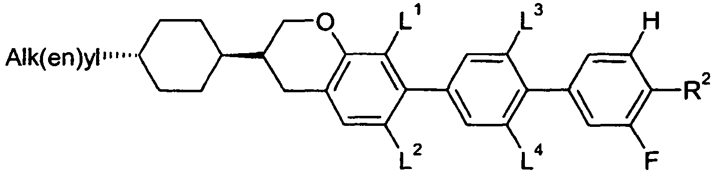

### Beispiele 702 bis 746

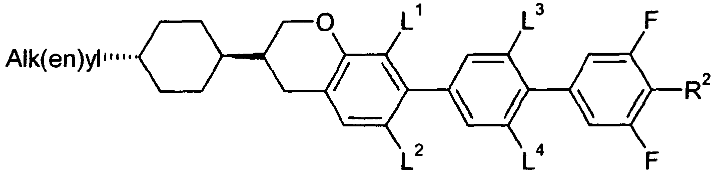

| **Beispiele** | | | **L¹** | **L²** | **L³** | **L⁴** | **R²** |
|---|---|---|---|---|---|---|---|
| 612, | 657, | 702, | H | H | H | H | F |
| 613, | 658, | 703, | H | H | H | F | F |
| 614, | 659, | 704, | H | F | H | H | F |
| 615, | 660, | 705, | F | F | H | H | F |
| 616, | 661, | 706, | H | H | F | F | F |
| 617, | 662, | 707, | H | F | H | F | F |
| 618, | 663, | 708, | H | F | F | F | F |
| 619, | 664, | 709, | F | F | H | F | F |
| 620, | 665, | 710, | F | F | F | F | F |
| 621, | 666, | 711, | H | H | H | H | CF₃ |
| 622, | 667, | 712, | H | H | H | F | CF₃ |
| 623, | 668, | 713, | H | F | H | H | CF₃ |
| 624, | 669, | 714, | F | F | H | H | CF₃ |
| 625, | 670, | 715, | H | H | F | F | CF₃ |
| 626, | 671, | 716, | H | F | H | F | CF₃ |
| 627, | 672, | 717, | H | F | F | F | CF₃ |
| 628, | 673, | 718, | F | F | H | F | CF₃ |
| 629, | 674, | 719, | F | F | F | F | CF₃ |
| 630, | 675, | 720, | H | H | H | H | OCF₃ |
| 631, | 676, | 721, | H | H | H | F | OCF₃ |
| 632, | 677, | 722, | H | F | H | H | OCF₃ |
| 633, | 678, | 723, | F | F | H | H | OCF₃ |
| 634, | 679, | 724, | H | H | F | F | OCF₃ |
| 635, | 680, | 725, | H | F | H | F | OCF₃ |
| 636, | 681, | 726, | H | F | F | F | OCF₃ |
| 637, | 682, | 727, | F | F | H | F | OCF₃ |
| 638, | 683, | 728, | F | F | F | F | OCF₃ |
| 639, | 684, | 729, | H | H | H | H | Cl |
| 640, | 685, | 730, | H | H | H | F | Cl |
| 641, | 686, | 731, | H | F | H | H | Cl |
| 642, | 687, | 732, | F | F | H | H | Cl |
| 643, | 688, | 733, | H | H | F | F | Cl |
| 644, | 689, | 734, | H | F | H | F | Cl |
| 645, | 690, | 735, | H | F | F | F | Cl |
| 646, | 691, | 736, | F | F | H | F | Cl |
| 647, | 692, | 737, | F | F | F | F | Cl |
| 648, | 693, | 738, | H | H | H | H | CN |
| 649, | 694, | 739, | H | H | H | F | CN |
| 650, | 695, | 740, | H | F | H | H | CN |
| 651, | 696, | 741, | F | F | H | H | CN |
| 652, | 697, | 742, | H | H | F | F | CN |
| 653, | 698, | 743, | H | F | H | F | CN |
| 654, | 699, | 744, | H | F | F | F | CN |
| 655, | 700, | 745, | F | F | H | F | CN |
| 656, | 701, | 746, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 747 bis 791

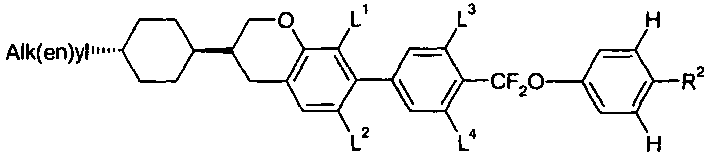

### Beispiele 792 bis 836

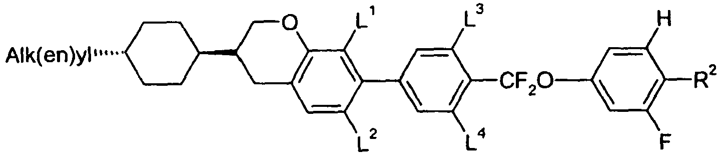

### Beispiele 837 bis 881

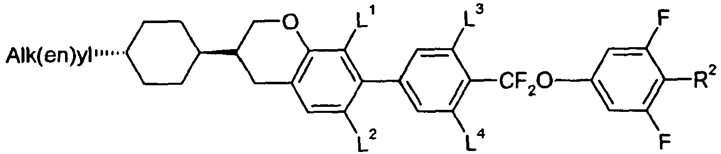

| **Beispiele** | | | **L¹** | **L²** | **L³** | **L⁴** | **R²** |
|---|---|---|---|---|---|---|---|
| 747, | 792, | 837, | H | H | H | H | F |
| 748, | 793, | 838, | H | H | H | F | F |
| 749, | 794, | 839, | H | F | H | H | F |
| 750, | 795, | 840, | F | F | H | H | F |
| 751, | 796, | 841, | H | H | F | F | F |
| 752, | 797, | 842, | H | F | H | F | F |
| 753, | 798, | 843, | H | F | F | F | F |
| 754, | 799, | 844, | F | F | H | F | F |
| 755, | 800, | 845, | F | F | F | F | F |
| 756, | 801, | 846, | H | H | H | H | CF₃ |
| 757, | 802, | 847, | H | H | H | F | CF₃ |
| 758, | 803, | 848, | H | F | H | H | CF₃ |
| 759, | 804, | 849, | F | F | H | H | CF₃ |
| 760, | 805, | 850, | H | H | F | F | CF₃ |
| 761, | 806, | 851, | H | F | H | F | CF₃ |
| 762, | 807, | 852, | H | F | F | F | CF₃ |
| 763, | 808, | 853, | F | F | H | F | CF₃ |
| 764, | 809, | 854, | F | F | F | F | CF₃ |
| 765, | 810, | 855, | H | H | H | H | OCF₃ |
| 766, | 811, | 856, | H | H | H | F | OCF₃ |
| 767, | 812, | 857, | H | F | H | H | OCF₃ |
| 768, | 813, | 858, | F | F | H | H | OCF₃ |
| 769, | 814, | 859, | H | H | F | F | OCF₃ |
| 770, | 815, | 860, | H | F | H | F | OCF₃ |
| 771, | 816, | 861, | H | F | F | F | OCF₃ |
| 772, | 817, | 862, | F | F | H | F | OCF₃ |
| 773, | 818, | 863, | F | F | F | F | OCF₃ |
| 774, | 819, | 864, | H | H | H | H | Cl |
| 775, | 820, | 865, | H | H | H | F | Cl |
| 776, | 821, | 866, | H | F | H | H | Cl |
| 777, | 822, | 867, | F | F | H | H | Cl |
| 778, | 823, | 868, | H | H | F | F | Cl |
| 779, | 824, | 869, | H | F | H | F | Cl |
| 780, | 825, | 870, | H | F | F | F | Cl |
| 781, | 826, | 871, | F | F | H | F | Cl |
| 782, | 827, | 872, | F | F | F | F | Cl |
| 783, | 828, | 873, | H | H | H | H | CN |
| 784, | 829, | 874, | H | H | H | F | CN |
| 785, | 830, | 875, | H | F | H | H | CN |
| 786, | 831, | 876, | F | F | H | H | CN |
| 787, | 832, | 877, | H | H | F | F | CN |
| 788, | 833, | 878, | H | F | H | F | CN |
| 789, | 834, | 879, | H | F | F | F | CN |
| 790, | 835, | 880, | F | F | H | F | CN |
| 791, | 836, | 881, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 882 bis 926

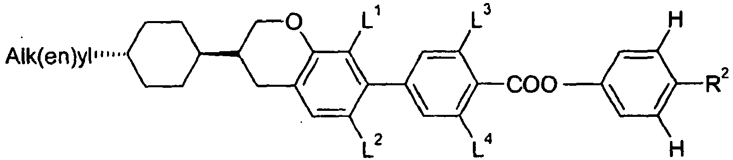

### Beispiele 927 bis 971

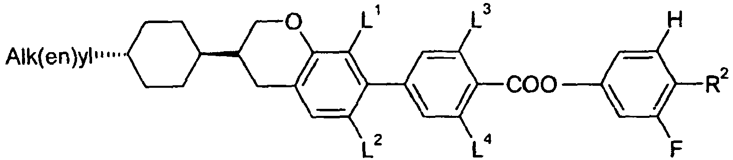

### Beispiele 972 bis 1016

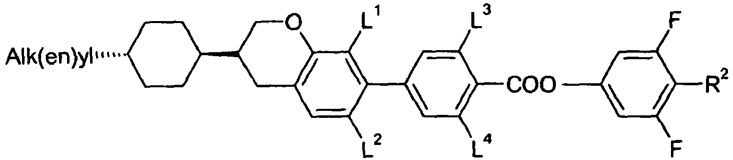

| **Beispiele** | | | **L¹** | **L²** | **L³** | **L⁴** | **R²** |
|---|---|---|---|---|---|---|---|
| 882, | 927, | 972, | H | H | H | H | F |
| 883, | 928, | 973, | H | H | H | F | F |
| 884, | 929, | 974, | H | F | H | H | F |
| 885, | 930, | 975, | F | F | H | H | F |
| 886, | 931, | 976, | H | H | F | F | F |
| 887, | 932, | 977, | H | F | H | F | F |
| 888, | 933, | 978, | H | F | F | F | F |
| 889, | 934, | 979, | F | F | H | F | F |
| 890, | 935, | 980, | F | F | F | F | F |
| 891, | 936, | 981, | H | H | H | H | CF₃ |
| 892, | 937, | 982, | H | H | H | F | CF₃ |
| 893, | 938, | 983, | H | F | H | H | CF₃ |
| 894, | 939, | 984, | F | F | H | H | CF₃ |
| 895, | 940, | 985, | H | H | F | F | CF₃ |
| 896, | 941, | 986, | H | F | H | F | CF₃ |
| 897, | 942, | 987, | H | F | F | F | CF₃ |
| 898, | 943, | 988, | F | F | H | F | CF₃ |
| 899, | 944, | 989, | F | F | F | F | CF₃ |
| 900, | 945, | 990, | H | H | H | H | OCF₃ |
| 901, | 946, | 991, | H | H | H | F | OCF₃ |
| 902, | 947, | 992, | H | F | H | H | OCF₃ |
| 903, | 948, | 993, | F | F | H | H | OCF₃ |
| 904, | 949, | 994, | H | H | F | F | OCF₃ |
| 905, | 950, | 995, | H | F | H | F | OCF₃ |
| 906, | 951, | 996, | H | F | F | F | OCF₃ |
| 907, | 952, | 997, | F | F | H | F | OCF₃ |
| 908, | 953, | 998, | F | F | F | F | OCF₃ |
| 909, | 954, | 999, | H | H | H | H | Cl |
| 910, | 955, | 1000, | H | H | H | F | Cl |
| 911, | 956, | 1001, | H | F | H | H | Cl |
| 912, | 957, | 1002, | F | F | H | H | Cl |
| 913, | 958, | 1003, | H | H | F | F | Cl |
| 914, | 959, | 1004, | H | F | H | F | Cl |
| 915, | 960, | 1005, | H | F | F | F | Cl |
| 916, | 961, | 1006, | F | F | H | F | Cl |
| 917, | 962, | 1007, | F | F | F | F | Cl |
| 918, | 963, | 1008, | H | H | H | H | CN |
| 919, | 964, | 1009, | H | H | H | F | CN |
| 920, | 965, | 1010, | H | F | H | H | CN |
| 921, | 966, | 1011, | F | F | H | H | CN |
| 922, | 967, | 1012, | H | H | F | F | CN |
| 923, | 968, | 1013, | H | F | H | F | CN |
| 924, | 969, | 1014, | H | F | F | F | CN |
| 925, | 970, | 1015, | F | F | H | F | CN |
| 926, | 971, | 1016, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 1017 bis 1061

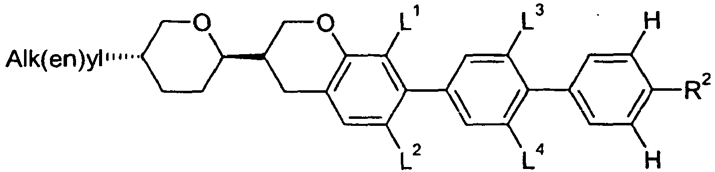

### Beispiele 1062 bis 1106

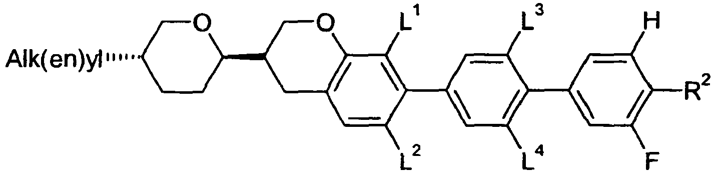

### Beispiele 1107 bis 1151

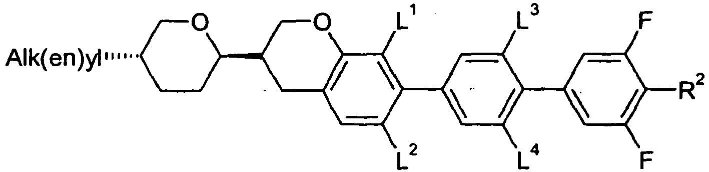

| **Beispiele** | | | **L¹** | **L²** | **L³** | **L⁴** | **R²** |
|---|---|---|---|---|---|---|---|
| 1017, | 1062, | 1107, | H | H | H | H | F |
| 1018, | 1063, | 1108, | H | H | H | F | F |
| 1019, | 1064, | 1109, | H | F | H | H | F |
| 1020, | 1065, | 1110, | F | F | H | H | F |
| 1021, | 1066, | 1111, | H | H | F | F | F |
| 1022, | 1067, | 1112, | H | F | H | F | F |
| 1023, | 1068, | 1113, | H | F | F | F | F |
| 1024, | 1069, | 1114, | F | F | H | F | F |
| 1025, | 1070, | 1115, | F | F | F | F | F |
| 1026, | 1071, | 1116, | H | H | H | H | CF₃ |
| 1027, | 1072, | 1117, | H | H | H | F | CF₃ |
| 1028, | 1073, | 1118, | H | F | H | H | CF₃ |
| 1029, | 1074, | 1119, | F | F | H | H | CF₃ |
| 1030, | 1075, | 1120, | H | H | F | F | CF₃ |
| 1031, | 1076, | 1121, | H | F | H | F | CF₃ |
| 1032, | 1077, | 1122, | H | F | F | F | CF₃ |
| 1033, | 1078, | 1123, | F | F | H | F | CF₃ |
| 1034, | 1079, | 1124, | F | F | F | F | CF₃ |
| 1035, | 1080, | 1125, | H | H | H | H | OCF₃ |
| 1036, | 1081, | 1126, | H | H | H | F | OCF₃ |
| 1037, | 1082, | 1127, | H | F | H | H | OCF₃ |
| 1038, | 1083, | 1128, | F | F | H | H | OCF₃ |
| 1039, | 1084, | 1129, | H | H | F | F | OCF₃ |
| 1040, | 1085, | 1130, | H | F | H | F | OCF₃ |
| 1041, | 1086, | 1131, | H | F | F | F | OCF₃ |
| 1042, | 1087, | 1132, | F | F | H | F | OCF₃ |
| 1043, | 1088, | 1133, | F | F | F | F | OCF₃ |
| 1044, | 1089, | 1134, | H | H | H | H | Cl |
| 1045, | 1090, | 1135, | H | H | H | F | Cl |
| 1046, | 1091, | 1136, | H | F | H | H | Cl |
| 1047, | 1092, | 1137, | F | F | H | H | Cl |
| 1048, | 1093, | 1138, | H | H | F | F | Cl |
| 1049, | 1094, | 1139, | H | F | H | F | Cl |
| 1050, | 1095, | 1140, | H | F | F | F | Cl |
| 1051, | 1096, | 1141, | F | F | H | F | Cl |
| 1052, | 1097, | 1142, | F | F | F | F | Cl |
| 1053, | 1098, | 1143, | H | H | H | H | CN |
| 1054, | 1099, | 1144, | H | H | H | F | CN |
| 1055, | 1100, | 1145, | H | F | H | H | CN |
| 1056, | 1101, | 1146, | F | F | H | H | CN |
| 1057, | 1102, | 1147, | H | H | F | F | CN |
| 1058, | 1103, | 1148, | H | F | H | F | CN |
| 1059, | 1104, | 1149, | H | F | F | F | CN |
| 1060, | 1105, | 1150, | F | F | H | F | CN |
| 1061, | 1106, | 1151, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 1152 bis 1196

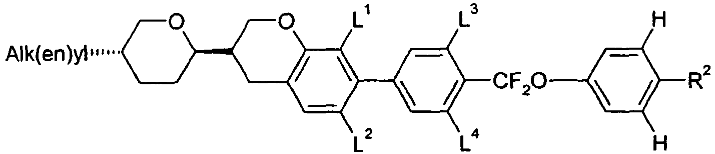

### Beispiele 1197 bis 1241

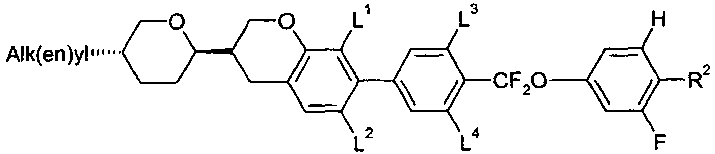

### Beispiele 1242 bis 1286

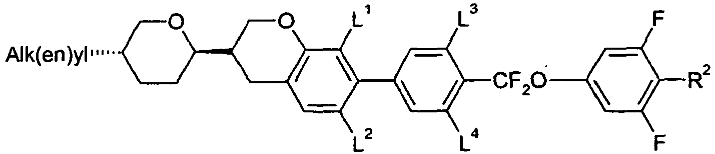

| **Beispiele** | | | **L¹** | **L²** | **L³** | **L⁴** | **R²** |
|---|---|---|---|---|---|---|---|
| 1152, | 1197, | 1242, | H | H | H | H | F |
| 1153, | 1198, | 1243, | H | H | H | F | F |
| 1154, | 1199, | 1244, | H | F | H | H | F |
| 1155, | 1200, | 1245, | F | F | H | H | F |
| 1156, | 1201, | 1246, | H | H | F | F | F |
| 1157, | 1202, | 1247, | H | F | H | F | F |
| 1158, | 1203, | 1248, | H | F | F | F | F |
| 1159, | 1204, | 1249, | F | F | H | F | F |
| 1160, | 1205, | 1250, | F | F | F | F | F |
| 1161, | 1206, | 1251, | H | H | H | H | CF₃ |
| 1162, | 1207, | 1252, | H | H | H | F | CF₃ |
| 1163, | 1208, | 1253, | H | F | H | H | CF₃ |
| 1164, | 1209, | 1254, | F | F | H | H | CF₃ |
| 1165, | 1210, | 1255, | H | H | F | F | CF₃ |
| 1166, | 1211, | 1256, | H | F | H | F | CF₃ |
| 1167, | 1212, | 1257, | H | F | F | F | CF₃ |
| 1168, | 1213, | 1258, | F | F | H | F | CF₃ |
| 1169, | 1214, | 1259, | F | F | F | F | CF₃ |
| 1170, | 1215, | 1260, | H | H | H | H | OCF₃ |
| 1171, | 1216, | 1261, | H | H | H | F | OCF₃ |
| 1172, | 1217, | 1262, | H | F | H | H | OCF₃ |
| 1173, | 1218, | 1263, | F | F | H | H | OCF₃ |
| 1174, | 1219, | 1264, | H | H | F | F | OCF₃ |
| 1175, | 1220, | 1265, | H | F | H | F | OCF₃ |
| 1176, | 1221, | 1266, | H | F | F | F | OCF₃ |
| 1177, | 1222, | 1267, | F | F | H | F | OCF₃ |
| 1178, | 1223, | 1268, | F | F | F | F | OCF₃ |
| 1179, | 1224, | 1269, | H | H | H | H | Cl |
| 1180, | 1225, | 1270, | H | H | H | F | Cl |
| 1181, | 1226, | 1271, | H | F | H | H | Cl |
| 1182, | 1227, | 1272, | F | F | H | H | Cl |
| 1183, | 1228, | 1273, | H | H | F | F | Cl |
| 1184, | 1229, | 1274, | H | F | H | F | Cl |
| 1185, | 1230, | 1275, | H | F | F | F | Cl |
| 1186, | 1231, | 1276, | F | F | H | F | Cl |
| 1187, | 1232, | 1277, | F | F | F | F | Cl |
| 1188, | 1233, | 1278, | H | H | H | H | CN |
| 1189, | 1234, | 1279, | H | H | H | F | CN |
| 1190, | 1235, | 1280, | H | F | H | H | CN |
| 1191, | 1236, | 1281, | F | F | H | H | CN |
| 1192, | 1237, | 1282, | H | H | F | F | CN |
| 1193, | 1238, | 1283, | H | F | H | F | CN |
| 1194, | 1239, | 1284, | H | F | F | F | CN |
| 1195, | 1240, | 1285, | F | F | H | F | CN |
| 1196, | 1241, | 1286, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 1287 bis 1331

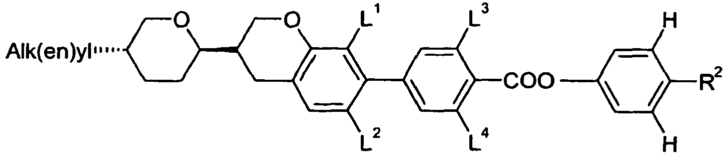

### Beispiele 1332 bis 1376

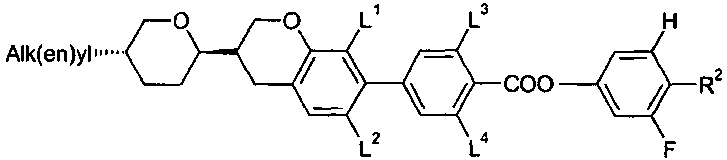

### Beispiele 1377 bis 1421

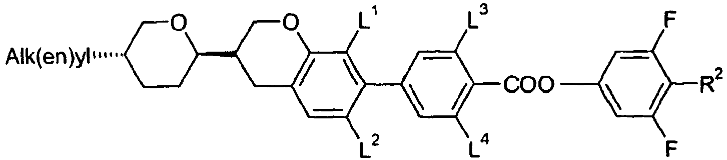

| **Beispiele** | | | **L¹** | **L²** | **L³** | **L⁴** | **R²** |
|---|---|---|---|---|---|---|---|
| 1287, | 1332, | 1377, | H | H | H | H | F |
| 1288, | 1333, | 1378, | H | H | H | F | F |
| 1289, | 1334, | 1379, | H | F | H | H | F |
| 1290, | 1335, | 1380, | F | F | H | H | F |
| 1291, | 1336, | 1381, | H | H | F | F | F |
| 1292, | 1337, | 1382, | H | F | H | F | F |
| 1293, | 1338, | 1383, | H | F | F | F | F |
| 1294, | 1339, | 1384, | F | F | H | F | F |
| 1295, | 1340, | 1385, | F | F | F | F | F |
| 1296, | 1341, | 1386, | H | H | H | H | CF₃ |
| 1297, | 1342, | 1387, | H | H | H | F | CF₃ |
| 1298, | 1343, | 1388, | H | F | H | H | CF₃ |
| 1299, | 1344, | 1389, | F | F | H | H | CF₃ |
| 1300, | 1345, | 1390, | H | H | F | F | CF₃ |
| 1301, | 1346, | 1391, | H | F | H | F | CF₃ |
| 1302, | 1347, | 1392, | H | F | F | F | CF₃ |
| 1303, | 1348, | 1393, | F | F | H | F | CF₃ |
| 1304, | 1349, | 1394, | F | F | F | F | CF₃ |
| 1305, | 1350, | 1395, | H | H | H | H | OCF₃ |
| 1306, | 1351, | 1396, | H | H | H | F | OCF₃ |
| 1307, | 1352, | 1397, | H | F | H | H | OCF₃ |
| 1308, | 1353, | 1398, | F | F | H | H | OCF₃ |
| 1309, | 1354, | 1399, | H | H | F | F | OCF₃ |
| 1310, | 1355, | 1400, | H | F | H | F | OCF₃ |
| 1311, | 1356, | 1401, | H | F | F | F | OCF₃ |
| 1312, | 1357, | 1402, | F | F | H | F | OCF₃ |
| 1313, | 1358, | 1403, | F | F | F | F | OCF₃ |
| 1314, | 1359, | 1404, | H | H | H | H | Cl |
| 1315, | 1360, | 1405, | H | H | H | F | Cl |
| 1316, | 1361, | 1406, | H | F | H | H | Cl |
| 1317, | 1362, | 1407, | F | F | H | H | Cl |
| 1318, | 1363, | 1408, | H | H | F | F | Cl |
| 1319, | 1364, | 1409, | H | F | H | F | Cl |
| 1320, | 1365, | 1410, | H | F | F | F | Cl |
| 1321, | 1366, | 1411, | F | F | H | F | Cl |
| 1322, | 1367, | 1412, | F | F | F | F | Cl |
| 1323, | 1368, | 1413, | H | H | H | H | CN |
| 1324, | 1369, | 1414, | H | H | H | F | CN |
| 1325, | 1370, | 1415, | H | F | H | H | CN |
| 1326, | 1371, | 1416, | F | F | H | H | CN |
| 1327, | 1372, | 1417, | H | H | F | F | CN |
| 1328, | 1373, | 1418, | H | F | H | F | CN |
| 1329, | 1374, | 1419, | H | F | F | F | CN |
| 1330, | 1375, | 1420, | F | F | H | F | CN |
| 1331, | 1376, | 1421, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 1422 bis 1466

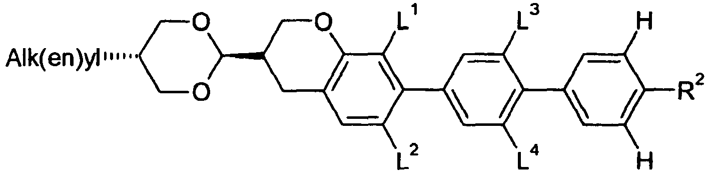

### Beispiele 1467 bis 1511

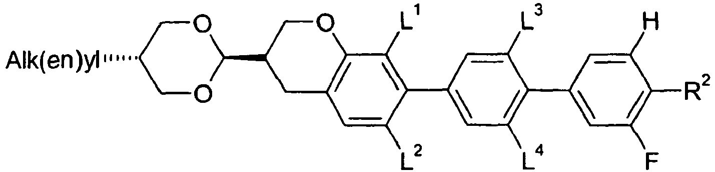

### Beispiele 1512 bis 1556

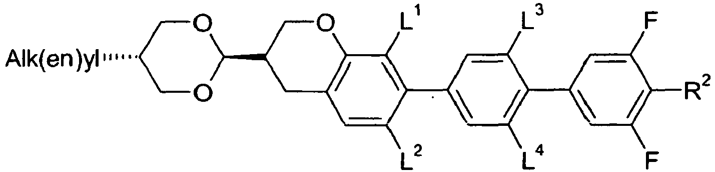

| **Beispiele** | | | **L¹** | **L²** | **L³** | **L⁴** | **R²** |
|---|---|---|---|---|---|---|---|
| 1422, | 1467, | 1512, | H | H | H | H | F |
| 1423, | 1468, | 1513, | H | H | H | F | F |
| 1424, | 1469, | 1514, | H | F | H | H | F |
| 1425, | 1470, | 1515, | F | F | H | H | F |
| 1426, | 1471, | 1516, | H | H | F | F | F |
| 1427, | 1472, | 1517, | H | F | H | F | F |
| 1428, | 1473, | 1518, | H | F | F | F | F |
| 1429, | 1474, | 1519, | F | F | H | F | F |
| 1430, | 1475, | 1520, | F | F | F | F | F |
| 1431, | 1476, | 1521, | H | H | H | H | CF₃ |
| 1432, | 1477, | 1522, | H | H | H | F | CF₃ |
| 1433, | 1478, | 1523, | H | F | H | H | CF₃ |
| 1434, | 1479, | 1524, | F | F | H | H | CF₃ |
| 1435, | 1480, | 1525, | H | H | F | F | CF₃ |
| 1436, | 1481, | 1526, | H | F | H | F | CF₃ |
| 1437, | 1482, | 1527, | H | F | F | F | CF₃ |
| 1438, | 1483, | 1528, | F | F | H | F | CF₃ |
| 1439, | 1484, | 1529, | F | F | F | F | CF₃ |
| 1440, | 1485, | 1530, | H | H | H | H | OCF₃ |
| 1441, | 1486, | 1531, | H | H | H | F | OCF₃ |
| 1442, | 1487, | 1532, | H | F | H | H | OCF₃ |
| 1443, | 1488, | 1533, | F | F | H | H | OCF₃ |
| 1444, | 1489, | 1534, | H | H | F | F | OCF₃ |
| 1445, | 1490, | 1535, | H | F | H | F | OCF₃ |
| 1446, | 1491, | 1536, | H | F | F | F | OCF₃ |
| 1447, | 1492, | 1537, | F | F | H | F | OCF₃ |
| 1448, | 1493, | 1538, | F | F | F | F | OCF₃ |
| 1449, | 1494, | 1539, | H | H | H | H | Cl |
| 1450, | 1495, | 1540, | H | H | H | F | Cl |
| 1451, | 1496, | 1541, | H | F | H | H | Cl |
| 1452, | 1497, | 1542, | F | F | H | H | Cl |
| 1453, | 1498, | 1543, | H | H | F | F | Cl |
| 1454, | 1499, | 1544, | H | F | H | F | Cl |
| 1455, | 1500, | 1545, | H | F | F | F | Cl |
| 1456, | 1501, | 1546, | F | F | H | F | Cl |
| 1457, | 1502, | 1547, | F | F | F | F | Cl |
| 1458, | 1503, | 1548, | H | H | H | H | CN |
| 1459, | 1504, | 1549, | H | H | H | F | CN |
| 1460, | 1505, | 1550, | H | F | H | H | CN |
| 1461, | 1506, | 1551, | F | F | H | H | CN |
| 1462, | 1507, | 1552, | H | H | F | F | CN |
| 1463, | 1508, | 1553, | H | F | H | F | CN |
| 1464, | 1509, | 1554, | H | F | F | F | CN |
| 1465, | 1510, | 1555, | F | F | H | F | CN |
| 1466, | 1511, | 1556, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele1557 bis 1601

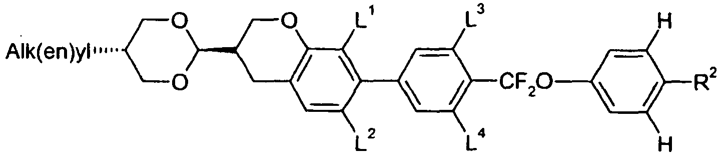

### Beispiele 1602 bis 1646

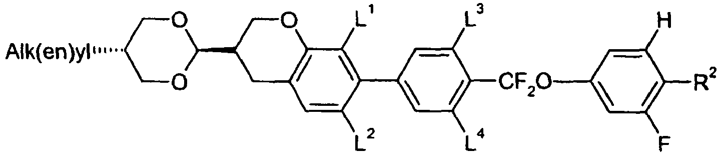

### Beispiele 1647 bis 1691

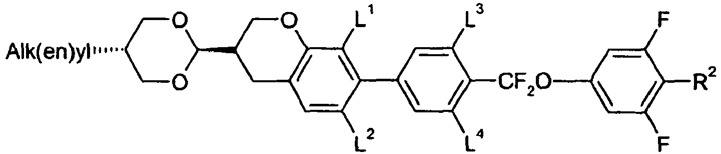

| **Beispiele** | | | **L¹** | **L²** | **L³** | **L⁴** | **R²** |
|---|---|---|---|---|---|---|---|
| 1557, | 1602, | 1647, | H | H | H | H | F |
| 1558, | 1603, | 1648, | H | H | H | F | F |
| 1559, | 1604, | 1649, | H | F | H | H | F |
| 1560, | 1605, | 1650, | F | F | H | H | F |
| 1561, | 1606, | 1651, | H | H | F | F | F |
| 1562, | 1607, | 1652, | H | F | H | F | F |
| 1563, | 1608, | 1653, | H | F | F | F | F |
| 1564, | 1609, | 1654, | F | F | H | F | F |
| 1565, | 1610, | 1655, | F | F | F | F | F |
| 1566, | 1611, | 1656, | H | H | H | H | CF₃ |
| 1567, | 1612, | 1657, | H | H | H | F | CF₃ |
| 1568, | 1613, | 1658, | H | F | H | H | CF₃ |
| 1569, | 1614, | 1659, | F | F | H | H | CF₃ |
| 1570, | 1615, | 1660, | H | H | F | F | CF₃ |
| 1571, | 1616, | 1661, | H | F | H | F | CF₃ |
| 1572, | 1617, | 1662, | H | F | F | F | CF₃ |
| 1573, | 1618, | 1663, | F | F | H | F | CF₃ |
| 1574, | 1619, | 1664, | F | F | F | F | CF₃ |
| 1575, | 1620, | 1665, | H | H | H | H | OCF₃ |
| 1576, | 1621, | 1666, | H | H | H | F | OCF₃ |
| 1577, | 1622, | 1667, | H | F | H | H | OCF₃ |
| 1578, | 1623, | 1668, | F | F | H | H | OCF₃ |
| 1579, | 1624, | 1669, | H | H | F | F | OCF₃ |
| 1580, | 1625, | 1670, | H | F | H | F | OCF₃ |
| 1581, | 1626, | 1671, | H | F | F | F | OCF₃ |
| 1582, | 1627, | 1672, | F | F | H | F | OCF₃ |
| 1583, | 1628, | 1673, | F | F | F | F | OCF₃ |
| 1584, | 1629, | 1674, | H | H | H | H | Cl |
| 1585, | 1630, | 1675, | H | H | H | F | Cl |
| 1586, | 1631, | 1676, | H | F | H | H | Cl |
| 1587, | 1632, | 1677, | F | F | H | H | Cl |
| 1588, | 1633, | 1678, | H | H | F | F | Cl |
| 1589, | 1634, | 1679, | H | F | H | F | Cl |
| 1590, | 1635, | 1680, | H | F | F | F | Cl |
| 1591, | 1636, | 1681, | F | F | H | F | Cl |
| 1592, | 1637, | 1682, | F | F | F | F | Cl |
| 1593, | 1638, | 1683, | H | H | H | H | CN |
| 1594, | 1639, | 1684, | H | H | H | F | CN |
| 1595, | 1640, | 1685, | H | F | H | H | CN |
| 1596, | 1641, | 1686, | F | F | H | H | CN |
| 1597, | 1642, | 1687, | H | H | F | F | CN |
| 1598, | 1643, | 1688, | H | F | H | F | CN |
| 1599, | 1644, | 1689, | H | F | F | F | CN |
| 1600, | 1645, | 1690, | F | F | H | F | CN |
| 1601, | 1646, | 1691, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiele 1692 bis 1736

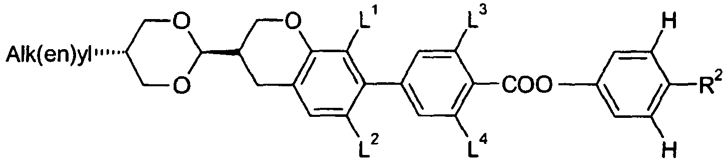

### Beispiele 1737 bis 1781

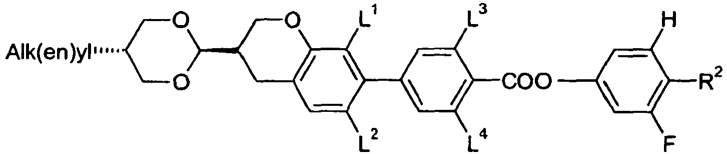

### Beispiele 1782 bis 1826

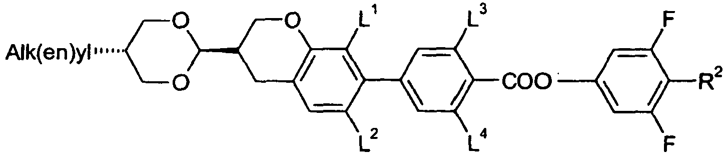

| **Beispiele** | | | **L¹** | **L²** | **L³** | **L⁴** | **R²** |
|---|---|---|---|---|---|---|---|
| 1692, | 1737, | 1782, | H | H | H | H | F |
| 1693, | 1738, | 1783, | H | H | H | F | F |
| 1694, | 1739, | 1784, | H | F | H | H | F |
| 1695, | 1740, | 1785, | F | F | H | H | F |
| 1696, | 1741, | 1786, | H | H | F | F | F |
| 1697, | 1742, | 1787, | H | F | H | F | F |
| 1698, | 1743, | 1788, | H | F | F | F | F |
| 1699, | 1744, | 1789, | F | F | H | F | F |
| 1700, | 1745, | 1790, | F | F | F | F | F |
| 1701, | 1746, | 1791, | H | H | H | H | CF₃ |
| 1702, | 1747, | 1792, | H | H | H | F | CF₃ |
| 1703, | 1748, | 1793, | H | F | H | H | CF₃ |
| 1704, | 1749, | 1794, | F | F | H | H | CF₃ |
| 1705, | 1750, | 1795, | H | H | F | F | CF₃ |
| 1706, | 1751, | 1796, | H | F | H | F | CF₃ |
| 1707, | 1752, | 1797, | H | F | F | F | CF₃ |
| 1708, | 1753, | 1798, | F | F | H | F | CF₃ |
| 1709, | 1754, | 1799, | F | F | F | F | CF₃ |
| 1710, | 1755, | 1800, | H | H | H | H | OCF₃ |
| 1711, | 1756, | 1801, | H | H | H | F | OCF₃ |
| 1712, | 1757, | 1802, | H | F | H | H | OCF₃ |
| 1713, | 1758, | 1803, | F | F | H | H | OCF₃ |
| 1714, | 1759, | 1804, | H | H | F | F | OCF₃ |
| 1715, | 1760, | 1805, | H | F | H | F | OCF₃ |
| 1716, | 1761, | 1806, | H | F | F | F | OCF₃ |
| 1717, | 1762, | 1807, | F | F | H | F | OCF₃ |
| 1718, | 1763, | 1808, | F | F | F | F | OCF₃ |
| 1719, | 1764, | 1809, | H | H | H | H | Cl |
| 1720, | 1765, | 1810, | H | H | H | F | Cl |
| 1721, | 1766, | 1811, | H | F | H | H | Cl |
| 1722, | 1767, | 1812, | F | F | H | H | Cl |
| 1723, | 1768, | 1813, | H | H | F | F | Cl |
| 1724, | 1769, | 1814, | H | F | H | F | Cl |
| 1725, | 1770, | 1815, | H | F | F | F | Cl |
| 1726, | 1771, | 1816, | F | F | H | F | Cl |
| 1727, | 1772, | 1817, | F | F | F | F | Cl |
| 1728, | 1773, | 1818, | H | H | H | H | CN |
| 1729, | 1774, | 1819, | H | H | H | F | CN |
| 1730, | 1775, | 1820, | H | F | H | H | CN |
| 1731, | 1776, | 1821, | F | F | H | H | CN |
| 1732, | 1777, | 1822, | H | H | F | F | CN |
| 1733, | 1778, | 1823, | H | F | H | F | CN |
| 1734, | 1779, | 1824, | H | F | F | F | CN |
| 1735, | 1780, | 1825, | F | F | H | F | CN |
| 1736, | 1781, | 1826, | F | F | F | F | CN |

wobei Alk(en)yl ausgewählt ist aus: CH₃, C₂H₅, C₃H₇, C₄H₉, C₅H₁₁, CH=CH₂, CH=CH-CH₃ und CH=CH-C₃H₇.

### Beispiel 1827

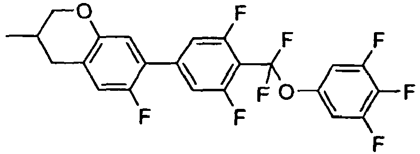

K 119 I

### Beispiel 1828

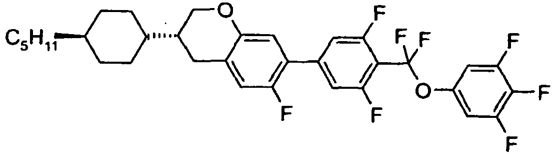

K 81 SmA (75) N 177
Δε = 31,3
Δn = 0,1430

### Beispiel 1829

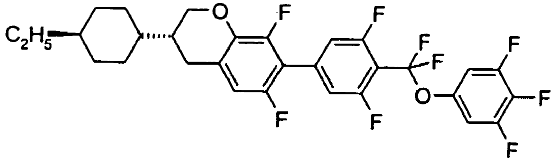

K 110 N 145,7 I
Δε = 36,9
Δn = 0,1358

### Beispiel 1830

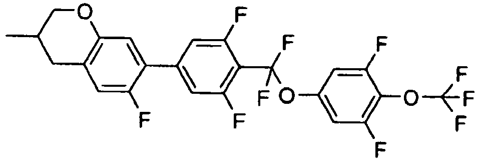

K 102 I
Δε = 42,3
Δn = 0,1354

### Beispiel 1831

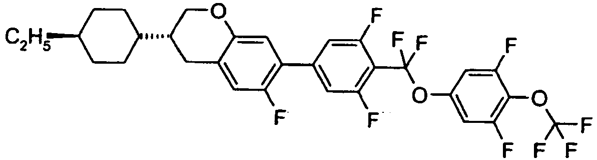

K 99 SmA 126 N 174 I
Δε = 41,6
Δn = 0,1366

### Beispiel 1832

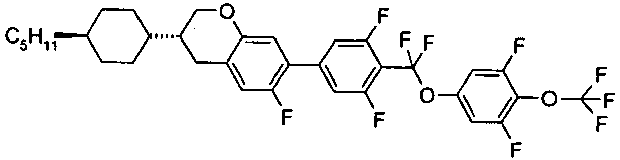

K 89 SmA 150 N 186,6 I
Δε = 34,3
Δn = 0,1351

### Beispiel 1833

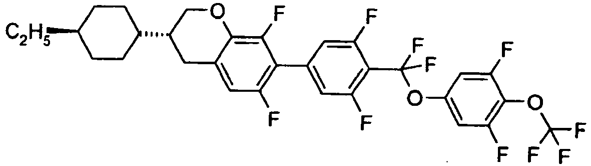

K 98 SmA 110 N 157,8 I
Δε = 37,9
Δn = 0,1295

### Beispiel 1834

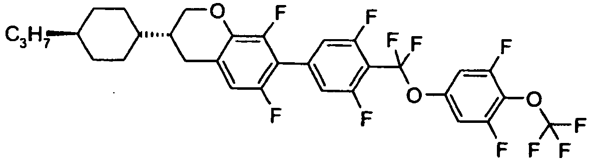

K 88 SmA 124 N 176,8
Δε = 39,1
Δn = 0,1317

### Beispiel 1835

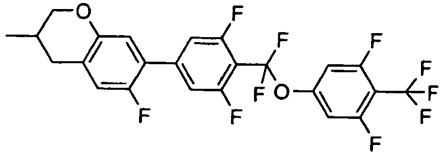

K 121 I

### Beispiel 1836

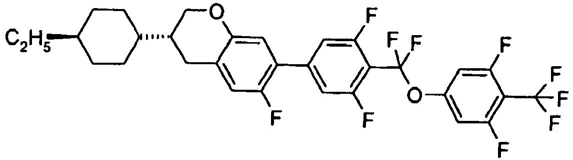

K 122 N 152,3 I
Δε = 46,0
Δn = 0,1406

### Beispiel 1837

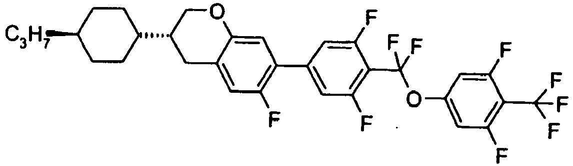

K 107 SmA (83) N 169,4 I
Δε = 44,2
Δn = 0,1404

### Beispiel 1838

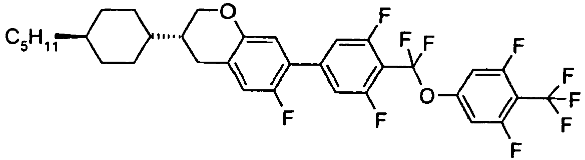

K 83 SmA 104 N 167,7 I
Δε = 42,6
Δn = 0,1392

### Beispiel 1839

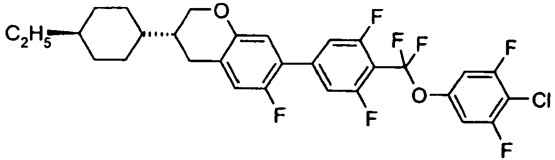

K 134 N 180,7 I
Δε = 32,3
Δn = 0,1498

### Beispiel 1840

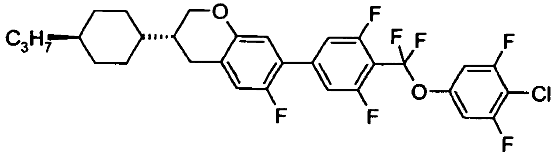

K 110 N 197,8 I
Δε = 32,5
Δn = 0,1535

### Beispiel 1841

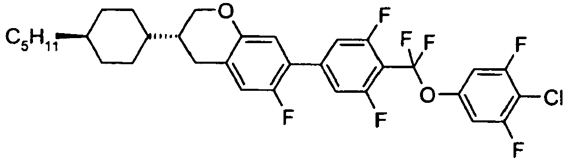

K 106 SmA (104) N 193,8 I
Δε = 30,3
Δn = 0,1501

### Beispiel 1842

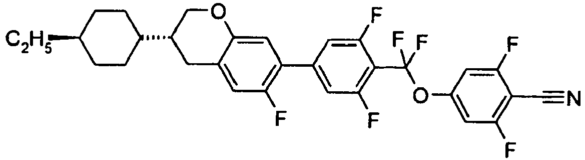

K 145 N 204,1 I
Δn = 0,1623

### Beispiel 1843

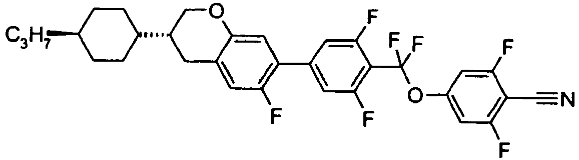

K 142 N 215,2 I
Δn = 0,1559

### Beispiel 1844

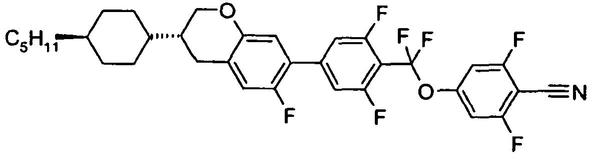

K 132 N 208,9 I

### Beispiel 1845

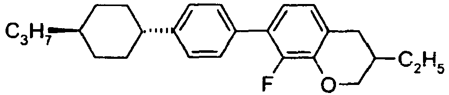

K 104 N 190,7 I

### Beispiel 1846

Eine Flüssigkristallmischung enthaltend

| | |
|---|---|
| BCH-3F.F | 10,80 % |
| BCH-5F.F | 9,00 % |
| ECCP-3OCF3 | 4,50 % |
| ECCP-5OCF3 | 4,50 % |
| CBC-33F | 1,80 % |
| CBC-53F | 1,80 % |
| CBC-55F | 1,80 % |
| PCH-6F | 7,20 % |
| PCH-7F | 5,40 % |
| CCP-2OCF3 | 7,20 % |
| CCP-3OCF3 | 10,80 % |
| CCP-4OCF3 | 6,30 % |
| CCP-5OCF | 9,90 % |
| PCH-5F | 9,00 % |
| Verbindung des Beispiels 21 | 10,00 % |

weist folgende Eigenschaften auf:

| | |
|---|---|
| Klärpunkt: | +92,3°C |
| Δε: | +7,3 |
| Δn: | +0,1012 |

### Beispiel 1847

### Eine Flüssigkristallmischung enthaltend

| | |
|---|---|
| BCH-3F.F | 10,80 % |
| BCH-5F.F | 9,00 % |
| ECCP-3OCF3 | 4,50 % |
| ECCP-5OCF3 | 4,50 % |
| CBC-33F | 1,80 % |
| CBC-53F | 1,80 % |
| CBC-55F | 1,80 % |
| PCH-6F | 7,20 % |
| PCH-7F | 5,40 % |
| CCP-2OCF3 | 7,20 % |
| CCP-3OCF3 | 10,80 % |
| CCP-4OCF3 | 6,30 % |
| CCP-5OCF | 9,90 % |
| PCH-5F | 9,00 % |
| Verbindung des Beispiels 23 | 10,00 % |

weist folgende Eigenschaften auf:

| | |
|---|---|
| Klärpunkt: | +97,1°C |
| Δε: | +8,4 |
| Δn: | +0,1028 |

### Beispiel 1848

Eine Flüssigkristallmischung enthaltend

| | |
|---|---|
| BCH-3F.F | 10,80 % |
| BCH-5F.F | 9,00 % |
| ECCP-3OCF3 | 4,50 % |
| ECCP-5OCF3 | 4,50 % |
| CBC-33F | 1,80 % |
| CBC-53F | 1,80 % |
| CBC-55F | 1,80 % |
| PCH-6F | 7,20 % |
| PCH-7F | 5,40 % |
| CCP-2OCF3 | 7,20 % |
| CCP-3OCF3 | 10,80 % |
| CCP-4OCF3 | 6,30 % |
| CCP-5OCF | 9,90 % |
| PCH-5F | 9,00 % |
| Verbindung des Beispiels 13 | 10,00 % |

weist folgende Eigenschaften auf:

| | |
|---|---|
| Klärpunkt: | +80,7°C |
| Δε: | +5,8 |
| Δn: | +0,0916 |

### Beispiel 1849

Eine Flüssigkristallmischung enthaltend

| | |
|---|---|
| PCH-3O1 | 9,00 % |
| PCH-3O2 | 9,00 % |
| CCH-3O1 | 29,70 % |
| CCN-47 | 9,90 % |
| CCN-55 | 9,00 % |
| CBC-33F | 4,50 % |
| CBC-53F | 4,50 % |
| CBC-55F | 4,50 % |
| CBC-33 | 4,50 % |
| CBC-53 | 5,40 % |
| Verbindung des Beispiels 13 | 10,00 % |

weist folgende Eigenschaften auf:

| | |
|---|---|
| Klärpunkt: | +72,0°C |
| Δε: | -0,4 |

## Patentansprüche

1. Chroman-Derivate der allgemeinen Formel (la) 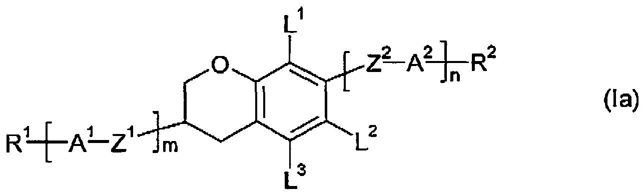 worin
R¹ einen linearen oder verzweigten, gegebenenfalls chiralen, unsubstituierten, einen einfach durch CN oder CF₃ oder einen mindestens einfach durch Halogen substituierten Alkylrest mit 1 bis 15 C-Atomen oder Alkenylrest mit 2 bis 15 C-Atomen, worin auch eine oder mehrere CH₂-Gruppen jeweils unabhängig voneinander durch -O-, -S-, -CO-, -CO-O-, -O-CO-, -O-CO-O-, -CH=CH-, -CH=CF-, -CF=CH-, -CF=CF-, -C≡C- oder 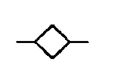 so ersetzt sein können, dass Heteroatome nicht direkt miteinander verknüpft sind,
R² H, F, CI, NCS, CN, SF₅, einen Alkyl- oder Alkoxyrest mit 1 bis 15 C-Atomen, einen Alkenyl- oder Alkenyloxyrest mit 2 bis 15 C-Atomen, einen durch ein oder mehrere Fluoratome substituierten Alkyl- oder Alkoxyrest mit 1 bis 15 C-Atomen oder einen durch ein oder mehrere Fluoratome substituierten Alkenyl- oder Alkenyloxyrest mit 2 bis 15 C-Atomen,
A¹, A² jeweils unabhängig voneinander, gleich oder verschieden
a) trans-1,4-Cyclohexylen, worin auch eine oder mehrere nicht benachbarte CH₂-Gruppen durch -O- und/oder -S-ersetzt sein können,
b) 1,4-Phenylen, worin eine oder zwei CH-Gruppen durch N ersetzt sein können und worin auch ein oder mehrere H-Atome durch F ersetzt sein können,
c) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl, oder
d) 1,4-Cyclohexenylen,
Z¹, Z² jeweils unabhängig voneinander, gleich oder verschieden, eine Einfachbindung, -O-, -CH₂O-, -OCH₂-, -CO-O-, -O-CO-, -CF₂O-, -OCF₂-, -CF₂CF₂-, -CH₂CF₂-, -CF₂CH₂-, -CH₂CF₂O-, -OCF₂CH₂-, -CH₂CH₂-, -CH=CH-, -CH=CF-, -CF=CH-, -CF=CF-, -CF=CF-CO-O-, -O-CO-CF=CF- oder -C≡C-,
L¹, L², L³ jeweils unabhängig voneinander, gleich oder verschieden H, F, CI, NCS, CN, SF₅ oder einen durch ein oder mehrere Fluoratome substituierten Alkyl- oder Alkoxyrest mit 1 bis 15 C Atomen, einen durch ein oder mehrere Fluoratome substituierten Alkenyl- oder Alkenyloxyrest mit 2 bis 15 C Atomen,
H,
m 0, 1, 2 oder 3, und
n 1, 2, 3 oder 4 bedeutet,
jedoch mit der Maßgabe, dass die Summe (m + n) = 1, 2, 3 oder 4 ist, und der allgemeinen Formel (IIa) 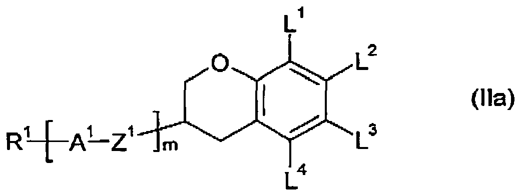 worin R¹, A¹ und Z¹ die in bezug auf Formel (Ia) angegebenen Bedeutungen aufweisen,
L¹, L², L³ und L⁴ jeweils unabhängig voneinander, gleich oder verschieden H, F, CI, NCS, CN, SF₅, einen durch ein oder mehrere Fluoratome substituierten Alkyl- oder Alkoxyrest mit 1 bis 15 C-Atomen oder einen durch ein oder mehrere Fluoratome substituierten Alkenyl- oder Alkenyloxyrest mit 2 bis 15 C-Atomen, wobei
einer der beiden Reste L² und L³ zusätzlich auch die Bedeutung von R² in bezug auf Formel (la) annehmen kann, und
m 1, 2 oder 3 bedeutet.

2. Chroman-Derivate nach Anspruch 1, **dadurch gekennzeichnet, dass**
R² F, CI, CN, SF₅, CF₃, OCF₃ oder OCHF₂
bedeutet.

3. Chroman-Derivate nach Anspruch 1 oder 2, **dadurch gekennzeichnet, dass** es sich bei den Verbindungen der Teilformel (la) um Verbindungen der folgenden Teilformeln handelt: 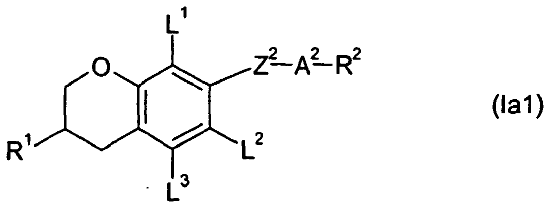 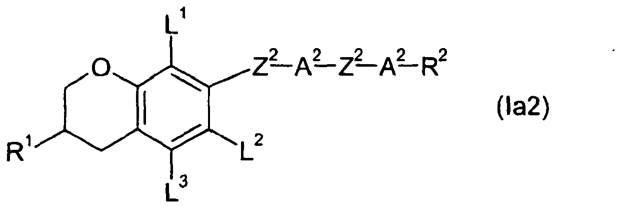 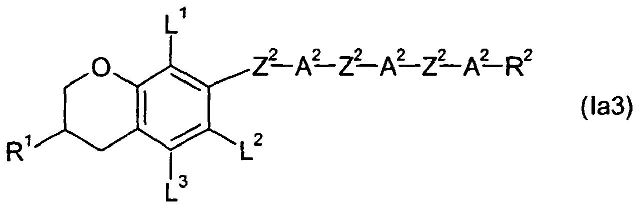 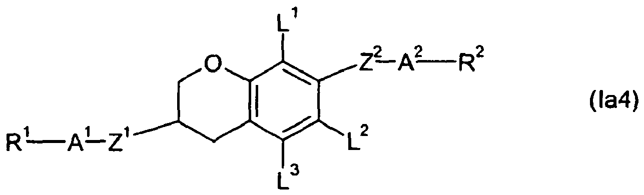 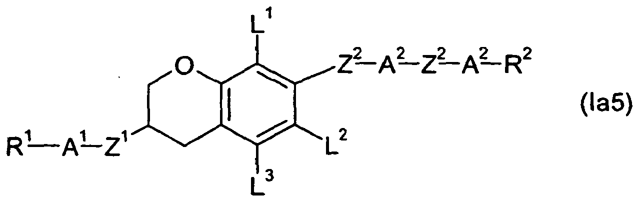 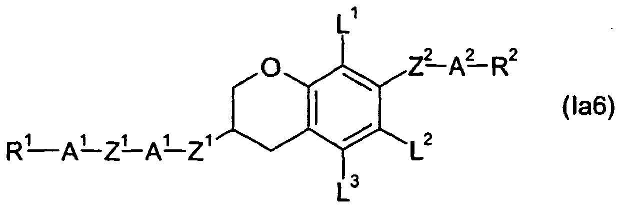 worin R¹, R², A¹, A², Z¹, Z², L¹, L² und L³ die in bezug auf Formel (I) in Anspruch 1 angegebenen Bedeutungen aufweisen.

4. Chroman-Derivate nach einem oder mehreren der Ansprüche 1 bis 3, **dadurch gekennzeichnet, dass** in Formel (la) oder (Ia1) bis (Ia6)
L³ = H und
L¹ und L² unabhängig H oder F
bedeuten.

5. Chroman-Derivate nach einem oder mehreren der Ansprüche 1 bis 4, **dadurch gekennzeichnet, dass** die Verbindungen (la) bzw. (Ia1) bis (Ia6) ausgewählt sind aus den Verbindungen der Teilformeln (Ia1 a), (Ia2a), (Ia2b), (Ia2c), (Ia3a), (Ia4a), (Ia4b), (Ia4c) oder (Ia5a) bis (Ia5i): 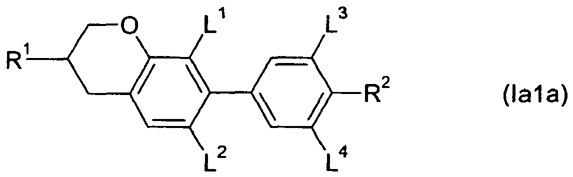 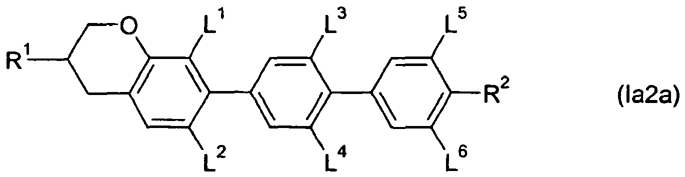 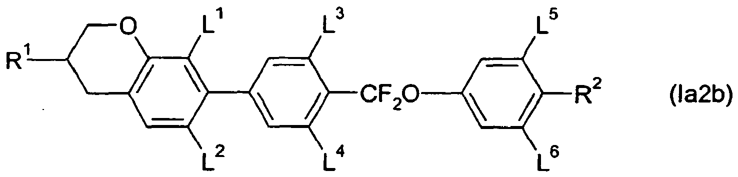 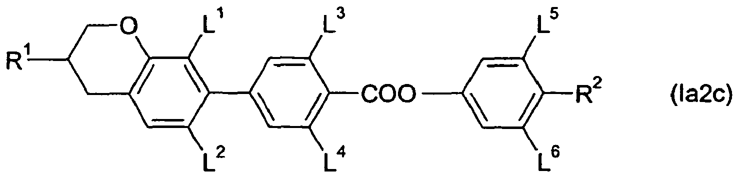 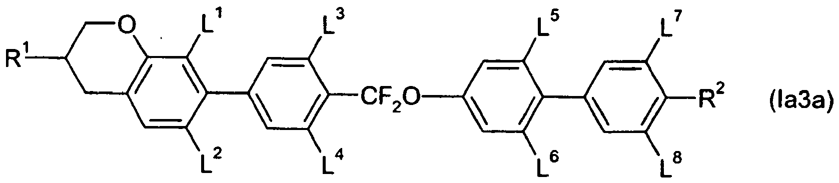 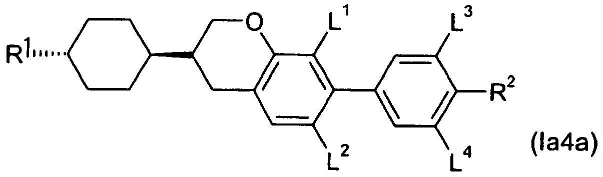 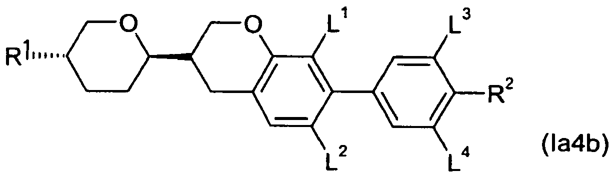 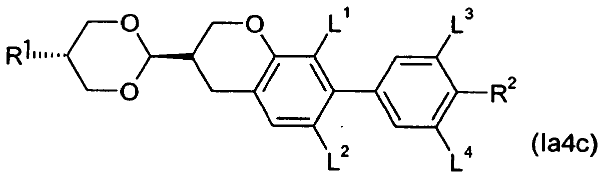 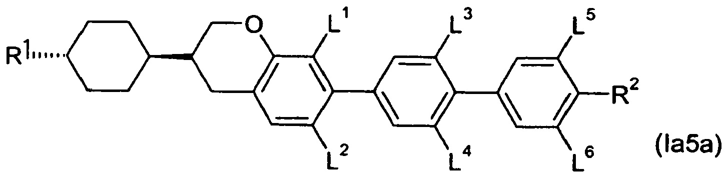 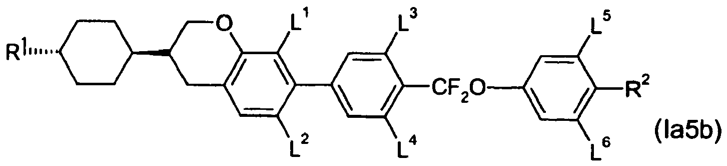 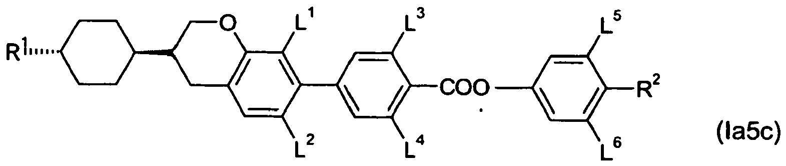 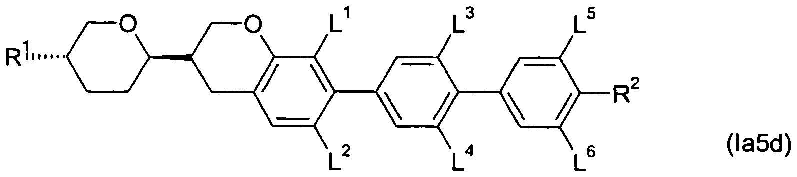 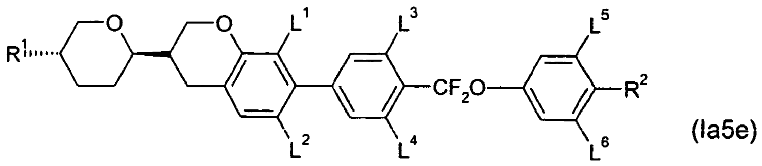 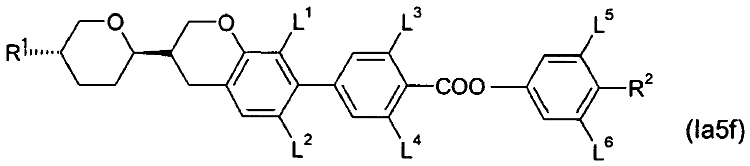 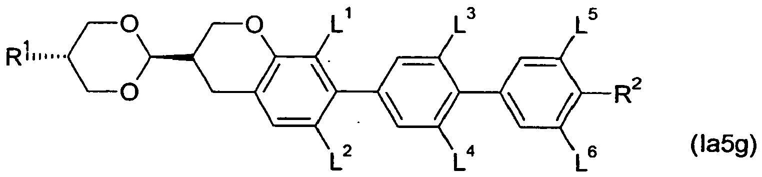 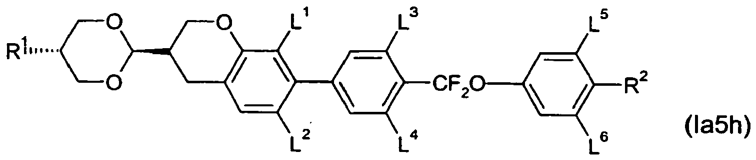 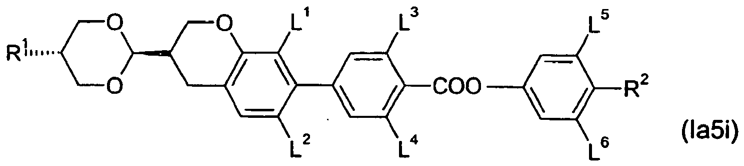 worin R¹ und R² die in bezug auf Formel (I) nach Anspruch 1 angegebenen Bedeutungen aufweisen und L¹, L², L³, L⁴, L⁵ und L⁶ unabhängig voneinander, gleich oder verschieden H oder F bedeuten.

6. Chroman-Derivate nach Anspruch 1, **dadurch gekennzeichnet, dass** es sich bei den Verbindungen der Teilformel (IIa) um Verbindungen der folgenden Teilformeln handelt: 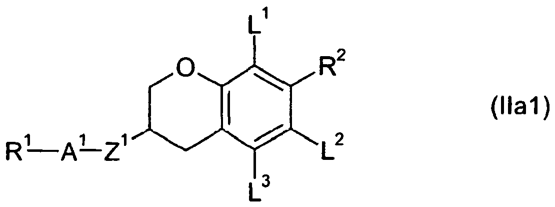 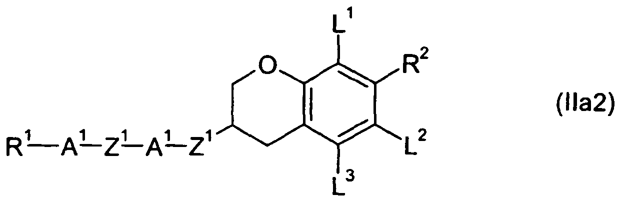 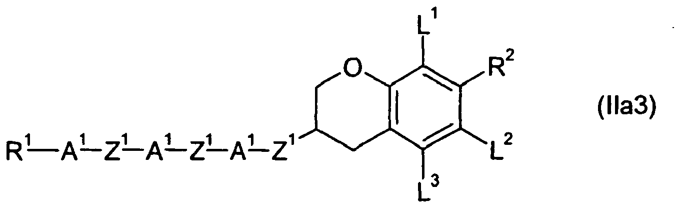 worin R¹, A¹, Z¹, L¹, L² und L³ die in bezug auf Formel (II) und R² die in bezug auf Formel (I) in Anspruch 1 angegebenen Bedeutungen aufweisen.

7. Chroman-Derivate nach einem oder mehreren der Ansprüche 1 bis 6, **dadurch gekennzeichnet, dass** in Formel la
L² F und
in Formel IIa
L³ F
bedeutet.

8. Chroman-Derivate der allgemeinen Formeln (IIa1) bis (IIa3) nach Anspruch 6, **dadurch gekennzeichnet, dass** sie die folgenden Strukturen aufweisen: 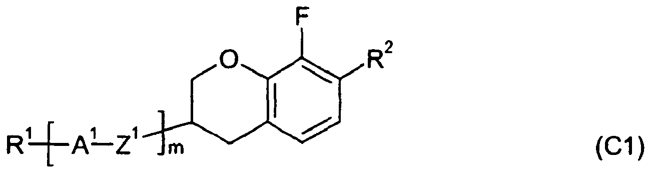 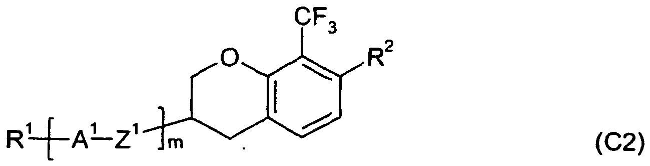 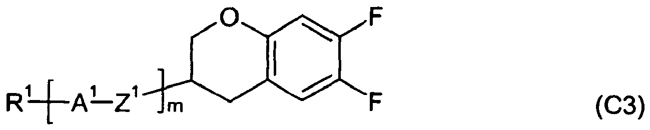 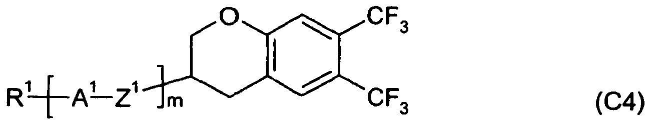 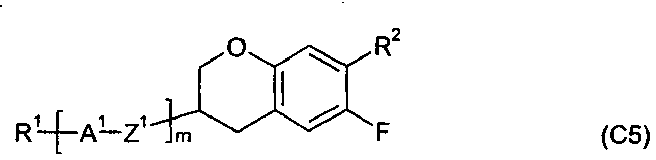 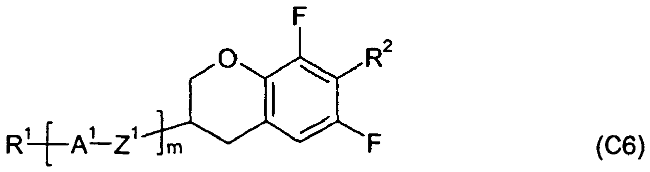 worin R¹, A¹ und Z¹ die in bezug auf Formel (IIa) in Anspruch 1 angegebenen Bedeutungen annehmen, R² die in bezug auf Formel (Ia) in Anspruch 1 angegebenen Bedeutungen annimmt und m = 1, 2 oder 3 bedeutet.

9. Verwendung von Chroman-Derivaten nach einem oder mehreren der Ansprüche 1 bis 8 als Komponente(n) in flüssigkristallinen Medien.

10. Flüssigkristallines Medium mit mindestens zwei flüssigkristallinen Komponenten, **dadurch gekennzeichnet, dass** es mindestens ein Chroman-Derivat nach einem oder mehreren der Ansprüche 1 bis 8 enthält.

11. Flüssigkristall-Anzeigeelement, **dadurch gekennzeichnet, dass** es ein flüssigkristallines Medium nach Anspruch 10 enthält.

12. Elektrooptisches Anzeigeelement, **dadurch gekennzeichnet, dass** es als Dielektrikum ein flüssigkristallines Medium nach Anspruch 10 enthält.

## Claims

1. Chromane derivatives of the general formula (Ia) 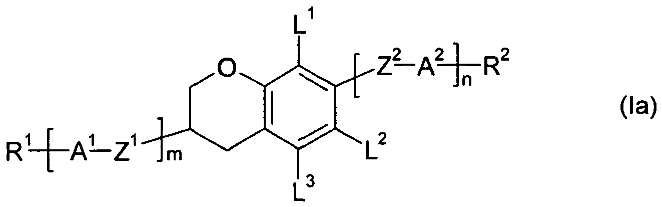 in which
R¹ denotes a linear or branched, optionally chiral alkyl radical having 1 to 15 C atoms or alkenyl radical having 2 to 15 C atoms which is unsubstituted, monosubstituted by CN or CF₃ or at least monosubstituted by halogen and in which one or more CH₂ groups may each be replaced, independently of one another, by -O-, -S-, -CO-, -CO-O-, -O-CO-, -O-CO-O-, -CH=CH-, -CH=CF-, -CF=CH-, -CF=CF-, -C≡C- or 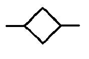 in such a way that heteroatoms are not linked directly to one another,
R² denotes H, F, Cl, NCS, CN, SF₅, an alkyl or alkoxy radical having 1 to 15 C atoms, an alkenyl or alkenyloxy radical having 2 to 15 C atoms, an alkyl or alkoxy radical having 1 to 15 C atoms which is substituted by one or more fluorine atoms, or an alkenyl or alkenyloxy radical having 2 to 15 C atoms which is substituted by one or more fluorine atoms,
A¹, A² each, independently of one another, identically or differently, denote
a) trans-1,4-cyclohexylene, in which, in addition, one or more non-adjacent CH₂ groups may be replaced by -O- and/or -S-,
b) 1,4-phenylene, in which one or two CH groups may be replaced by N and in which, in addition, one or more H atoms may be replaced by F,
c) a radical from the group 1,4-bicyclo(2,2,2)octylene, piperidine-1,4-diyl, naphthalene-2,6-diyl, decahydronaphthalene-2,6-diyl and 1,2,3,4-tetrahydronaphthalene-2,6-diyl, or
d) 1,4-cyclohexenylene,
Z¹, Z² each, independently of one another, identically or differently, denote a single bond, -O-, -CH₂O-, -OCH₂-, -CO-O-, -O-CO-, -CF₂O-, -OCF₂-, -CF₂CF₂-, -CH₂CF₂-, -CF₂CH₂-, -CH₂CF₂O-, -OCF₂CH₂-, -CH₂CH₂-, -CH=CH-, -CH=CF-, -CF=CH-, -CF=CF-, -CF=CF-CO-O-, -O-CO-CF=CF- or -C≡C-,
L¹, L², L³ each, independently of one another, identically or differently, denote H, F, Cl, NCS, CN, SF₅, an alkyl or alkoxy radical having 1 to 15 C atoms which is substituted by one or more fluorine atoms, or an alkenyl or alkenyloxy radical having 2 to 15 C atoms which is substituted by one or more fluorine atoms,
m denotes 0, 1, 2 or 3, and
n denotes 1, 2, 3 or 4,
but with the proviso that the sum (m + n) = 1, 2, 3 or 4,
and of the general formula (IIa) 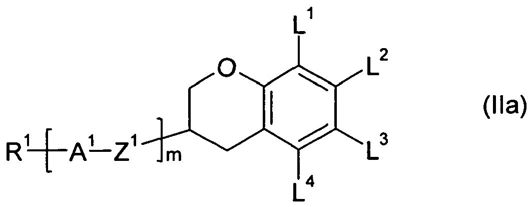 in which R¹, A¹ and Z¹ have the meanings indicated in relation to formula (Ia),
L¹, L², L³ and L⁴ each, independently of one another, identically or differently, denote H, F, Cl, NCS, CN, SF₅, an alkyl or alkoxy radical having 1 to 15 C atoms which is substituted by one or more fluorine atoms, or an alkenyl or alkenyloxy radical having 2 to 15 C atoms which is substituted by one or more fluorine atoms, where one of the two radicals L² and L³ may additionally also adopt the meaning of R² in relation to formula (Ia), and
m denotes 1, 2 or 3.

2. Chromane derivatives according to Claim 1, **characterised in that**
R² denotes F, Cl, CN, SF₅, CF₃, OCF₃ or OCHF₂.

3. Chromane derivatives according to Claim 1 or 2, **characterised in that** the compounds of the sub-formula (Ia) are compounds of the following sub-formulae: 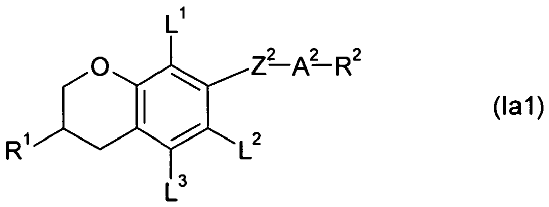 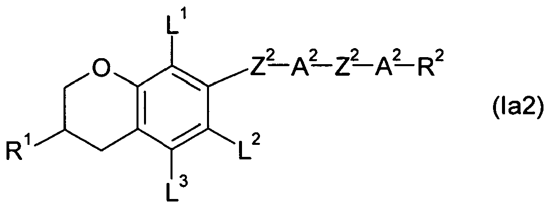 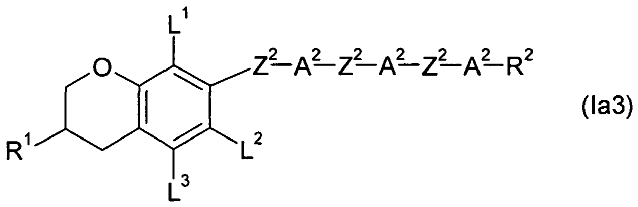 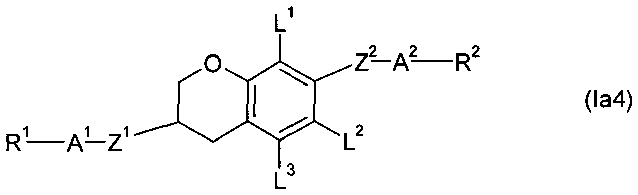 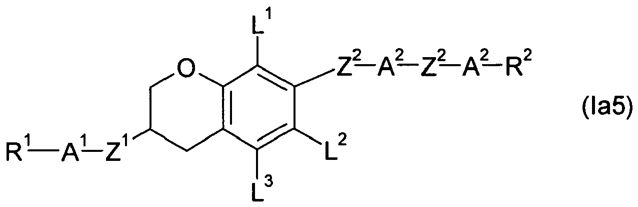 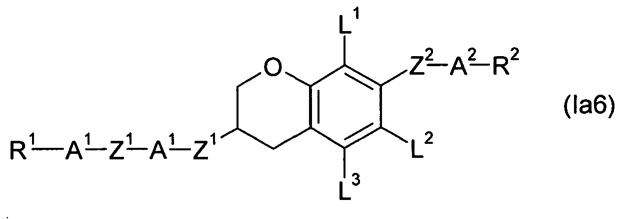 in which R¹, R², A¹, A², Z¹, Z², L¹, L² and L³ have the meanings indicated in relation to formula (Ia) in Claim 1.

4. Chromane derivatives according to one or more of Claims 1 to 3, **characterised in that**, in formula (Ia) or (Ia1) to (la6),
L³ = H and
L¹ and L² independently denote H or F.

5. Chromane derivatives according to one or more of Claims 1 to 4, **characterised in that** the compounds (Ia) or (Ia1) to (la6) are selected from the compounds of the sub-formulae (Ia1a), (la2a), (la2b), (la2c), (la3a), (la4a), (la4b), (la4c) or (Ia5a) to (Ia5i): 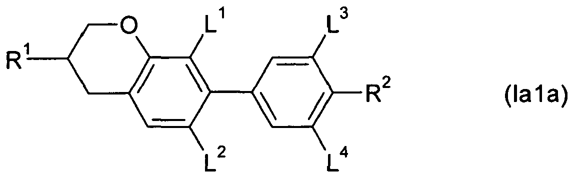 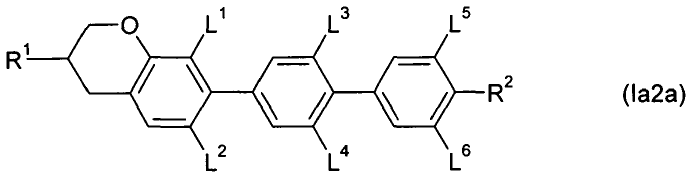 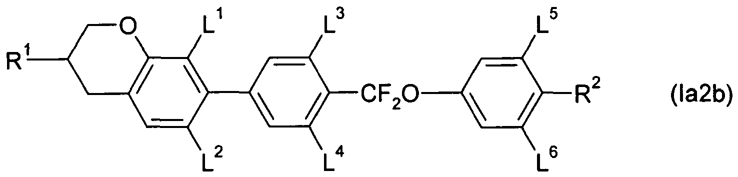 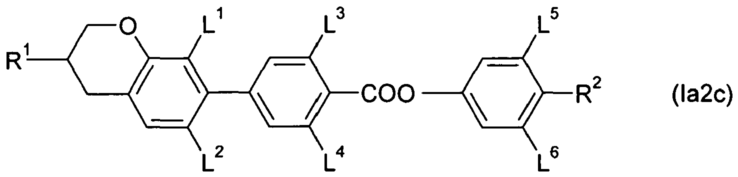 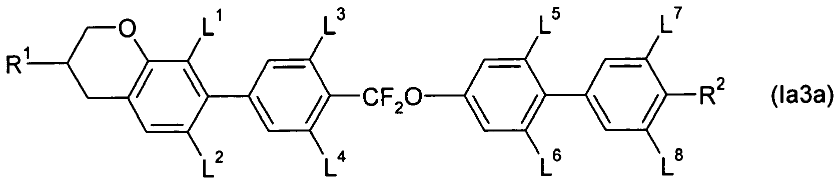 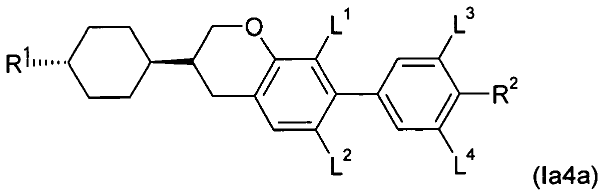 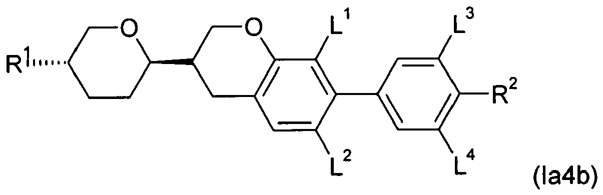 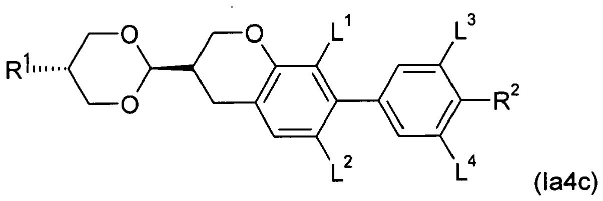 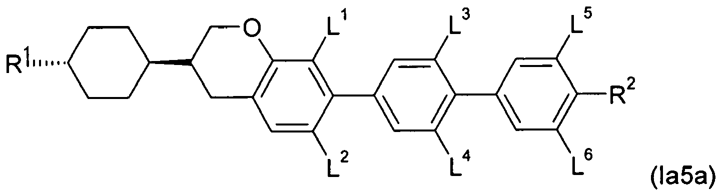 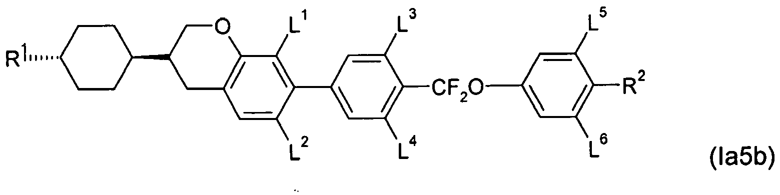 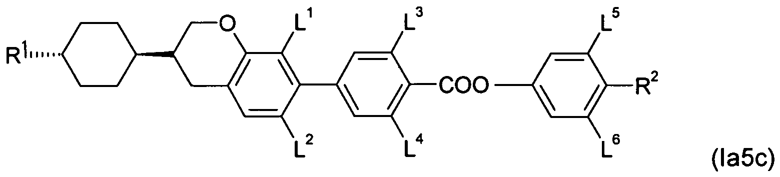 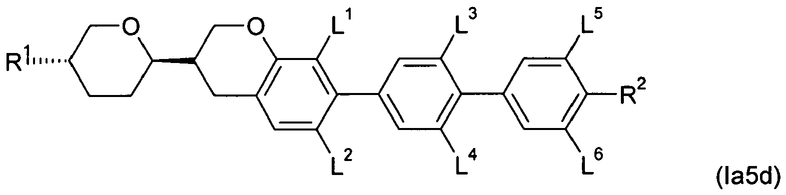 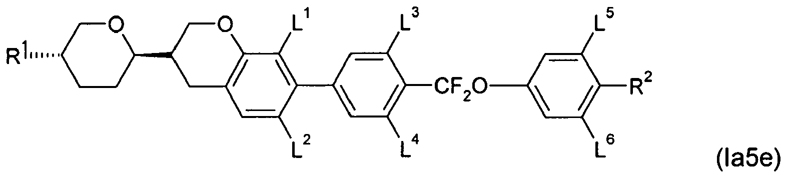 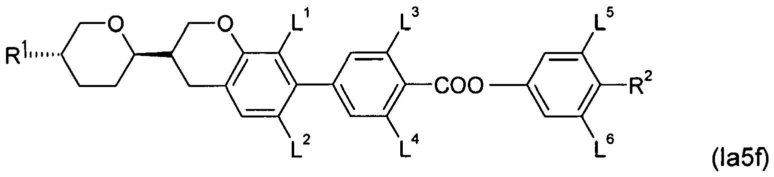 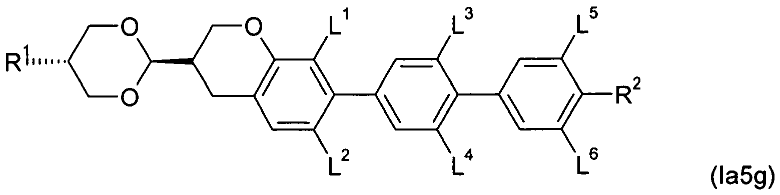 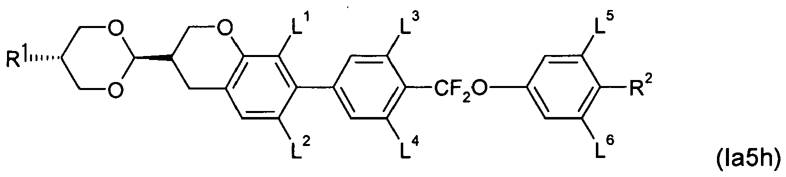 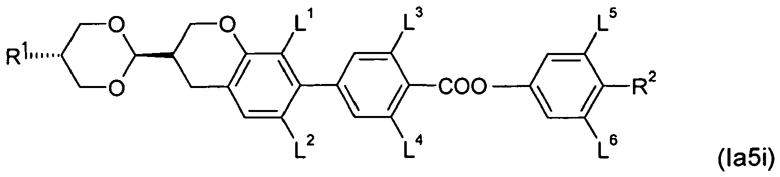 in which
R¹ and R² have the meanings indicated in relation to formula (Ia) according to Claim 1 and L¹, L², L³, L⁴, L⁵ and L⁶, independently of one another, identically or differently, denote H or F.

6. Chromane derivatives according to Claim 1, **characterised in that** the compounds of the sub-formula (IIa) are compounds of the following sub-formulae: 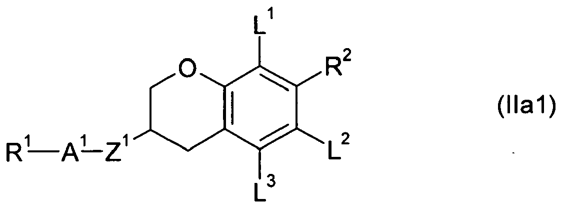 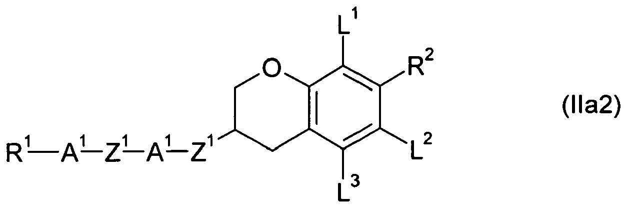 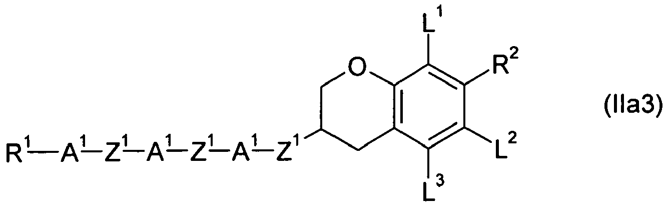 in which R¹, A¹, Z¹, L¹, L² and L³ have the meanings indicated in relation to formula (IIa) and R² has the meanings indicated in relation to formula (Ia) in Claim 1.

7. Chromane derivatives according to one or more of Claims 1 to 6, **characterised in that** in formula la
L² denotes F and in formula IIa L³ denotes F.

8. Chromane derivatives of the general formulae (IIa1) to (IIa3) according to Claim 6, **characterised in that** they have the following structures: 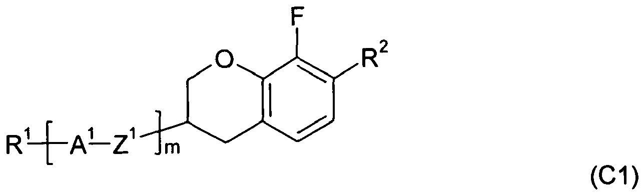 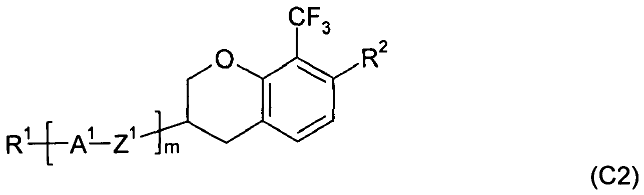 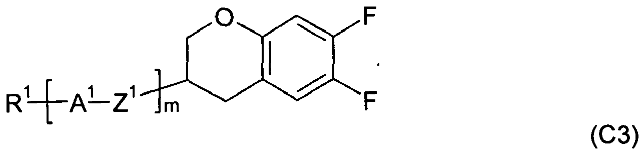 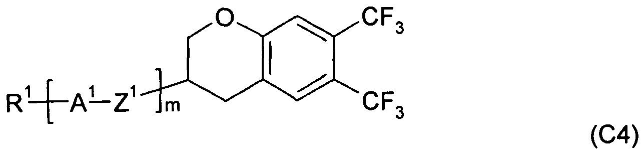 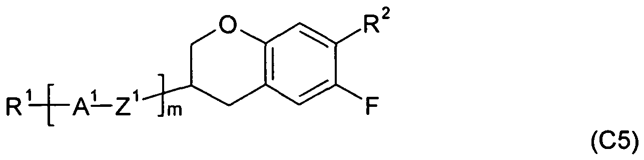 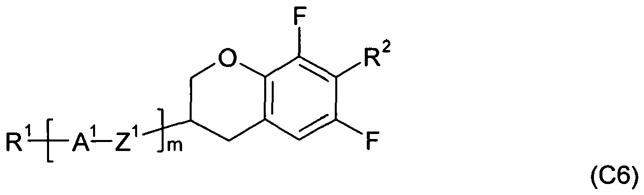 in which R¹, A¹ and Z¹ adopt the meanings indicated in relation to formula (IIa) in Claim 1, R² adopts the meanings indicated in relation to formula (Ia) in Claim 1 and m = 1, 2 or 3.

9. Use of chromane derivatives according to one or more of Claims 1 to 8 as component(s) in liquid-crystalline media.

10. Liquid-crystalline medium having at least two liquid-crystalline components, **characterised in that** it comprises at least one chromane derivative according to one or more of Claims 1 to 8.

11. Liquid-crystal display element, **characterised in that** it contains a liquid-crystalline medium according to Claim 10.

12. Electro-optical display element, **characterised in that** it contains, as dielectric, a liquid-crystalline medium according to Claim 10.

## Revendications

1. Dérivés de chromane de la formule générale (la) 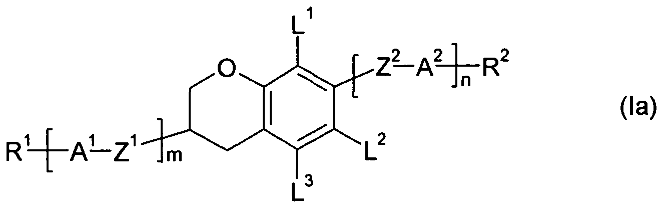 dans laquelle
R¹ représente un radical alkyle linéaire ou ramifié, en option chiral, comportant de 1 à 15 atomes de C ou un radical alkényle comportant de 2 à 15 atomes de C, lequel est non substitué, monosubstitué par CN ou CF₃ ou au moins monosubstitué par halogène et où un ou plusieurs groupes CH₂ peuvent chacun être remplacés, indépendamment les uns des autres, par -O-, -S-, -CO-, -CO-O-, -O-CO-, -O-CO-O-, -CH=CH-, -CH=CF-, -CF=CH-, -CF=CF-, -C≡C- ou 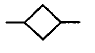 de telle sorte que des hétéroatomes ne soient pas liés directement les uns aux autres,
R² représente H, F, CI, NCS, CN, SF₅, un radical alkyle ou alcoxy comportant de 1 à 15 atomes de C, un radical alkényle ou alkényloxy comportant de 2 à 15 atomes de C, un radical alkyle ou alcoxy comportant de 1 à 15 atomes de C, lequel est substitué par un ou plusieurs atomes de fluor, ou un radical alkényle ou alkényloxy comportant de 2 à 15 atomes de C, lequel est substitué par un ou plusieurs atomes de fluor,
A¹, A² représentent, chacun indépendamment de l'autre, de manière identique ou différente,
a) trans-1,4-cyclohexylène, où, en outre, un ou plusieurs groupes CH₂ non adjacents peut/peuvent être remplacé(s) par -O- et/ou -S-,
b) 1,4-phénylène, où un ou deux groupes CH peut/peuvent être remplacé(s) par N et où, en outre, un ou plusieurs atomes de H peut/peuvent être remplacé(s) par F,
c) un radical pris parmi le groupe 1,4-bicyclo(2,2,2)-octylène, pipéridine-1,4-diyle, naphtalène-2,6-diyle, décahydronaphtalène-2,6-diyle et 1,2,3,4-tétra-hydronaphtalène-2,6-diyle, ou
d) 1,4-cyclohexénylène,
Z¹, Z² représentent, chacun indépendamment de l'autre, de manière identique ou différente, une liaison simple, -O-, -CH₂O-, -OCH₂-, -CO-O-, -O-CO-, -CF₂O-, -OCF₂-, -CF₂CF₂-, -CH₂CF₂-, -CF₂CH₂-, -CH₂CF₂O-, -OCF₂CH₂-, -CH₂CH₂-, -CH=CH-, -CH=CF-, -CF=CH-, -CF=CF-, -CF=CF-CO-O-, -O-CO-CF=CF- ou -C≡C-,
L¹, L², L³ représentent, chacun indépendamment des autres, de manière identique ou différente, H, F, Cl, NCS, CN, SF₅, un radical alkyle ou alcoxy comportant de 1 à 15 atomes de C, lequel est substitué par un ou plusieurs atomes de fluor, ou un radical alkényle ou alkényloxy comportant de 2 à 15 atomes de C, lequel est substitué par un ou plusieurs atomes de fluor,
m représente 0, 1, 2 ou 3, et
n représente 1, 2, 3 ou 4,
mais étant entendu que la somme (m + n) = 1, 2, 3 ou 4, et de la formule générale (IIa) 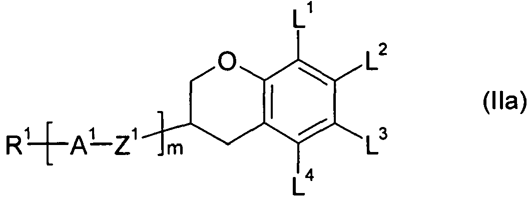 dans laquelle R¹, A¹ et Z¹ présentent les significations indiquées en relation avec la formule (la),
L¹, L², L³ et L⁴ représentent, chacun indépendamment des autres, de manière identique ou différente, H, F, CI, NCS, CN, SF₅, un radical alkyle ou alcoxy comportant de 1 à 15 atomes de C, lequel est substitué par un ou plusieurs atomes de fluor, ou un radical alkényle ou alkényloxy comportant de 2 à 15 atomes de C, lequel est substitué par un ou plusieurs atomes de fluor, où l'un des deux radicaux L² et L³ peut additionnellement également adopter la signification de R² en relation avec la formule (la), et
m représente 1, 2 ou 3.

2. Dérivés de chromane selon la revendication 1, **caractérisés en ce que** :
R² représente F, CI, CN, SF₅, CF₃, OCF₃ ou OCHF₂.

3. Dérivés de chromane selon la revendication 1 ou 2, **caractérisés en ce que** les composés de la sous-formule (la) sont des composés des sous-formules qui suivent : 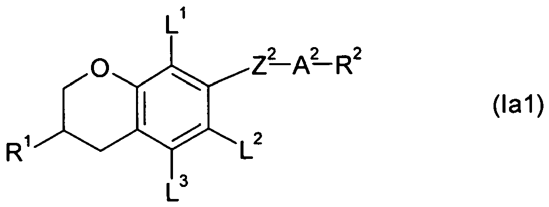 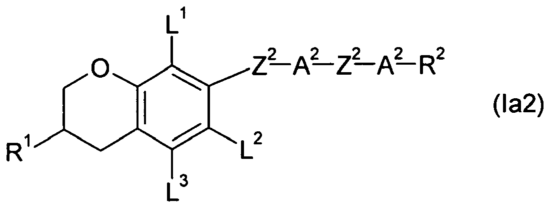 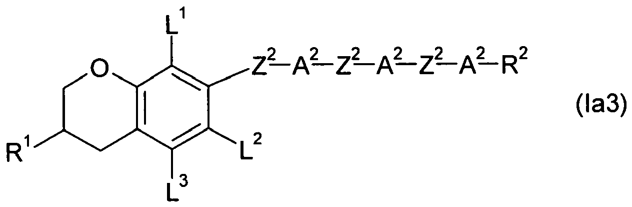 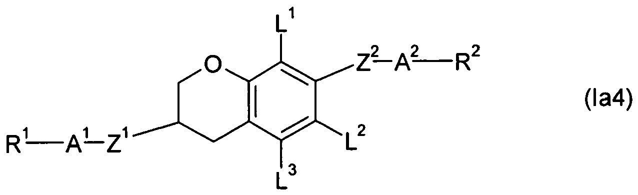 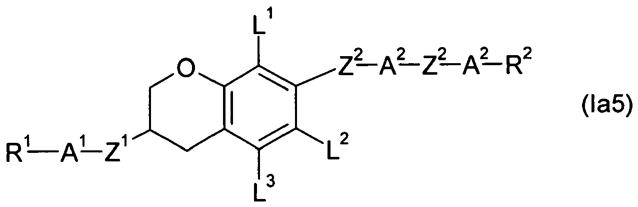 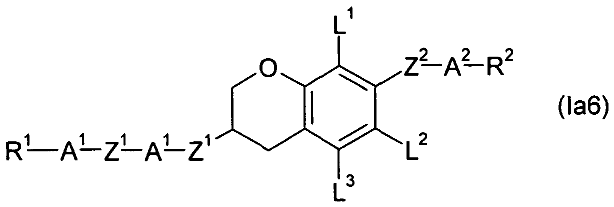 dans lesquelles R¹, R², A¹, A², Z¹, Z² , L¹, L² et L³ présentent les significations indiquées en relation avec la formule (la) selon la revendication 1.

4. Dérivés de chromane selon une ou plusieurs des revendications 1 à 3, **caractérisés en ce que**, dans les formules (la) ou (Ia1) à (la6),
L³ = H et
L¹ et L² représentent, indépendamment l'un de l'autre, H ou F.

5. Dérivés de chromane selon une ou plusieurs des revendications 1 à 4, **caractérisés en ce que** les composés (la) ou (Ia1) à (la6) sont choisis parmi les composés des sous-formules (Ia1a), (la2a), (la2b), (la2c), (la3a), (la4a), (la4b), (la4c) ou (Ia5a) à (Ia5i) : 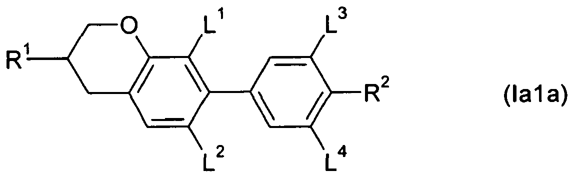 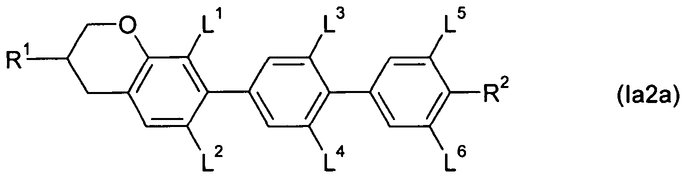 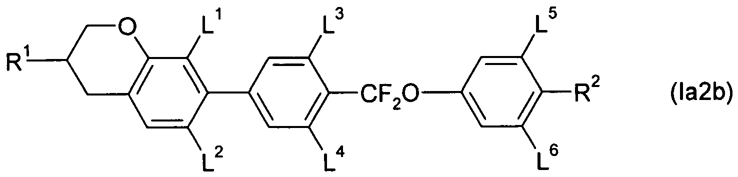 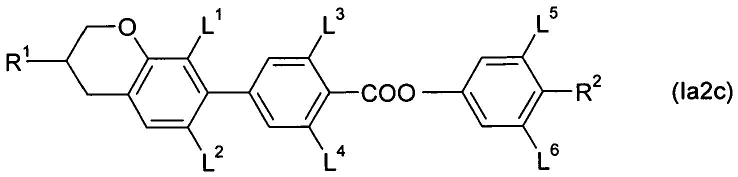 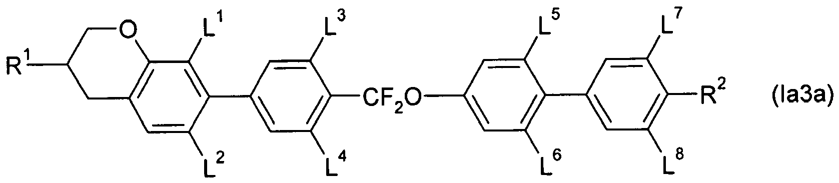 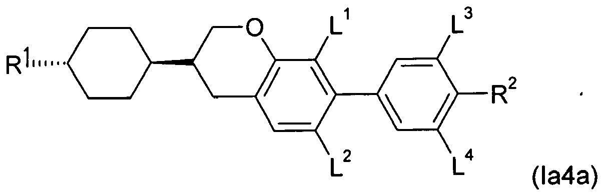 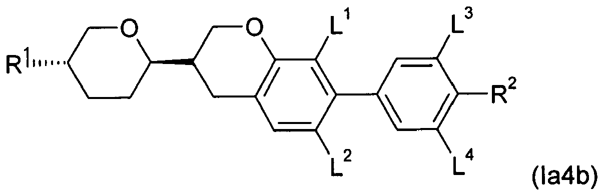 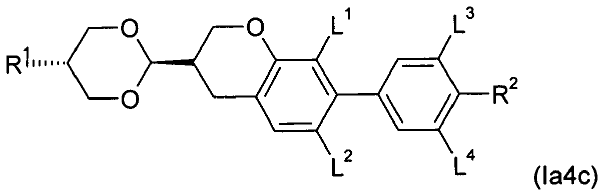 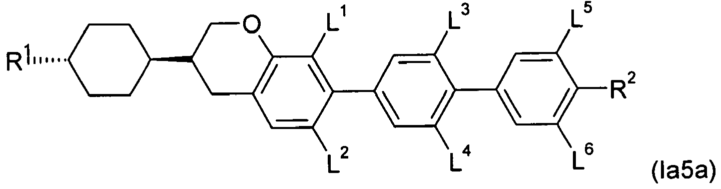 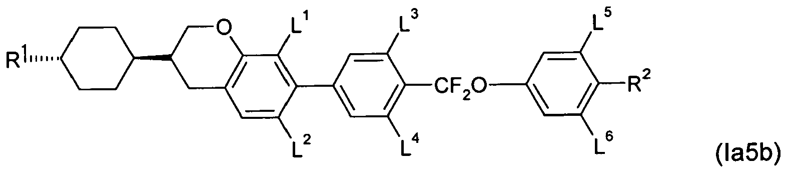 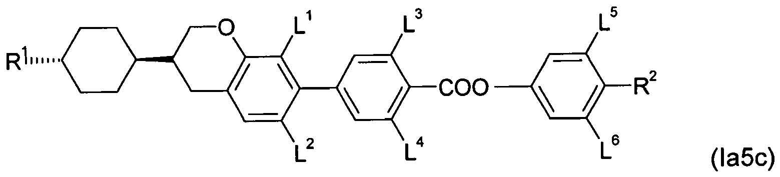 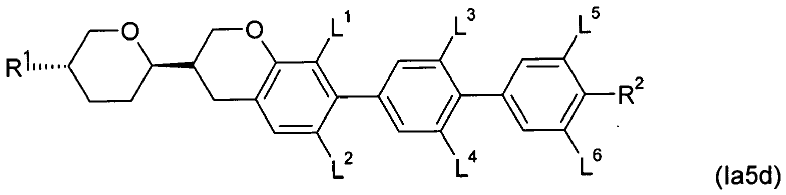 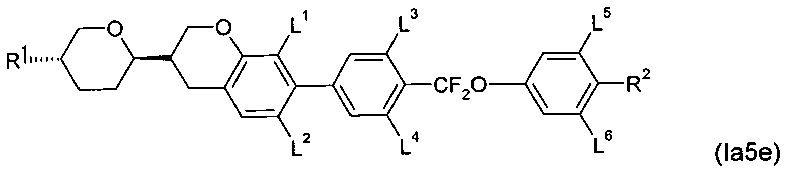 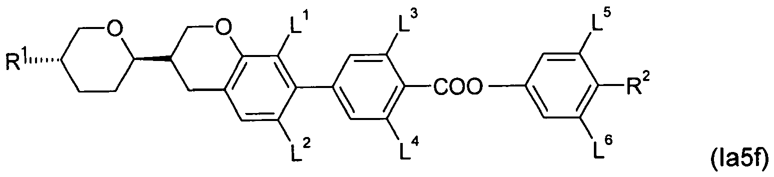 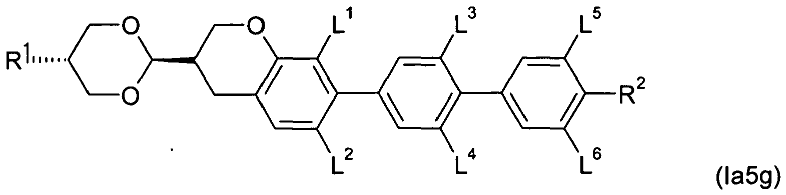 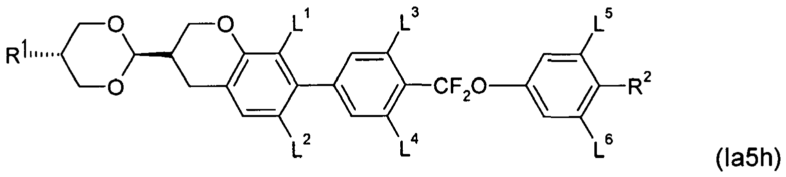 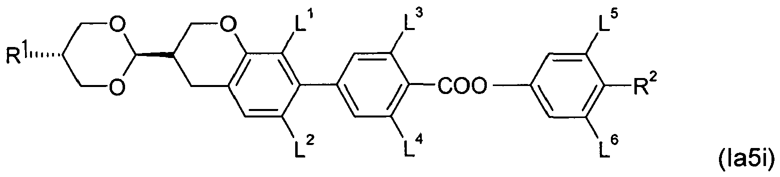 dans lesquelles
R¹ et R² présentent les significations indiquées en relation avec la formule (la) selon la revendication 1 et L¹, L², L³, L⁴, L⁵ et L⁶, indépendamment les uns des autres, de manière identique ou différente, représentent H ou F.

6. Dérivés de chromane selon la revendication 1, **caractérisés en ce que** les composés de la sous-formule (IIa) sont des composés des sous-formules qui suivent : 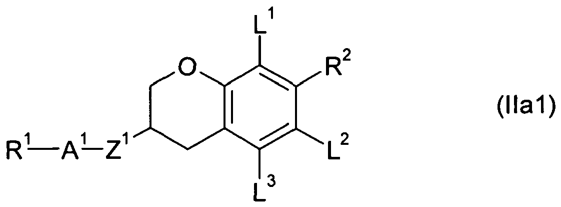 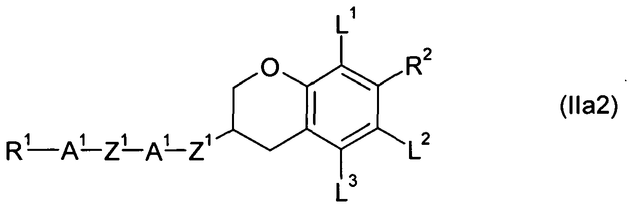 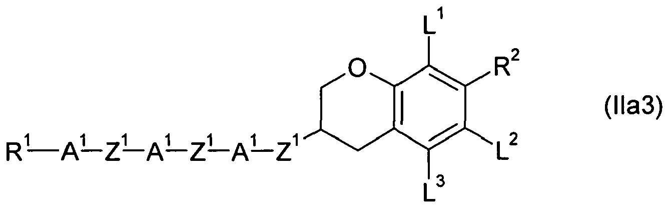 dans lesquelles R¹, A¹, Z¹, L¹, L² et L³ présentent les significations indiquées en relation avec la formule (IIa) et R² présente les significations indiquées en relation avec la formule (la) selon la revendication 1.

7. Dérivés de chromane selon une ou plusieurs des revendications 1 à 6, **caractérisés en ce que**, dans la formule la
L² représente F et
dans la formule IIa
L³ représente F.

8. Dérivés de chromane des formules générales (IIa1) à (IIa3) selon la revendication 6, **caractérisés en ce qu'**ils présentent les structures qui suivent : 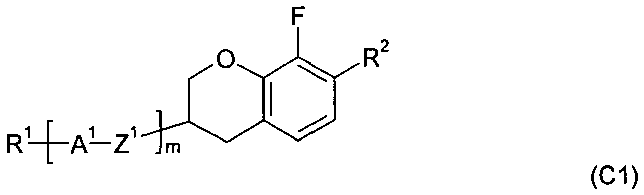 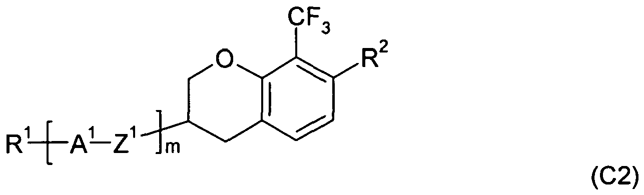 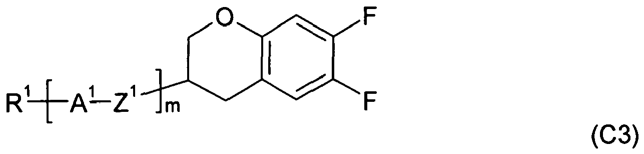 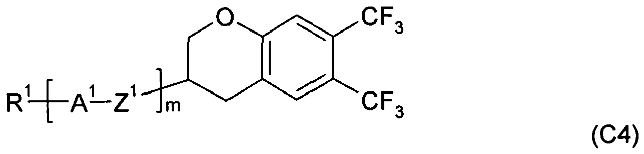 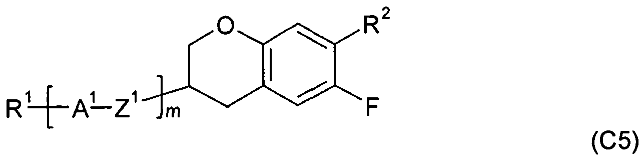 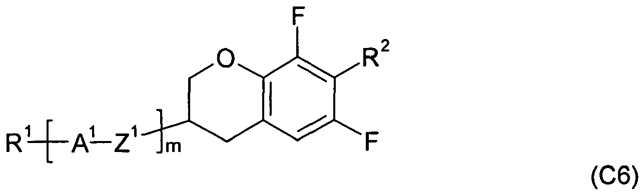 dans lesquelles R¹, A¹ et Z¹ adoptent les significations indiquées en relation avec la formule (IIa) selon la revendication 1, R² adopte les significations indiquées en relation avec la formule (la) selon la revendication 1 et m = 1, 2 ou 3.

9. Utilisation de dérivés de chromane selon une ou plusieurs des revendications 1 à 8 en tant que composant(s) dans des milieux cristallins liquides.

10. Milieu cristallin liquide comportant au moins deux composants cristallins liquides, **caractérisé en ce qu'**il comprend au moins un dérivé de chromane selon une ou plusieurs des revendications 1 à 8.

11. Elément d'affichage à cristaux liquides, **caractérisé en ce qu'**il contient un milieu cristallin liquide selon la revendication 10.

12. Elément d'affichage électro-optique, **caractérisé en ce qu'**il contient, en tant que diélectrique, un milieu cristallin liquide selon la revendication 10.
